(19) Europäisches Patentamt
European Patent Office
Office européen des brevets

(11) EP 2 280 006 A1

(12) **EUROPEAN PATENT APPLICATION**

(43) Date of publication:
**02.02.2011 Bulletin 2011/05**

(21) Application number: **10170818.8**

(22) Date of filing: **08.08.2006**

(51) Int Cl.:
***C07D 263/32*** *(2006.01)* ***C07D 277/30*** *(2006.01)*
***A61K 31/421*** *(2006.01)* ***A61K 31/426*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **08.08.2005 GB 0516313**

(62) Document number(s) of the earlier application(s) in accordance with Art. 76 EPC:
**06765253.7 / 1 924 570**

(71) Applicant: **Pulmagen Therapeutics (Synergy) Limited**
**Flex Meadow**
**Harlow**
**Essex CM19 5TR (GB)**

(72) Inventors:
- **Bravo, Jose, Antonio**
  **Harlow, Essex CM19 5TR (GB)**
- **Bull, Richard, James**
  **Harlow, Essex CM19 5TR (GB)**
- **Finch, Harry**
  **Harlow, Essex CM19 5TR (GB)**
- **Ray, Nicholas, Charles**
  **Harlow, Essex CM19 5TR (GB)**
- **Van Den Heuvel, Marco**
  **Harlow, Essex CM19 5TR (GB)**

(74) Representative: **Phillips, Neil Godfrey Alasdair**
**AstraZeneca AB,**
**Global Intellectual Property**
**151 85 Sodertalje (SE)**

Remarks:
This application was filed on 26-07-2010 as a divisional application to the application mentioned under INID code 62.

(54) **PHARMACEUTICAL COMPOSITION FOR INHALATION COMPRISING AN OXAZOLE OR THIAZOLE M3 MUSCARINIC RECEPTOR ANTAGONIST**

(57) Pharmaceutical compositions for inhalation comprising compounds of formula (I) are useful in the treatment of diseases where enhanced M3 receptor activation is implicated, such as respiritory tract diseases:

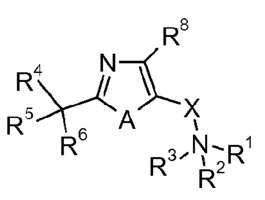

(I)

wherein (i) $\mathbf{R^1}$ is $C_1$-$C_6$-alkyl or hydrogen; and $\mathbf{R^2}$ is hydrogen or a group $-R^7$, $-Z-Y-R^7$, $-Z-NR^9R^{10}$; $-Z-CO-NR^9R^{10}$, $-Z-NR^9-C(O)O-R^7$, or $-Z-C(O)-R^7$; and $\mathbf{R^3}$ is a lone pair, or $C_1$-$C_6$-alkyl; or (ii) $\mathbf{R^1}$ and $\mathbf{R^3}$ together with the nitrogen to which they are attached form a heterocycloalkyl ring, and $\mathbf{R^2}$ is a lone pair or a group $-R^7$, $-Z-Y-R^7$, $-Z-NR^9R^{10}$, $-Z-CO-NR^9R^{10}$, $-Z-NR^9-C(O)O-R^7$; or; $-Z-C(O)-R^7$; or (iii) **$R^1$ and $R^2$** together with the nitrogen to which they are attached form a heterocycloalkyl ring, said ring being substituted by a group $-Y-R^7$, $-Z-Y-R^7$, $-Z-NR^9R^{10}$; $-Z-CO-NR^9R^{10}$; $-Z-NR^9-C(O)OR^7$; or; $-Z-C(O)-R^7$; and $\mathbf{R^3}$ is a lone pair, or $C_1$-$C_6$-alkyl; $\mathbf{R^4}$ and $\mathbf{R^5}$ are independently selected from the group consisting of aryl, aryl-fused-heterocycloalkyl, heteroaryl, $C_1$-$C_6$-alkyl, cycloalkyl; $\mathbf{R^6}$ is -OH, $C_1$-$C_6$-alkyl, $C_1$-$C_6$-alkoxy, hydroxy-$C_1$-$C_6$-alkyl, nitrile, a group $CONR^8_2$ or a hydrogen atom; **A** is an oxygen or a sulfur atom; **X** is an alkylene, alkenylene or alkynylene group; $\mathbf{R^7}$ is an $C_1$-$C_6$-alkyl, aryl, aryl-fused-cycloalkyl, aryl-fused-heterocycloalkyl, heteroaryl, aryl ($C_1$-$C_8$-alkyl)-, heteroaryl($C_1$-$C_8$-alkyl)-, cycloalkyl or heterocycloalkyl group; $\mathbf{R^8}$ is $C_1$-$C_6$-alkyl or a hydrogen atom; **Z** is a $C_1$-$C_{16}$-alkylene, $C_2$-$C_{16}$-alkenylene or $C_2$-$C_{16}$-alkynylene group; **Y** is a bond or oxygen atom; $\mathbf{R^9}$ and $\mathbf{R^{10}}$ are independently a hydrogen atom, $C_1$-$C_6$-alkyl, aryl, aryl-fused-heterocycloalkyl, aryl-fused-cycloalkyl, heteroaryl, aryl($C_1$-$C_6$-alkyl)-, or heteroaryl

EP 2 280 006 A1

($C_1$-$C_6$-alkyl)- group; or **$R^9$** and **$R^{10}$** together with the nitrogen atom to which they are attached form a heterocyclic ring of 4-8 atoms, optionally containing a further nitrogen or oxygen atom.

## Description

### Field of the Invention

This invention relates to oxazole and thiazole derivatives, pharmaceutical compositions, methods for their preparation and use in the treatment of diseases where enhanced M3 receptor activation is implicated.

### Background to the invention

Anti-cholinergic agents prevent the passage of, or effects resulting from the passage of, impulses through the parasympathetic nerves. This is a consequence of the ability of such compounds to inhibit the action of acetylcholine (Ach) by blocking its binding to the muscarinic cholinergic receptors.

There are five subtypes of muscarinic acetylcholine receptors (mAChRs), termed M1-M5, and each is the product of a distinct gene and each displays unique pharmacological properties. mAChRs are widely distributed in vertebrate organs, and these receptors can mediate both inhibitory and excitatory actions. For example, in smooth muscle found in the airways, bladder and gastrointestinal tract, M3 mAChRs mediate contractile responses (reviewed by Caulfield, 1993, Pharmac. Ther., 58, 319 - 379).

In the lungs, muscarinic receptors M1, M2 and M3 have been demonstrated to be important and are localized to the trachea, the bronchi, submucosal glands and parasympathetic ganglia (reviewed in Fryer and Jacoby, 1998, Am J Resp Crit Care Med., 158 (5 part 3) S 154 - 160). M3 receptors on airway smooth muscle mediate contraction and therefore bronchoconstriction. Stimulation of M3 receptors localised to submucosal glands results in mucus secretion.

Increased signalling through muscarinic acetylcholine receptors has been noted in a variety of different pathophysiological states including asthma and COPD. In COPD, vagal tone may either be increased (Gross et al. 1989, Chest; 96:984-987) and/or may provoke a higher degree of obstruction for geometric reasons if applied on top of oedematous or mucus-laden airway walls (Gross et al. 1984, Am Rev Respir Dis; 129:856-870). In addition, inflammatory conditions can lead to a loss of inhibitory M2 receptor activity which results in increased levels of acetylcholine release following vagal nerve stimulation (Fryer et al, 1999, Life Sci., 64, (6-7) 449-455). The resultant increased activation of M3 receptors leads to enhanced airway obstruction. Thus the identification of potent muscarinic receptor antagonists would be useful for the therapeutic treatment of those disease states where enhanced M3 receptor activity is implicated. Indeed, contemporary treatment strategies currently support regular use of M3 antagonist bronchodilators as firstline therapy for COPD patients (Pauwels et al. 2001, Am Rev Respir Crit Care Med; 163:1256-1276)

Incontinence due to bladder hypercontractility has also been demonstrated to be mediated through increased stimulation of M3 mAChRs. Thus M3 mAChR antagonists may be useful as therapeutics in these mAChR-mediated diseases.

Despite the large body of evidence supporting the use of anti-muscarinic receptor therapy for treatment of airway disease states, relatively few anti-muscarinic compounds are in use in the clinic for pulmonary indications. Thus, there remains a need for novel compounds that are capable of causing blockade at M3 muscarinic receptors, especially those compounds with a long duration of action, enabling a once-daily dosing regimen. Since muscarinic receptors are widely distributed throughout the body, the ability to deliver anticholinergic drugs directly to the respiratory tract is advantageous as it allows lower doses of the drug to be administered. The design and use of topically active drugs with a long duration of action and that are retained on the receptor or in the lung would allow reduction of unwanted side effects that could be seen with systemic administration of the same drugs.

Tiotropium (Spiriva ™) is a long-acting muscarinic antagonist currently marketed for the treatment of chronic obstructive pulmonary disease, administered by the inhaled route.

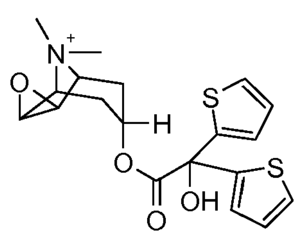

Tiotropium

Additionally ipratropium is a muscarinic antagonist marketed for the treatment of COPD.

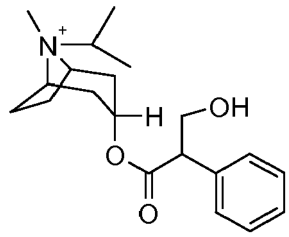

Ipratropium

Chem. Pharm. Bull. 27 (12) 3149-3152 (1979 ) and J. Pharm. Sci 69 (5) 534-537 (1980) describe furyl derivatives as possessing atropine-like activities.
Med. Chem. Res 10 (9), 615-633 (2001) describes isoxazoles and $\Delta^2$-isoxazolines as muscarinic antagonists.
WO97/30994 describes oxadiazoles and thiadiazoles as muscarinic receptor antagonists.
EP0323864 describes oxadiazoles linked to a mono- or bicyclic ring as muscarinic receptor modulators.

The class of β2 adrenergic receptor agonists is well known. Many known β2-agonists, in particular, long-acting β2-agonists such as salmeterol and formoterol, have a role in the treatment of asthma and COPD. These compounds are also generally administered by inhalation. Compounds currently under evaluation as once-daily β2 agonists are described in Expert Opin. Investig. Drugs 14 (7), 775-783 (2005). A well known β2-agonist pharmacophore is the moiety:

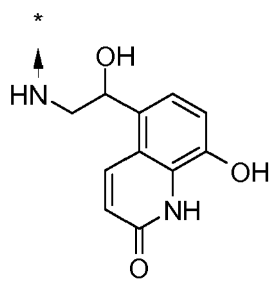

Also known in the art are pharmaceutical compositions that contain both a muscarinic antagonist and a β2-agonist for use in the treatment of respiratory disorders. For example, US2005/0025718 describes a β2-agonist in combination with tiotropium, oxotropium, ipratropium and other muscarinic antagonists; WO02/060532 describes the combination of ipratropium with β2-agonists and WO02/060533 describes the combination of oxotropium with β2-agonists. Other M3 antagonist / β2-agonist combinations are described in WO04/10575 and WO03/087097.

Also known in the art are compounds possessing both muscarinic receptor antagonist and β2-agonist activity present in the same molecule. Such bifunctional molecules provide bronchodilation through two separate modes of action whilst possessing single molecule pharmacokinetics. Such a molecule should be easier to formulate for therapeutic use as compared to two separate compounds and could be more easily co-formulated with a third active ingredient, for example a steroid. Such molecules are described in for example, WO04/074246, WO04/089892, WO05/11104, WO06/023457 and WO06/023460, all of which use different linker radicals for covalently linking the M3 antagonist to the β2-agonist, indicating that the structure of the linker radical is not critical to preserve both activities. This is not surprising since the molecule is not required to interact with the M3 and β2 receptors simultaneously.

Summary of the Invention

According to the invention, there is provided a compound of formula (I):

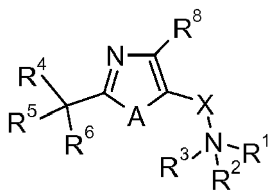

(I)

wherein

(i) **$R^1$** is $C_1$-$C_6$-alkyl or hydrogen; and **$R^2$** is hydrogen or a group -$R^7$, -Z-Y-$R^7$, -Z-$NR^9R^{10}$; -Z-CO-$NR^9R^{10}$, -Z-$NR^9$-C(O)O-$R^7$, or;-Z-C(O)-$R^7$; and **$R^3$** is a lone pair, or $C_1$-$C_6$-alkyl; **or**
(ii) **$R^1$ and $R^3$** together with the nitrogen to which they are attached form a heterocycloalkyl ring, and $R^2$ is a lone pair or a group -$R^7$, -Z-Y-$R^7$, -Z-$NR^9R^{10}$, -Z-CO-$NR^9R^{10}$, -Z-$NR^9$-C(O)O-$R^7$; or -Z-C(O)-$R^7$; **or**
(iii) **$R^1$ and $R^2$** together with the nitrogen to which they are attached form a heterocycloalkyl ring, said ring being substituted by a group -Y-$R^7$, -Z-Y-$R^7$, -Z-$NR^9R^{10}$; -Z-CO-$NR^9R^{10}$; -Z-$NR^9$-C(O)O-$R^7$; or; -Z-C(O)-$R^7$; and **$R^3$** is a lone pair, or $C_1$-$C_6$-alkyl;
**$R^4$** and **$R^5$** are independently selected from the group consisting of aryl, aryl-fused-heterocycloalkyl, heteroaryl, $C_1$-$C_6$-alkyl, cycloalkyl;
**$R^6$** is -OH, $C_1$-$C_6$-alkyl, $C_1$-$C_6$-alkoxy hydroxy-$C_1$-$C_6$-alkyl, nitrile, a group $CONR^8{}_2$ or a hydrogen atom;
**A** is an oxygen or a sulfur atom;
**X** is an alkylene, alkenylene or alkynylene group;
**$R^7$** is an $C_1$-$C_6$-alkyl, aryl, aryl-fused-cycloalkyl, aryl-fused-heterocycloalkyl, heteroaryl, aryl($C_1$-$C_8$-alkyl)-, heteroaryl ($C_1$-$C_8$-alkyl)-, cycloalkyl or heterocycloalkyl group;
**$R^8$** is $C_1$-$C_6$-alkyl or a hydrogen atom;
**Z** is a $C_1$-$C_{16}$-alkylene, $C_2$-$C_{16}$-alkenylene or $C_2$-$C_{16}$-alkynylene group;
**Y** is a bond or oxygen atom;
**$R^9$** and $R^{10}$ are independently a hydrogen atom, $C_1$-$C_6$-alkyl, aryl, aryl-fused-heterocycloalkyl, aryl-fused-cycloalkyl, heteroaryl, aryl($C_1$-$C_6$-alkyl)-, or heteroaryl($C_1$-$C_6$-alkyl)- group; or **$R^9$** and **$R^{10}$** together with the nitrogen atom to which they are attached form a heterocyclic ring of 4-8 atoms, optionally containing a further nitrogen or oxygen atom; or a pharmaceutically acceptable salt, solvate, N-oxide or prodrug thereof.

In one subset of the compounds of the invention:

$R^1$ is $C_1$-$C_6$-alkyl or a hydrogen atom; $R^2$ is $C_1$-$C_6$-alkyl, a hydrogen atom or a group -Z-Y-$R^7$ and $R^3$ is a lone pair or $C_1$-$C_6$-alkyl, or
$R^1$ and $R^2$ together with the nitrogen to which they are attached represent a heterocycloalkyl ring, or $R^1$ and $R^3$ together with the nitrogen to which they are attached represent a heterocycloalkyl ring;
$R^4$ and $R^5$ are independently selected from the group consisting of aryl, heteroaryl, $C_1$-$C_6$-alkyl, cycloalkyl;
$R^6$ is -OH, halogen, $C_1$-$C_6$-alkyl, hydroxy-$C_1$-$C_6$-alkyl or a hydrogen atom;
A is an oxygen or a sulfur atom;
X is an alkylene, alkenylene or alkynylene group;
Z is an alkylene, alkenylene or alkynylene group;
Y is a bond or oxygen atom;
$R^7$ is aryl, heteroaryl, heterocycloalkyl.

It will be appreciated that the carbon atom to which $R^4$, $R^5$ and $R^6$ are attached can be an asymmetric centre so compounds of the invention may be in the form of single enantiomers or mixtures of enantiomers.
A preferred class of compounds of the invention consists of quaternary ammonium salts of formula (I) wherein the nitrogen shown in formula (I) is quaternary nitrogen, carrying a positive charge.
Compounds of the invention may be useful in the treatment or prevention of diseases in which activation of muscarinic receptors are implicated, for example the present compounds are useful for treating a variety of indications, including but not limited to respiratory-tract disorders such as chronic obstructive lung disease, chronic bronchitis of all types (including dyspnoea associated therewith), asthma (allergic and non-allergic; 'wheezy-infant syndrome'), adult/acute respiratory distress syndrome (ARDS), chronic respiratory obstruction, bronchial hyperactivity, pulmonary fibrosis, pulmonary emphysema, and allergic rhinitis, exacerbation of airway hyperreactivity consequent to other drug therapy, particularly other inhaled drug therapy, pneumoconiosis (for example aluminosis, anthracosis, asbestosis, chalicosis,

ptilosis, siderosis, silicosis, tabacosis and byssinosis); gastrointestinal-tract disorders such as irritable bowel syndrome, spasmodic colitis, gastroduodenal ulcers, gastrointestinal convulsions or hyperanakinesia, diverticulitis, pain accompanying spasms of gastrointestinal smooth musculature; urinary-tract disorders accompanying micturition disorders including neurogenic pollakisuria, neurogenic bladder, nocturnal enuresis, psychosomatic bladder, incontinence associated with bladder spasms or chronic cystitis, urinary urgency or pollakiuria; motion sickness; and cardiovascular disorders such as vagally induced sinus bradycardia.

For treatment of respiratory conditions, administration by inhalation will often be preferred, and in such cases administration of compounds (I) which are quaternary ammonium salts will often be preferred. In many cases, the duration of action of quaternary ammonium salts of the invention administered by inhalation is may be more than 12, or more than 24 hours for a typical dose. For treatment of gastrointestinal-tract disorders and cardiovascular disorders, administration by the parenteral route, usually the oral route, may be preferred.

Another aspect of the invention is a pharmaceutical composition comprising a compound of the invention and a pharmaceutically acceptable carrier or excipient.

Another aspect of the invention is the use of a compound of the invention for the manufacture of a medicament for the treatment or prevention of a disease or condition in which muscarinic M3 receptor activity is implicated.

Description of Definitions

Unless otherwise qualified in the context in which they are used, the following terms have the following meanings when used herein:

"Acyl" means a -CO-alkyl group in which the alkyl group is as described herein. Exemplary acyl groups include $-COCH_3$ and $-COCH(CH_3)_2$.

"Acylamino" means a -NR-acyl group in which R and acyl are as described herein. Exemplary acylamino groups include $-NHCOCH_3$ and $-N(CH_3)COCH_3$.

"Alkoxy" and "alkyloxy" means an -O-alkyl group in which alkyl is as described below. Exemplary alkoxy groups include methoxy ($-OCH_3$) and ethoxy ($-OC_2H_5$).

"Alkoxycarbonyl" means a -COO-alkyl group in which alkyl is as defined below. Exemplary alkoxycarbonyl groups include methoxycarbonyl and ethoxycarbonyl.

"Alkyl" as a group or part of a group refers to a straight or branched chain saturated hydrocarbon group having from 1 to 12, preferably 1 to 6, carbon atoms, in the chain. Exemplary alkyl groups include methyl, ethyl, 1-propyl and 2-propyl.

"Alkenyl" as a group or part of a group refers to a straight or branched chain hydrocarbon group having from 2 to 12, preferably 2 to 6, carbon atoms and one carbon-carbon double bond in the chain. Exemplary alkenyl groups include ethenyl, 1-propenyl, and 2-propenyl.

"Alkylamino" means a -NH-alkyl group in which alkyl is as defined above. Exemplary alkylamino groups include methylamino and ethylamino.

"Alkylene means an -alkyl- group in which alkyl is as defined previously. Exemplary alkylene groups include $-CH_2-$, $-(CH_2)_2-$ and $-C(CH_3)HCH_2-$.

"Alkenylene" means an -alkenyl- group in which alkenyl is as defined previously. Exemplary alkenylene groups include -CH=CH-, $-CH=CHCH_2-$, and - $CH_2CH=CH-$.

"Alkynylene" means an -alkynyl- group in which -alkynyl- refers to a straight or branched chain hydrocarbon group having from 2 to 12, preferably 2 to 6, carbon atoms and one carbon-carbon triple bond in the chain. Exemplary alkynylene groups include ethynyl and propargyl.

"Alkylsulfinyl" means a -SO-alkyl group in which alkyl is as defined above. Exemplary alkylsulfinyl groups include methylsulfinyl and ethylsulfinyl.

"Alkylsulfonyl" means a $-SO_2$-alkyl group in which alkyl is as defined above. Exemplary alkylsulfonyl groups include methylsulfonyl and ethylsulfonyl.

"Alkylthio" means a -S-alkyl group in which alkyl is as defined above. Exemplary alkylthio groups include methylthio and ethylthio.

"Aminoacyl" means a -CO-NRR group in which R is as herein described. Exemplary aminoacyl groups include $-CONH_2$ and $-CONHCH_3$.

"Aminoalkyl" means an alkyl-$NH_2$ group in which alkyl is as previously described. Exemplary aminoalkyl groups include $-CH_2NH_2$.

"Aminosulfonyl" means a $-SO_2$-NRR group in which R is as herein described. Exemplary aminosulfonyl groups include $-SO_2NH_2$ and $-SO_2NHCH_3$.

"Aryl" as a group or part of a group denotes an optionally substituted monocyclic or multicyclic aromatic carbocyclic moiety of from 6 to 14 carbon atoms, preferably from 6 to 10 carbon atoms, such as phenyl or naphthyl. The aryl

group may be substituted by one or more substituent groups.

"Arylalkyl" means an aryl-alkyl- group in which the aryl and alkyl moieties are as previously described. Preferred arylalkyl groups contain a $C_{1-4}$ alkyl moiety. Exemplary arylalkyl groups include benzyl, phenethyl and naphthlenemethyl.

"Arylalkyloxy" means an aryl-alkyloxy- group in which the aryl and alkyloxy moieties are as previously described. Preferred arylalkyloxy groups contain a $C_{1-4}$ alkyl moiety. Exemplary arylalkyl groups include benzyloxy.

"Aryl-fused-cycloalkyl" means a monocyclic aryl ring, such as phenyl, fused to a cycloalkyl group, in which the aryl and cycloalkyl are as described herein. Exemplary aryl-fused-cycloalkyl groups include tetrahydronaphthyl and indanyl. The aryl and cycloalkyl rings may each be substituted by one or more substituent groups. The aryl-fused-cycloalkyl group may be attached to the remainder of the compound by any available carbon atom.

"Aryl-fused-heterocycloalkyl" means a monocyclic aryl ring, such as phenyl, fused to a heterocycloalkyl group, in which the aryl and heterocycloalkyl are as described herein. Exemplary aryl-fused-heterocycloalkyl groups include tetrahydroquinolinyl, indolinyl, benzodioxinyl, benxodioxolyl, dihydrobenzofuranyl and isoindolonyl. The aryl and heterocycloalkyl rings may each be substituted by one or more substituent groups. The aryl-fused-heterocycloalkyl group may be attached to the remainder of the compound by any available carbon or nitrogen atom.

"Aryloxy" means an -O-aryl group in which aryl is described above. Exemplary aryloxy groups include phenoxy.

"Cyclic amine" means an optionally substituted 3 to 8 membered monocyclic cycloalkyl ring system where one of the ring carbon atoms is replaced by nitrogen, and which may optionally contain an additional heteroatom selected from O, S or NR (where R is as described herein). Exemplary cyclic amines include pyrrolidine, piperidine, morpholine, piperazine and N-methylpiperazine. The cyclic amine group may be substituted by one or more substituent groups.

"Cycloalkyl" means an optionally substituted saturated monocyclic or bicyclic ring system of from 3 to 12 carbon atoms, preferably from 3 to 8 carbon atoms, and more preferably from 3 to 6 carbon atoms. Exemplary monocyclic cycloalkyl rings include cyclopropyl, cyclopentyl, cyclohexyl and cycloheptyl. The cycloalkyl group may be substituted by one or more substituent groups.

"Cycloalkylalkyl" means a cycloalkyl-alkyl- group in which the cycloalkyl and alkyl moieties are as previously described. Exemplary monocyclic cycloalkylalkyl groups include cyclopropylmethyl, cyclopentylmethyl, cyclohexylmethyl and cycloheptylmethyl.

"Dendrimer" means a multifunctional core group with a branching group attached to each functional site. Each branching site can be attached to another branching molecule and this process may be repeated multiple times.

"Dialkylamino" means a $-N(alkyl)_2$ group in which alkyl is as defined above. Exemplary dialkylamino groups include dimethylamino and diethylamino.

"Halo" or "halogen" means fluoro, chloro, bromo, or iodo. Preferred are fluoro or chloro.

"Haloalkoxy" means an -O-alkyl group in which the alkyl is substituted by one or more halogen atoms. Exemplary haloalkyl groups include trifluoromethoxy and difluoromethoxy.

"Haloalkyl" means an alkyl group which is substituted by one or more halo atoms. Exemplary haloalkyl groups include trifluoromethyl.

"Heteroaryl" as a group or part of a group denotes an optionally substituted aromatic monocyclic or multicyclic organic moiety of from 5 to 14 ring atoms, preferably from 5 to 10 ring atoms, in which one or more of the ring atoms is/are element(s) other than carbon, for example nitrogen, oxygen or sulfur. Examples of such groups include benzimidazolyl, benzoxazolyl, benzothiazolyl, benzofuranyl, benzothienyl, furyl, imidazolyl, indolyl, indolizinyl, isoxazolyl, isoquinolinyl, isothiazolyl, oxazolyl, oxadiazolyl, pyrazinyl, pyridazinyl, pyrazolyl, pyridyl, pyrimidinyl, pyrrolyl, quinazolinyl, quinolinyl, tetrazolyl, 1,3,4-thiadiazolyl, thiazolyl, thienyl and triazolyl groups. The heteroaryl group may be substituted by one or more substituent groups. The heteroaryl group may be attached to the remainder of the compound of the invention by any available carbon or nitrogen atom.

"Heteroarylalkyl" means a heteroaryl-alkyl- group in which the heteroaryl and alkyl moieties are as previously described. Preferred heteroarylalkyl groups contain a lower alkyl moiety. Exemplary heteroarylalkyl groups include pyridylmethyl.

"Heteroarylalkyloxy" means a heteroaryl-alkyloxy- group in which the heteroaryl and alkyloxy moieties are as previously described. Preferred heteroarylalkyloxy groups contain a lower alkyl moiety. Exemplary heteroarylalkyloxy groups include pyridylmethyloxy.

"Heteroaryloxy" means a heteroaryloxy- group in which the heteroaryl is as previously described. Exemplary heteroaryloxy groups include pyridyloxy.

"Heteroaryl-fused-cycloalkyl" means a monocyclic heteroaryl group, such as pyridyl or furanyl, fused to a cycloalkyl group, in which heteroaryl and cycloalkyl are as previously described. Exemplary heteroaryl-fused-cycloalkyl groups include tetrahydroquinolinyl and tetrahydrobenzofuranyl. The heteroaryl and cycloalkyl rings may each be substituted by one or more substituent groups. The heteroaryl-fused-cycloalkyl group may be attached to the remainder of the compound by any available carbon or nitrogen atom.

"Heteroaryl-fused-heterocycloalkyl" means a monocyclic heteroaryl group, such as pyridyl or furanyl, fused to a

heterocycloalkyl group, in which heteroaryl and heterocycloalkyl are as previously described. Exemplary heteroaryl-fused-heterocycloalkyl groups include dihydrodioxinopyridinyl, dihydropyrrolopyridinyl, dihydrofuranopyridinyl and dioxolopyridinyl. The heteroaryl and heterocycloalkyl rings may each be substituted by one or more substituents groups. The heteroaryl-fused-heterocycloalkyl group may be attached to the remainder of the compound by any available carbon or nitrogen atom.

"Heterocycloalkyl" means: (i) an optionally substituted cycloalkyl group of from 4 to 8 ring members which contains one or more heteroatoms selected from O, S or NR; (ii) a cycloalkyl group of from 4 to 8 ring members which contains CONR and CONRCO (examples of such groups include succinimidyl and 2-oxopyrrolidinyl). The heterocycloalkyl group may be substituted by one or more substituent groups. The heterocycloalkyl group may be attached to the remainder of the compound by any available carbon or nitrogen atom.

"Heterocycloalkylalkyl" means a heterocycloalkyl-alkyl- group in which the heterocycloalkyl and alkyl moieties are as previously described.

"Lower alkyl" as a group means unless otherwise specified, an aliphatic hydrocarbon group which may be straight or branched having 1 to 4 carbon atoms in the chain, i.e. methyl, ethyl, propyl (propyl or iso-propyl) or butyl (butyl, iso-butyl or *tert*-butyl).

"Sulfonyl" means a $-SO_2$-alkyl group in which alkyl is as described herein. Exemplary sulfonyl groups include methanesulfonyl.

"Sulfonylamino" means a -NR-sulfonyl group in which R and sulfonyl are as described herein. Exemplary sulfonylamino groups include $-NHSO_2CH_3$. R means alkyl, aryl, or heteroaryl as described herein.

"Pharmaceutically acceptable salt" means a physiologically or toxicologically tolerable salt and includes, when appropriate, pharmaceutically acceptable base addition salts, pharmaceutically acceptable acid addition salts, and pharmaceutically acceptable quaternary ammonium salts. For example (i) where a compound of the invention contains one or more acidic groups, for example carboxy groups, pharmaceutically acceptable base addition salts that may be formed include sodium, potassium, calcium, magnesium and ammonium salts, or salts with organic amines, such as, diethylamine, N-methyl-glucamine, diethanolamine or amino acids (e.g. lysine) and the like; (ii) where a compound of the invention contains a basic group, such as an amino group, pharmaceutically acceptable acid addition salts that may be formed include hydrochlorides, hydrobromides, sulfates, phosphates, acetates, citrates, lactates, tartrates, mesylates, maleates, fumarates, succinates and the like; (iii) where a compound contains a quaternary ammonium group acceptable counter-ions may be, for example, chlorides, bromides, sulfates, methanesulfonates, benzenesulfonates, toluenesulfonates (tosylates), phosphates, acetates, citrates, lactates, tartrates, mesylates, maleates, fumarates, succinates and the like.

It will be understood that, as used herein, references to the compounds of the invention are meant to also include the pharmaceutically acceptable salts.

"Prodrug" refers to a compound which is convertible *in vivo* by metabolic means (e.g. by hydrolysis, reduction or oxidation) to a compound of the invention. Suitable groups for forming pro-drugs are described in 'The Practice of Medicinal Chemistry, 2nd Ed. pp561-585 (2003) and in F. J. Leinweber, Drug Metab. Res., , 18, 379. (1987)

It will be understood that, as used in herein, references to the compounds of the invention are meant to also include the prodrug forms.

"Saturated" pertains to compounds and/or groups which do not have any carbon-carbon double bonds or carbon-carbon triple bonds.

The cyclic groups referred to above, namely, aryl, heteroaryl, cycloalkyl, aryl-fused-cycloalkyl, heteroaryl-fused-cycloalkyl, heterocycloalkyl, aryl-fused-heterocycloalkyl, heteroaryl-fused-heterocycloalkyl and cyclic amine may be substituted by one or more substituent groups. Suitable optional substituent groups include acyl (e.g. $-COCH_3$), alkoxy (e.g., $-OCH_3$), alkoxycarbonyl (e.g. $-COOCH_3$), alkylamino (e.g. $-NHCH_3$), alkylsulfinyl (e.g. $-SOCH_3$), alkylsulfonyl (e.g. $-SO_2CH_3$), alkylthio (e.g. $-SCH_3$), $-NH_2$, aminoacyl (e.g. $-CON(CH_3)_2$), aminoalkyl (e.g. $- CH_2NH_2$), arylalkyl (e.g. $-CH_2Ph$ or $-CH_2-CH_2-Ph$), cyano, dialkylamino (e.g. $-N(CH_3)_2$), halo, haloalkoxy (e.g. $-OCF_3$ or $-OCHF_2$), haloalkyl (e.g. $-CF_3$), alkyl (e.g. $- CH_3$ or $-CH_2CH_3$), -OH, -CHO, $-NO_2$, aryl (optionally substituted with alkoxy, haloalkoxy, halogen, alkyl or haloalkyl), heteroaryl (optionally substituted with alkoxy, haloalkoxy, halogen, alkyl or haloalkyl), heterocycloalkyl, aminoacyl (e.g. $-CONH_2$, $- CONHCH_3$), aminosulfonyl (e.g. $-SO_2NH_2$, $-SO_2NHCH_3$), acylamino (e.g. $-NHCOCH_3$), sulfonylamino (e.g. $-NHSO_2CH_3$), heteroarylalkyl, cyclic amine (e.g. morpholine), aryloxy, heteroaryloxy, arylalkyloxy (e.g. benzyloxy) and heteroarylalkyloxy.

Alkylene or alkenylene groups may be optionally substituted. Suitable optional substituent groups include alkoxy (e.g., $-OCH_3$), alkylamino (e.g. $-NHCH_3$), alkylsulfinyl (e.g. $-SOCH_3$), alkylsulfonyl (e.g. $-SO_2CH_3$), alkylthio (e.g. $-SCH_3$), $-NH_2$, aminoalkyl (e.g. $-CH_2NH_2$), arylalkyl (e.g. $-CH_2Ph$ or $-CH_2-CH_2-Ph$), cyano, dialkylamino (e.g. $-N(CH_3)_2$), halo, haloalkoxy (e.g. $-OCF_3$ or$-OCHF_{2)}$, haloalkyl (e.g. $-CF_3$), alkyl (e.g. $-CH_3$ or $-CH_2CH_3$), -OH, -CHO, and $-NO_2$.

Compounds of the invention may exist in one or more geometrical, optical, enantiomeric, diastereomeric and tautomeric forms, including but not limited to cis- and trans-forms, E- and Z-forms, R-, S- and meso-forms, keto-, and

enol-forms. Unless otherwise stated a reference to a particular compound includes all such isomeric forms, including racemic and other mixtures thereof. Where appropriate such isomers can be separated from their mixtures by the application or adaptation of known methods (e.g. chromatographic techniques and recrystallisation techniques). Where appropriate such isomers may be prepared by the application of adaptation of known methods (e.g. asymmetric synthesis).

**The groups $R^1$, $R^2$ and $R^3$**

There are three combinations of groups $R^1$, $R^2$ and $R^3$
In combination (i) $\mathbf{R^1}$ is $C_1$-$C_6$-alkyl or hydrogen; and $\mathbf{R^2}$ is hydrogen or a group -$R^7$, -Z-Y-$R^7$, -Z-N$R^9R^{10}$, -Z-CO-N$R^9R^{10}$, -Z-N$R^9$-C(O)O-$R^7$ or -Z-C(O)-$R^7$; and $\mathbf{R^3}$ is a lone pair, or $C_{1-}C_6$-alkyl in which case the nitrogen atom to which it is attached is a quaternary nitrogen and carries a positive charge.

In combination (ii) **$R^1$ and $R^3$** together with the nitrogen to which they are attached form a heterocycloalkyl ring, and $\mathbf{R^2}$ is a lone pair (ie the substituent $R^2$ is absent) or a group -$R^7$, -Z-Y-$R^7$, -Z-N$R^9R^{10}$, -Z-CO-N$R^9R^{10}$, -Z-N$R^9$-C(O)O-$R^7$ or -Z-C(O)-$R^7$. In this case, of course, when $R^2$ is other than a lone pair, the nitrogen atom to which it is attached is a quaternary nitrogen and carries a positive charge. In particular **$R^1$ and $R^3$** together with the nitrogen to which they are attached may form a monocyclic ring of from 3 to 7 ring atoms, in which the hetero-atoms are nitrogen. Examples of such rings include azetidinyl, piperidinyl, piperazinyl, N-substituted piperazinyl such as methylpiperazinyl, and pyrrolidinyl rings.
In combination (iii) **$R^1$ and $R^2$** together with the nitrogen to which they are attached form a heterocycloalkyl ring, said ring being substituted by a group -Y-R , -Z-Y-$R^7$,- Z-N$R^9R^{10}$, -Z-CO-N$R^9R^{10}$, -Z-N$R^9$-C(O)O-$R^7$ or -Z-C(O)-$R^7$ and $\mathbf{R^3}$ is a lone pair (ie the substituent $R^3$ is absent), or $C_1$-$C_6$-alkyl especially methyl. In particular **$R^1$ and $R^2$** together with the nitrogen to which they are attached may form a monocyclic ring of from 3 to 7 ring atoms, in which the hetero-atoms are nitrogen. Examples of such rings include azetidinyl, piperidinyl, piperazinyl, N-substituted piperazinyl such as methylpiperazinyl, and pyrrolidinyl rings Of course, when $R^3$ is other than a lone pair, the nitrogen atom to which it is attached is a quaternary nitrogen and carries a positive charge.
Where a group -$R^7$, or -Y-$R^7$, -Z-Y-$R^7$, or a group -Z-N$R^9R^{10}$; or a group -Z-CO-N$R^9R^{10}$; or a group -Z-N$R^9$-C(O)O-$R^7$ or a group -Z-$CO_2$-$R^7$; is present in $R^2$, or the ring formed by $R^1$, $R^2$ and the nitrogen to which they are attached:

Z may be, for example -$(CH_2)_{1-8}$- the latter being optionally substituted on up to three carbons in the chain by methyl;
Y is a bond or -O-;
$R^7$ may be

- $C_1$-$C_6$-alkyl, such as methyl, ethyl, n- or isopropyl, n-, sec- or tertbutyl;
- Optionally substituted aryl such as phenyl or naphthyl, or aryl-fused-heterocycloalkyl such as 3,4-methylenedioxyphenyl, 3,4-ethylenedioxyphenyl, or dihydrobenzofuranyl;
- Optionally substituted heteroaryl such as pyridyl, pyrrolyl, pyrimidinyl, oxazolyl, isoxazolyl, benzisoxazolyl, benzoxazolyl, thiazolyl, benzothiazolyl, quinolyl, thienyl, benzothienyl, furyl, benzofuryl, imidazolyl, benzimidazolyl, isothiazolyl, benzisothiazolyl, pyrazolyl, isothiazolyl, triazolyl, benzotriazolyl, thiadiazolyl, oxadiazolyl, pyridazinyl, pyridazinyl, triazinyl, indolyl and indazolyl;
- Optionally substituted aryl($C_1$-$C_6$-alkyl)- such as those wherein the aryl part is any of the foregoing specifically mentioned aryl groups and the -($C_1$-$C_6$-alkyl)- part is -$CH_2$- or -$CH_2CH_2$-;
- Optionally substituted aryl-fused-cycloalkyl such as indanyl or 1,2,3,4-tetrahydronaphthalenyl;
- Optionally substituted heteroaryl($C_1$-$C_8$-alkyl)- such as those wherein the heteroaryl part is any of the foregoing specifically mentioned heteroaryl groups and the -($C_1$-$C_6$-alkyl)- part is -$CH_2$- or -$CH_2CH_2$-;
- Optionally substituted cycloalkyl such as cyclopropyl, cyclobutyl, cyclopentyl or cyclohexyl; or
- Optionally substituted heterocycloalkyl($C_1$-$C_8$-alkyl)-, such as those wherein the heterocycloalkyl part is azetidinyl, piperidinyl, piperazinyl, N-substituted piperazinyl such as methylpiperazinyl, or pyrrolidinyl and the -($C_1$-$C_6$-alkyl)- part is -$CH_2$- or -$CH_2CH_2$-;

$R^9$ and $R^{10}$ may be independently selected from hydrogen; $C_1$-$C_6$-alkyl such as methyl, ethyl or n- or isopropyl; or any of those optionally substituted aryl, aryl-fused-heterocycloalkyl heteroaryl or aryl($C_1$-$C_8$-alkyl)- groups specifically mentioned in the discussion of $R^7$ above; or
$R^9$ and $R^{10}$ together with the nitrogen atom to which they are attached may form a heterocyclic ring of 4-8 ring atoms, preferably 4-6 ring atoms optionally containing a further nitrogen or oxygen atom, such as azetidinyl, piperidinyl, piperazinyl, N-substituted piperazinyl such as methylpiperazinyl, pyrrolidinyl, morpholinyl, and thiomorpholinyl.

In one preferred embodiment of the invention, in the group $-NR^1R^2R^3$, $R^3$ is methyl or ethyl, $R^2$ is $-Z-NR^9R^{10}$ or $-Z-Y-R^7$ as defined and discussed above, Y is a bond or-O- and -Z- is a straight or branched alkylene radical linking the nitrogen and $-NR^9R^{10}$ or
$-YR^7$ by a chain of up to 16, for example up to 10, carbon atoms, and $R^3$ is methyl, so that the nitrogen is quaternised and carries a positive charge. In these cases, $R^7$ is preferably a cyclic lipophilic group such as phenyl, benzyl, dihydrobenzofuryl or phenylethyl and $R^9$ and $R^{10}$ are as defined and discussed above.

In another preferred embodiment of the invention, in the group $-NR^1R^2R^3$, $R^2$ is $-Z-NR^9R^{10}$ or $-Z-Y-R^7$ as defined and discussed above, Y is a bond or -O-, and -Z- is a straight or branched alkylene radical linking the nitrogen and $-NR^9R^{10}$ or $-YR^7$ by a chain of up to 16, for example up to 10, carbon atoms, and $R^1$ and $R^3$ together with the nitrogen to which they are attached form a heterocyclic ring of 4-8 ring atoms, preferably 4-6 ring atoms optionally containing a further nitrogen or oxygen atom, such as azetidinyl, piperidinyl, piperazinyl, N-substituted piperazinyl such as methylpiperazinyl, pyrrolidinyl, morpholinyl, or thiomorpholinyl ring, so that the nitrogen is quaternised and carries a positive charge. In these cases, $R^7$ is preferably a cyclic lipophilic group such as phenyl, benzyl, dihydrobenzofuryl or phenylethyl; $R^9$ and $R^{10}$ are as defined above. In one subset of compounds of this embodiment, $R^1$ and $R^3$ together with the nitrogen to which they are attached form a piperidinyl or pyrrolidinyl ring.

**The groups $R^4$, $R^5$ and $R^6$**

$R^4$ and $R^5$ may be independently selected from any of those aryl, aryl-fused-heterocycloalkyl, aryl-fused-cycloalkyl, heteroaryl, $C_1$-$C_6$-alkyl, or cycloalkyl groups specifically mentioned in the discussion of $R^5$ above. $R^6$ may be -OH, a hydrogen atom, $C_1$-$C_6$-alkyl such as methyl or ethyl, $C_1$-$C_6$-alkoxy such as methoxy or ethoxy, hydroxy-$C_1$-$C_6$-alkyl such as hydroxymethyl, nitrile, or a group $CONR^8{}_2$ wherein each $R^8$ is independently $C_1$-$C_6$-alkyl such as methyl or ethyl, or a hydrogen atom. Presently preferred is the case where $R^6$ is -OH. Preferred combinations of $R^4$ and $R^5$, especially when $R^6$ is -OH, include those wherein (i) each of $R^4$ and $R^5$ is optionally substituted monocyclic heteroaryl of 5 or 6 ring atoms such as pyridyl, oxazolyl, thiazolyl, furyl and especially thienyl such a 2-thienyl; (ii) each of $R^4$ and $R^5$ is optionally substituted phenyl; (iii) one of $R^4$ and $R^5$ is optionally substituted phenyl and the other is cycloalkyl such as cyclopropyl, cyclobutyl, or especially cyclopentyl or cyclohexyl; and (iv) one of $R^4$ and $R^5$ is optionally substituted monocyclic heteroaryl of 5 or 6 ring atoms such as pyridyl, thienyl, oxazolyl, thiazolyl, or furyl; and the other is cycloalkyl such as cyclopropyl, cyclobutyl, cyclopentyl or cyclohexyl.

**The ring atom A**

A may be an oxygen or sulphur atom.

**The substituent $R^8$**

Although $R^3$ may be $C_1$-$C_6$-alkyl, such as methyl or ethyl, it is currently preferred that $R^8$ is a hydrogen atom.

**The Radical X**

Although X may be an alkylene, alkenylene or alkynylene radical, it is currently preferred that it be alkylene, for example ethylene or methylene.

A preferred subclass of compounds with which the invention is concerned consists of those of formula (IA)

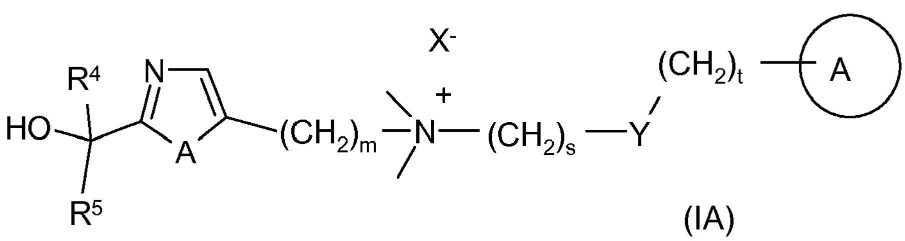

(IA)

wherein A is -O- or -S-; m is 1 or 2; ring A is an optionally substituted phenyl ring, or monocyclic heterocyclic ring of 5 or 6 ring atoms, or phenyl-fused-heterocycloalkyl ring system wherein the heterocycloalkyl ring is a monocyclic heterocyclic ring of 5 or 6 ring atoms; $R^4$ is phenyl, thienyl, cyclopentyl or cyclohexyl; $R^5$ is phenyl; thienyl, cyclopentyl or cyclohexyl; s is 1, 2, 3, 4, 5, 6 or 7 and t is 0, 1, 2, 3, 4, 5, 6 or 7 provided that s+t is not greater than 16; Y is a bond or

-O-, and X- is a pharmaceutically acceptable anion.

Another preferred subclass of compounds with which the invention is concerned consists of those of formula (IB)

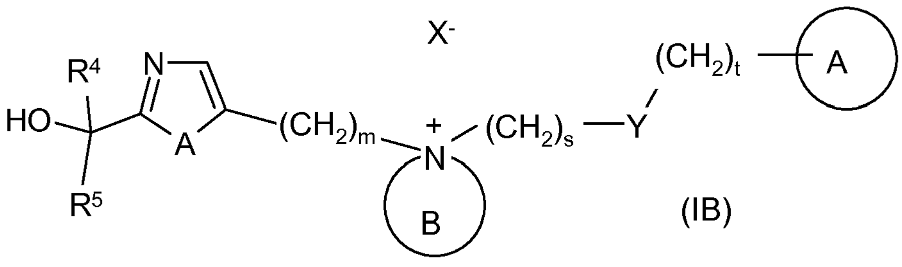

(IB)

wherein A is -O- or -S-; m is 1 or 2; ring B is a pyrrolidinium or piperidinium ring; ring A is an optionally substituted phenyl ring, or monocyclic heterocyclic ring of 5 or 6 ring atoms, or phenyl-fused-heterocycloalkyl ring system wherein the heterocycloalkyl ring is a monocyclic heterocyclic ring of 5 or 6 ring atoms; $R^4$ is phenyl, thienyl, cyclopentyl or cyclohexyl; $R^5$ is phenyl; thienyl, cyclopentyl or cyclohexyl; s is 1, 2, 3, 4, 5, 6 or 7 and t is 0, 1, 2, 3, 4, 5, 6 or 7 provided that s+t is not greater than 16; Y is a bond or-O-, and X- is a pharmaceutically acceptable anion.

Another preferred subclass of compounds with which the invention is concerned consists of those of formula (IC)

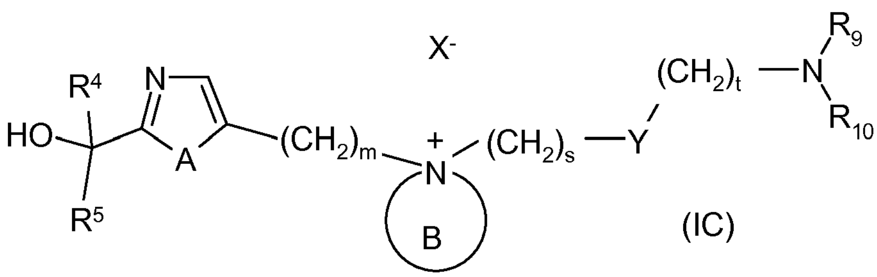

(IC)

wherein A is -O- or -S-; m is 1 or 2; ring B is a pyrrolidinium or piperidinium ring; $R^4$ is phenyl, thienyl, cyclopentyl or cyclohexyl; $R^5$ is phenyl; thienyl, cyclopentyl or cyclohexyl; $R^9$ and $R^{10}$ are independently a hydrogen atom, or optionally substituted $C_1$-$C_6$-alkyl or aryl, such as optionally substituted phenyl; s is 1, 2, 3, 4, 5, 6 or 7 and t is 0, 1, 2, 3, 4, 5, 6 or 7 provided that s+t is not greater than 16; Y is a bond or -O-, and X- is a pharmaceutically acceptable anion.

In compounds (IA) and (IB), it is currently preferred that ring A is (i) optionally substituted phenyl, wherein optional substituents are selected from alkoxy, halo especially fluoro or chloro, $C_1$-$C_3$-alkyl, amino $C_1$-$C_3$-acyl, amino $C_1$-$C_3$-alkyl, and, or (ii) a phenyl-fused-heterocycloalkyl ring system wherein the heterocycloalkyl ring is a monocyclic heterocyclic ring of 5 or 6 ring atoms, such as dihydrobenzofuranyl.

In each of subclasses (IA), (IB) and (IC), s+t may be, for example 1, 2, 3, 4, 5, 6, or 7 and may arise from suitable combinations of t and s such as where t is 0, 1, 2, 3, 4, 5 or 6 and s is 1, 2, 3, 4, 5, 6 or 7.

In compounds (IA) and (IB), a currently preferred combination of t, Y and s is where t is 0, s is 3, and Y is -O-. A further currently preferred combination is where Y is a bond and s+t is 2, 3 or 4.

In compounds (IC) a currently preferred combination of t, Y and s is where Y is a bond and s+t is 8, 9 or 10.

It will be appreciated that certain combinations of $R^4$, $R^5$ and $R^6$ can give rise to optical enantiomers. In such cases, both enantiomers of the invention generally exhibit affinity at the $M_3$ receptor, although one enantiomer is generally preferred on criteria of potency at the $M_3$ receptor and/or selectivity against the $M_2$ receptor. In some embodiments of the invention, the absolute stereochemistry of the preferred enantiomer is known. For example, in one preferred embodiment $R^4$ is a phenyl group; $R^5$ is a cyclohexyl or cyclopentyl group; $R^6$ is a hydroxyl group; and the carbon atom to which they are attached has the R- absolute configuration as dictated by Cahn-Ingold-Prelog rules.

Examples of compounds of the invention include those of the Examples herein. Preferred compounds of the invention include:

[2-(Hydroxy-diphenyl-methyl)-oxazol-5-ylmethyl]-dimethyl-(3-phenoxy-propyl)-ammonium salts
[2-((R)-Cyclohexyl-hydroxy-phenyl-methyl)-oxazol-5-ylmethyl]-dimethyl-(3-phenoxypropyl)-ammonium salts
[2-((R)-Cyclohexyl-hydroxy-phenyl-methyl)-oxazol-5-ylmethyl]-dimethyl-phenethyl-ammonium salts
[2-((R)-Cyclohexyl-hydroxy-phenyl-methyl)-oxazol-5-ylmethyl]-dimethyl-(4-methylpent-3-enyl)-ammonium salts

[2-((R)-Cyclohexyl-hydroxy-phenyl-methyl)-oxazol-5-ylmethyl]-[2-(2,3-dihydrobenzofuran-5-yl)-ethyl]-dimethyl-ammonium salts
[2-((R)-cyclohexyl-hydroxy-phenyl-methyl)-oxazol-5-ylmethyl]-dimethyl-(6-methylpyridin-2-ylmethyl)-ammonium salts
[2-(Cyclopentyl-hydroxy-phenyl-methyl)-oxazol-5-ylmethyl]-dimethyl-(3-phenoxypropyl)-ammonium salts
1-[2-(Cyclohexyl-hydroxy-phenyl-methyl)-oxazol-5-ylmethyl]-1-(3-phenoxy-propyl)-pyrrolidinium salts
[2-((R)-Cyclohexyl-hydroxy-phenyl-methyl)-oxazol-5-ylmethyl]-dimethyl-(4-phenoxybutyl)-ammonium salts
(2-Benzyloxy-ethyl)-[2-((R)-cyclohexyl-hydroxy-phenyl-methyl)-oxazol-5-ylmethyl]-dimethyl-ammonium salts
[2-((R)-Cyclohexyl-hydroxy-phenyl-methyl)-oxazol-5-ylmethyl]-dimethyl-(4-phenylbutyl)-ammonium salts
[2-((R)-Cyclohexyl-hydroxy-phenyl-methyl)-oxazol-5-ylmethyl]-[3-(4-fluoro-phenoxy)-propyl]-dimethyl-ammonium salts
[2-((R)-cyclohexyl-hydroxy-phenyl-methyl)-oxazol-5-ylmethyl]-dimethyl-(3-phenylpropyl)-ammonium salts
[2-((R)-cyclohexyl-hydroxy-phenyl-methyl)-oxazol-5-ylmethyl]-dimethyl-(2-phenoxyethyl)-ammonium salts
[2-((R)-Cyclohexyl-hydroxy-phenyl-methyl)-oxazol-5-ylmethyl]-dimethyl-(3-p-tolyloxypropyl)-ammonium salts
[3-(4-Chloro-phenoxy)-propyl]-[2-((R)-cyclohexyl-hydroxy-phenyl-methyl)-oxazol-5-ylmethyl]-dimethyl-ammonium salts
[2-((R)-Cyclohexyl-hydroxy-phenyl-methyl)-oxazol-5-ylmethyl]-[3-(3,4-dichlorophenoxy)-propyl]-dimethyl-ammonium salts
[2-((R)-cyclohexyl-hydroxy-phenyl-methyl)-oxazol-5-ylmethyl]-dimethyl-(8-methylamino-octyl)-ammonium salts
[2-((R)-cyclohexyl-hydroxy-phenyl-methyl)-oxazol-5-ylmethyl]-dimethyl-[2-(4-methylaminomethyl-phenyl)-ethyl]-ammonium salts
{2-[2-(Cyclohexyl-hydroxy-phenyl-methyl)-oxazol-5-yl]-ethyl}-dimethyl-(3-phenoxypropyl)-ammonium salts
{2-[2-(Hydroxy-diphenyl-methyl)-oxazol-5-yl]-ethyl}-dimethyl-(3-phenoxy-propyl)-ammonium salts
[2-(Hydroxydiphenylmethyl)thiazol-5-ylmethyl]dimethyl-(3-phenoxypropyl)ammonium salts
(3-Benzyloxypropyl)-[2-((R)-cyclohexyl-hydroxy-phenyl-methyl)-oxazol-5-ylmethyl]-dimethyl-ammonium salts
[2-(4-Chloro-benzyloxy)-ethyl]-[2-((R)-cyclohexyl-hydroxy-phenyl-methyl)-oxazol-5-ylmethyl]-dimethyl-ammonium salts

As referred to in the Background to the Invention section above, compounds with dual M3 receptor antagonist and β2-adrenoreceptor agonist activity are known, and treatment of respiratory disease with such dual activity compounds is a recognised form of treatment. The known strategy for the provision of compounds with such dual activity mechanisms is simple covalent linkage of a compound with M3 receptor antagonist activity to a compound with a β2-adrenoreceptor agonist activity. Such covalent conjugates of an M3 receptor agonist compound (I) as defined and discussed above and a β2-adrenoreceptor agonist also form part of the invention. For example, such dual activity conjugates include compounds of formula (I), as defined and discussed above, modified by replacement of the $R^2$ group by a -L-B group wherein L is a linker radical and B is a moiety having β2 adrenoreceptor agonist activity. Structurally, such dual activity conjugates may be represented as in formula (III):

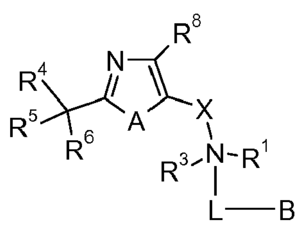
(III)

wherein $R^1$, $R^3$, $R^4$, $R^5$, $R^6$ and $R^8$ are as defined and discussed above in relation to compounds (I) of the invention, L is a divalent linker radical and B is a moiety having β2- adrenoreceptor agonist activity, such as the β2-agonist pharmacophor referred to above in the Background to the Invention section. Such compounds (III) form another aspect of the present invention. An example of such a compound is that of Example No. 77 herein.

The present invention is also concerned with pharmaceutical formulations comprising, as an active ingredient, a compound of the invention. Other compounds may be combined with compounds of this invention for the prevention and treatment of inflammatory diseases of the lung. Thus the present invention is also concerned with pharmaceutical compositions for preventing and treating respiratory-tract disorders such as chronic obstructive lung disease, chronic bronchitis, asthma, chronic respiratory obstruction, pulmonary fibrosis, pulmonary emphysema, and allergic rhinitis comprising a therapeutically effective amount of a compound of the invention and one or more other therapeutic agents.

Other compounds may be combined with compounds of this invention for the prevention and treatment of inflammatory diseases of the lung. Accordingly the invention includes a combination of an agent of the invention as hereinbefore described with one or more anti-inflammatory, bronchodilator, antihistamine, decongestant or anti-tussive agents, said agents of the invention hereinbefore described and said combination agents existing in the same or different pharmaceutical compositions, administered separately or simultaneously. Preferred combinations would have two or three different pharmaceutical compositions. Suitable therapeutic agents for a combination therapy with compounds of the invention include:

One or more other bronchodilators such as PDE3 inhibitors;
Methyl xanthines such as theophylline;
Other muscarinic receptor antagonists;

A corticosteroid, for example fluticasone propionate, ciclesonide, mometasone furoate or budesonide, or steroids described in WO02/88167, WO02/12266, WO02/100879, WO02/00679, WO03/35668, WO03/48181, WO03/62259, WO03/64445, WO03/72592, WO04/39827 and WO04/66920;
A non-steroidal glucocorticoid receptor agonist;
A β2-adrenoreceptor agonist, for example albuterol (salbutamol), salmeterol, metaproterenol, terbutaline, fenoterol, procaterol, carmoterol, indacaterol, formoterol, arformoterol, picumeterol, GSK-159797, GSK-597901, GSK-159802, GSK-64244, GSK-678007, TA-2005 and also compounds of EP1440966, JP05025045, WO93/18007, WO99/64035, US2002/0055651, US2005/0133417, US2005/5159448, WO00/075114, WO01/42193, WO01/83462, WO02/66422, WO02/70490, WO02/76933, WO03/24439, WO03/42160, WO03/42164, WO03/72539, WO03/91204, WO03/99764, WO04/16578, WO04/016601, WO04/22547, WO04/32921, WO04/33412, WO04/37768, WO04/37773, WO04/37807, WO0439762, WO04/39766, WO04/45618, WO04/46083, WO04/71388, WO04/80964, EP1460064, WO04/087142, WO04/89892, EP01477167, US2004/0242622, US2004/0229904, WO04/108675, WO04/108676, WO05/033121, WO05/040103, WO05/044787, WO04/071388, WO05/058299, WO05/058867, WO05/065650, WO05/066140, WO05/070908, WO05/092840, WO\05/092841, WO05/092860, WO05/092887, WO05/092861, WO05/090288, WO05/092087, WO05/080324, WO05/080313, US20050182091, US20050171147, WO05/092870, WO05/077361, DE10258695, WO05/111002, WO05/111005, WO05/110990, US2005/0272769 WO05/110359, WO05/121065, US2006/0019991, WO06/016245, WO06/014704, WO06/031556, WO06/032627, US2006/0106075, US2006/0106213, WO06/051373, WO06/056471;
A leukotriene modulator, for example montelukast, zafirlukast or pranlukast; protease inhibitors, such as inhibitors of matrix metalloprotease for example MMP12 and TACE inhibitors such as marimastat, DPC-333, GW-3333;
Human neutrophil elastase inhibitors, such as sivelestat and those described in WO04/043942, WO05/021509, WO05/021512, WO05/026123, WO05/026124, WO04/024700, WO04/024701, WO04/020410, WO04/020412, WO05/080372, WO05/082863, WO05/082864, WO03/053930;
Phosphodiesterase-4 (PDE4) inhibitors, for example roflumilast, arofylline, cilomilast, ONO-6126 or IC-485;
Phosphodiesterase-7 inhibitors;
An antitussive agent, such as codeine or dextramorphan;
Kinase inhibitors, particularly P38 MAPKinase inhibitors;
P2X7 anatgonists;
iNOS inhibitors;
A non-steroidal anti-inflammatory agent (NSAID), for example ibuprofen or ketoprofen;
A dopamine receptor antagonist;
TNF-α inhibitors, for example anti-TNF monoclonal antibodies, such as Remicade and CDP-870 and TNF receptor immunoglobulin molecules, such as Enbrel;
A2a agonists such as those described in EP1052264 and EP1241176;
A2b antagonists such as those described in WO2002/42298;
Modulators of chemokine receptor function, for example antagonists of CCR1, CCR2, CCR3, CXCR2, CXCR3, CX3CR1 and CCR8, such as SB-332235, SB-656933, SB-265610, SB-225002, MCP-1(9-76), RS-504393, MLN-1202, INCB-3284; Compounds which modulate the action of prostanoid receptors, for example a $PGD_2$ (DP1 or CRTH2), or a thromboxane $A_2$ antagonist eg ramatrobant;
Compounds which modulate Th1 or Th2 function, for example, PPAR agonists; Interleukin 1 receptor antagonists, such as Kineret;
Interleukin 10 agonists, such as Ilodecakin;
HMG-CoA reductase inhibitors (statins); for example rosuvastatin, mevastatin, lovastatin, simvastatin, pravastatin and fluvastatin;
Mucus regulators such as INS-37217, diquafosol, sibenadet, CS-003, talnetant, DNK-333, MSI-1956, gefitinib;
Antiinfective agents (antibiotic or antiviral), and antiallergic drugs including, but not limited to, anti-histamines.

The weight ratio of the first and second active ingredients may be varied and will depend upon the effective dose of each ingredient. Generally, an effective dose of each will be used.

Any suitable route of administration may be employed for providing a mammal, especially a human, with an effective dosage of a compound of the present invention. In therapeutic use, the active compound may be administered by any convenient, suitable or effective route. Suitable routes of administration are known to those skilled in the art, and include oral, intravenous, rectal, parenteral, topical, ocular, nasal, buccal and pulmonary.

The magnitude of prophylactic or therapeutic dose of a compound of the invention will, of course, vary depending upon a range of factors, including the activity of the specific compound that is used, the age, body weight, diet, general health and sex of the patient, time of administration, the route of administration, the rate of excretion, the use of any other drugs, and the severity of the disease undergoing treatment. In general, the daily dose range for inhalation will lie within the range of from about 0.1 μg to about 10 mg per kg body weight of a human, preferably 0.1 μg to about 0.5 mg per kg, and more preferably 0.1μg to 50μg per kg, in single or divided doses. On the other hand, it may be necessary to use dosages outside these limits in some cases. Compositions suitable for administration by inhalation are known, and may include carriers and/or diluents that are known for use in such compositions. The composition may contain 0.01-99% by weight of active compound. Preferably, a unit dose comprises the active compound in an amount of 1μg to 10 mg. For oral administration suitable doses are 10μg per kg to 100mg per kg, preferably 40μg per kg to 4 mg per kg.

Another aspect of the present invention provides pharmaceutical compositions which comprise a compound of the invention and a pharmaceutically acceptable carrier. The term "composition", as in pharmaceutical composition, is intended to encompass a product comprising the active ingredient(s), and the inert ingredient(s) (pharmaceutically acceptable excipients) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the invention, additional active ingredient(s), and pharmaceutically acceptable excipients. The pharmaceutical compositions of the present invention comprise a compound of the invention as an active ingredient or a pharmaceutically acceptable salt thereof, and may also contain a pharmaceutically acceptable carrier and optionally other therapeutic ingredients. The term "pharmaceutically acceptable salts" refers to salts prepared from pharmaceutically acceptable non-toxic bases or acids including inorganic bases or acids and organic bases or acids, and salts of quaternary ammonium compounds with pharmaceutically acceptable counter-ions.

For delivery by inhalation, the active compound is preferably in the form of microparticles. They may be prepared by a variety of techniques, including spray-drying, freeze-drying and micronisation.

By way of example, a composition of the invention may be prepared as a suspension for delivery from a nebuliser or as an aerosol in a liquid propellant, for example for use in a pressurised metered dose inhaler (PMDI). Propellants suitable for use in a PMDI are known to the skilled person, and include CFC-12, HFA-134a, HFA-227, HCFC-22 ($CCl_2F_2$) and HFA-152 ($C_2H_4F_2$) and isobutane.

In a preferred embodiment of the invention, a composition of the invention is in dry powder form, for delivery using a dry powder inhaler (DPI). Many types of DPI are known.

Microparticles for delivery by administration may be formulated with excipients that aid delivery and release. For example, in a dry powder formulation, microparticles may be formulated with large carrier particles that aid flow from the DPI into the lung. Suitable carrier particles are known, and include lactose particles; they may have a mass median aerodynamic diameter of greater than 90 μm.

In the case of an aerosol-based formulation, an example is:

| | |
|---|---|
| Compound of the invention | 24 mg / canister |
| Lecithin, NF Liq. Conc. | 1.2 mg / canister |
| Trichlorofluoromethane, NF | 4.025 g / canister |
| Dichlorodifluoromethane, NF | 12.15 g / canister. |

The active compounds may be dosed as described depending on the inhaler system used. In addition to the active compounds, the administration forms may additionally contain excipients, such as, for example, propellants (e.g. Frigen in the case of metered aerosols), surface-active substances, emulsifiers, stabilizers, preservatives, flavorings, fillers (e.g. lactose in the case of powder inhalers) or, if appropriate, further active compounds.

For the purposes of inhalation, a large number of systems are available with which aerosols of optimum particle size can be generated and administered, using an inhalation technique which is appropriate for the patient. In addition to the use of adaptors (spacers, expanders) and pear-shaped containers (e.g. Nebulator®, Volumatic®), and automatic devices emitting a puffer spray (Autohaler®), for metered aerosols, in particular in the case of powder inhalers, a number of technical solutions are available (e.g. Diskhaler®, Rotadisk®, Turbohaler® or the inhalers for example as described

EP-A-0505321). Additionally, compounds of the invention may be delivered in multi-chamber devices thus allowing for delivery of combination agents.

**Methods of Synthesis**

The compounds of the invention of the present invention can be prepared according to the procedures of the following schemes and examples, using appropriate materials, and are further exemplified by the following specific examples. Moreover, by utilising the procedures described with the disclosure contained herein, one of ordinary skill in the art can readily prepare additional compounds of the present invention claimed herein. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds.

The compounds of the invention may be isolated in the form of their pharmaceutically acceptable salts, such as those described previously herein above. It may be necessary to protect reactive functional groups (e.g. hydroxy, amino, thio or carboxy) in intermediates used in the preparation of compounds of the invention to avoid their unwanted participation in a reaction leading to the formation of the compounds. Conventional protecting groups, for example those described by T. W. Greene and P. G. M. Wuts in "Protective groups in organic chemistry" John Wiley and Sons, 1999, may be used.

Compounds of the invention may be prepared according to the routes illustrated in Schemes 1-4.

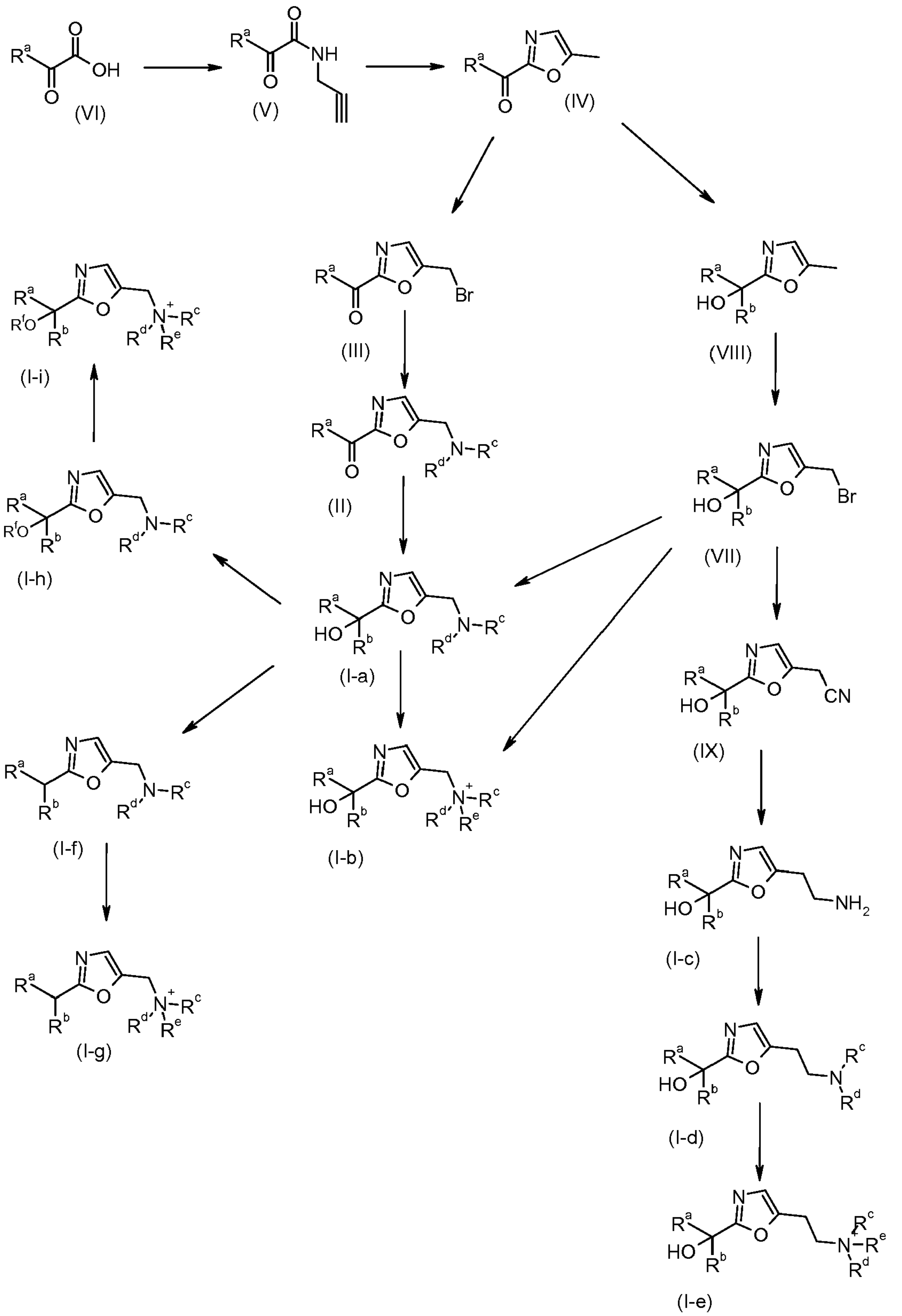

(VI) (V) (IV) (III) (VIII) (I-i) (II) (VII) (I-h) (I-a) (IX) (I-f) (I-b) (I-c) (I-g) (I-d) (I-e)

**Scheme 1**

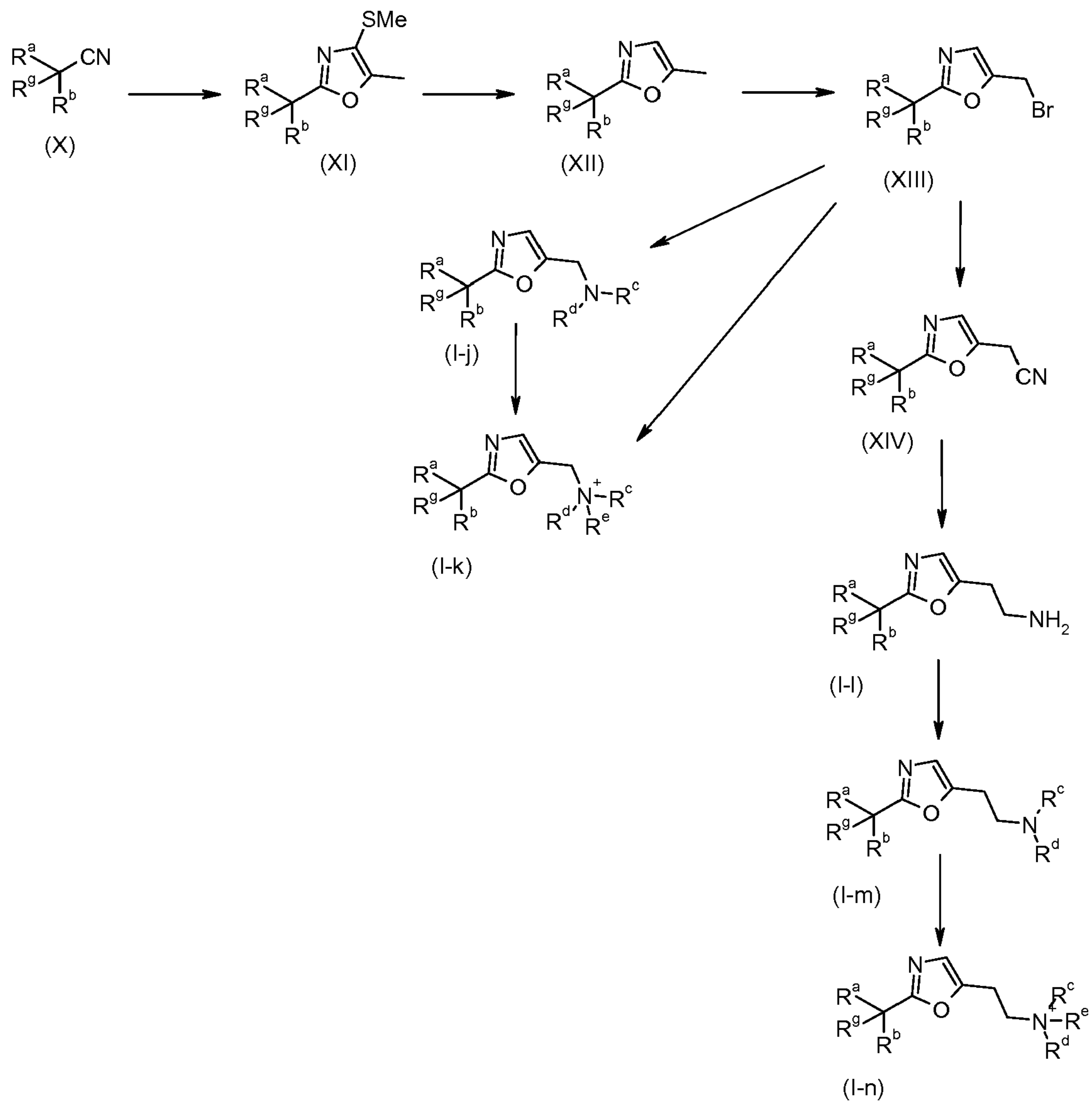

(X) (XI) (XII) (XIII) (I-j) (I-k) (XIV) (I-l) (I-m) (I-n)

**Scheme 2**

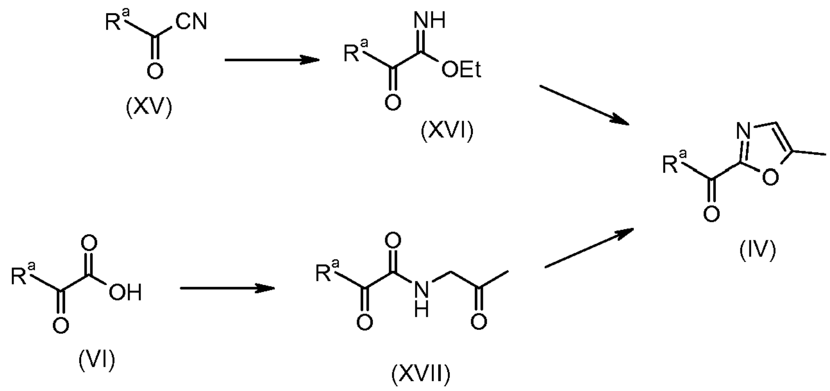

(XV) (XVI) (IV) (VI) (XVII)

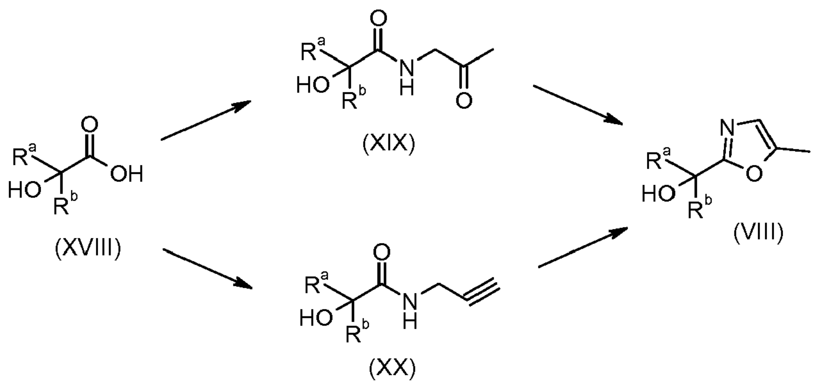

(XIX) (XVIII) (VIII) (XX)

## Scheme 3

Compounds of formula (I-b) wherein $R^c$, $R^d$ and $R^e$ are as defined for $R^1$, $R^2$ and $R^3$ and are not a hydrogen atom can be prepared from compounds of formula (I-a) by reaction with an alkylating agent of formula (XXI):

$R^e$-W (XXI) wherein W is a leaving group such as halogen, tosylate, mesylate. The reaction can be performed in a range of solvents, preferably DMF, chloroform or acetonitrile at a temperature from 0˚C to the reflux temperature of the solvent. In a similar manner, compounds of formula (I-e), (I-g), (I-i), (I-k) and (I-n) may be prepared from compounds of formula (I-d), (I-f), (I-h), (I-j) and (I-m) respectively. Scheme 3

It will be apparent that some compounds can contain a chiral centre and thus exist in enatiomeric forms which can be separated by chiral preparative HPLC techniques using conditions know to those skilled in the art and exemplified below. Compounds of general formula (I-a) may be prepared from compounds of general formula (II):

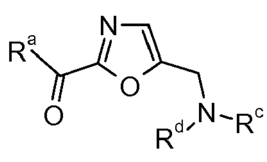

(II)

by reaction with a compound of general formula (XXII):

$R^bM$ (XXII)

wherein $R^a$ and $R^b$ are as defined for $R^4$ and $R^5$ in general formula (I) and M represents a metallic counterion such as Li or MgBr. The reaction may take place in an aprotic organic solvent such as THF or diethyl ether at a range of

temperatures, preferably between -78 ˚C and the reflux temperature of the solvent.

Compounds of general formula (XXII) are well known in the art and are readily available or can be prepared by known methods.

Compounds of general formula (II) can be prepared from compounds of general formula (III):

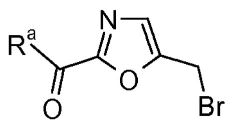

(III)

by reaction with an amine of formula (XXIII):

$R^cR^dNH$ (XXIII)

wherein $R^c$ and $R^d$ are as defined for $R^1$ and $R^2$ in general formula (I). The reaction is performed in a range of solvents, preferably THF/DCM at a range of temperatures, preferably between 0 and 100˚C.

Compounds of general formula (XXIII) are well known in the art and can be prepared by known methods, or are commercially available.

Compounds of formula (III) can be prepared from compounds of general formula (IV):

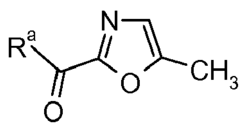

(IV)

by reaction with a brominating agent such as N-bromosuccinimide in the presence of a radical initiator such as AIBN or benzoyl peroxide. The reaction can be carried out in suitable solvents, such as $CCl_4$, at a range of temperatures, preferably between ambient temperature and the reflux temperature of the solvent.

Compounds of formula (IV) can be prepared from compounds of general formula (V):

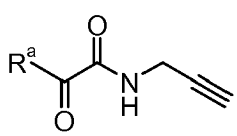

(V)

by reaction with an acid such as hydrochloric acid, sulphuric acid, methanesulfonic or trifluoromethansulfonic acid in a range of solvents such as THF, DCM, water, and preferably 1,4-dioxan at a range of temperatures, preferably between ambient temperature and the reflux temperature of the solvent.

Alternatively compounds of formula (IV) can be prepared from compounds of general formula (V) by palladium-catalysed cyclisation using a palladium catalyst such as bis(dibenzylideneacetone)palladium in the presence of a ligand such as triphenylphosphine and a base such as sodium tert-butoxide in a solvent such as THF from room temperature to the reflux temperature of the solvent.

Alternatively compounds of formula (IV) can be prepared from compounds of formula (XVI):

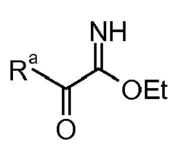

(XVI)

according to the method described in J. Chem. Soc. 1948, 1960. Compounds of general formula (XVI) are known in the

art and can be prepared by known methods such as those described in Tetrahedron 2002, 58(14), 2813.

Alternatively compounds of formula (IV) can be prepared from compounds of formula (XVII):

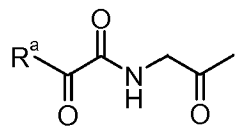

(XVII)

according to the method described in J. Org. Chem., 1938, 2, 319. Compounds of general formula (XVII) are well known in the art and can be prepared by known methods such as those described in GB2214180.

Compounds of general formula (V) can be prepared from compounds of general formula (VI):

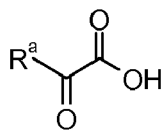

(VI)

by reaction with propargylamine in the presence of a suitable coupling agent, such as DCC/HOBt or many other known coupling methodologies. Alternatively compounds of formula (VI) may be converted to, for example, the acid chloride and amide formation effected optionally in the presence of a suitable non-nucleophilic base and compatible solvent under well known conditions. Compounds of general formula (VI) are readily available or can be prepared by known methods.

Alternatively compounds of general formula (I-a) can be prepared from compounds of general formula (VII):

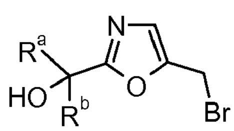

(VII)

according to methods described above for the preparation of compounds of formula (II) from compounds of formula (III)

Compounds of general formula (VII) can be prepared from compounds of formula (VIII):

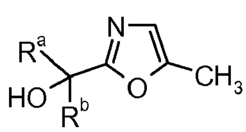

(VIII)

according to methods similar to those used to prepare compounds of formula (III) from compounds of formula (IV) as described above.

Compounds of general formula (VIII) can be prepared from compounds of formula (IV) using methods described above for the preparation of compounds of formula (I-a) from compounds of formula (II).

Alternatively compounds of formula (VIII) may be prepared from compounds of formula (XIX):

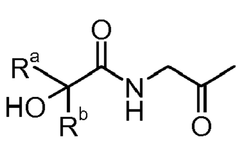

(XIX)

using methods described above for the preparation of compounds of formula (IV) from compounds of formula (XVII). Compounds of general formula (XIX) can be prepared by known methods such as those described in GB2214180.

Alternatively compounds of formula (VIII) may be prepared from compounds of formula (XX):

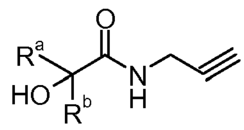

(XX)

using methods described above for the preparation of compounds of formula (IV) from compounds of formula (V).

Compounds of general formula (XX) can be prepared from compounds of formula (XVIII) using methods described above for the preparation of compounds of formula (V) from compounds of formula (VI).

Alternatively, compounds of formula (I-b) may be prepared directly from compounds of formula (VII) by quaternisation with a suitably substituted tertiary amine as described above.

Alternatively compounds of formula (I-a) wherein $-NR^cR^d$ is a secondary amine (i.e. one of $R^c$ or $R^d$ is a hydrogen atom) may be prepared from compounds of formula (I-a) wherein $-NR^cR^d$ is a $-NH_2$ group by reductive alkylation with a suitably substituted aldehyde. The reaction is carried out in the presence of a reducing agent such as sodium cyanoborohydride or sodium borohydride, preferably sodium triacetoxyborohydride in a range of organic solvents, preferably dichloroethane. Compounds of formula (I-d) and (I-e) may be prepared from compounds of formula (I-c) by alkylation or reductive alkylation methods as described above and according to standard methods well-known to those skilled in the art.

Compounds of formula (I-c) can be prepared from compounds of general formula (IX):

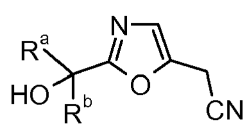

(IX)

by reaction with a reducing agent such as lithium aluminium hydride, diisobutyl aluminium hydride, or borane in a range of aprotic solvents such as diethyl ether, or THF or preferably by hydrogenation in the presence of a catalyst such as Raney Nickel in a suitable solvent such as EtOAc or EtOH at a range of temperatures from room temperature to the reflux temperature of the solvent.

Compounds of general formula (IX) can be prepared from compounds of general formula (VIII) by reaction with a source of cyanide ion such as acetone cyanohydrin or an inorganic cyanide, preferably sodium cyanide, in the presence of a non-nucleophilic base such as tetramethyl guanidine, in a range of solvents, preferably ethanol, at a range of temperatures, preferably between ambient temperature and the reflux temperature of the solvent.

Compounds of formula (I-f) can be prepared from compounds of formula (I-a) by reaction with a reducing agent such as triethylsilane in the presence of an acid such as trifluoroacetic acid in a solvent such as DCM from room temperature to the reflux temperature of the solvent.

Compounds of formula (I-h) can be prepared from compounds of formula (I-a) by reaction with an alkylating agent of formula (XXIV):

$R^fY$ (XXIV)

wherein $R^f$ is as defined for $R^6$ in general formula (I) and Y is a leaving group such as halogen, tosylate, mesylate. The reaction is performed in the presence of a base such as sodium hydride in a solvent such as THF from 0˚C to the reflux temperature of the solvent.

Compounds of general formula (I-m) can be prepared from compounds of formula (I-l) using methods described above for the preparation of compounds of formula (I-d) from compounds of formula (I-c).

Compounds of general formula (I-l) can be prepared from compounds of formula (XIV) using methods described above for the preparation of compounds of formula (I-c) from compounds of formula (IX).

Compounds of general formula (XIV) can be prepared from compounds of formula (XIII) using methods described above for the preparation of compounds of formula (IX) from compounds of formula (VII).

Alternatively, compounds of formula (I-k) may be prepared directly from compounds of formula (XIII) by quaternisation with a suitably substituted tertiary amine as described above.

Compounds of general formula (I-j) can be prepared from compounds of formula (XIII) using methods described above for the preparation of compounds of formula (I-a) from compounds of formula (VII).

Compounds of general formula (XIII) can be prepared from compounds of formula (XII) using methods described above for the preparation of compounds of formula (III) from compounds of formula (IV).

Compounds of general formula (XII) may be prepared from compounds of general formula (XI):

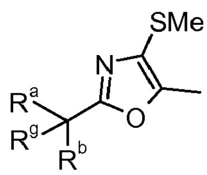

(XI)

by reaction with a reducing agent such as Raney Nickel in a solvent such as ethanol at a temperature from room temperature to the reflux temperature of the solvent according to the method described in J. Org. Chem. 2006, 71(8), 3026. Compounds of general formula (XI) may be prepared from compounds of general formula (X):

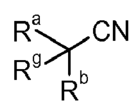

(X)

by reaction with 1-(methylthio)acetone in the presence of trifluoromethanesulfonic anhydride in a solvent such as DCM at a temperature from 0˚C to the reflux temperature of the solvent according to the method described in J. Org. Chem. 2006, 71(8), 3026.

Compounds of general formula (X) are well known in the art and can be prepared by known methods, or are commercially available.

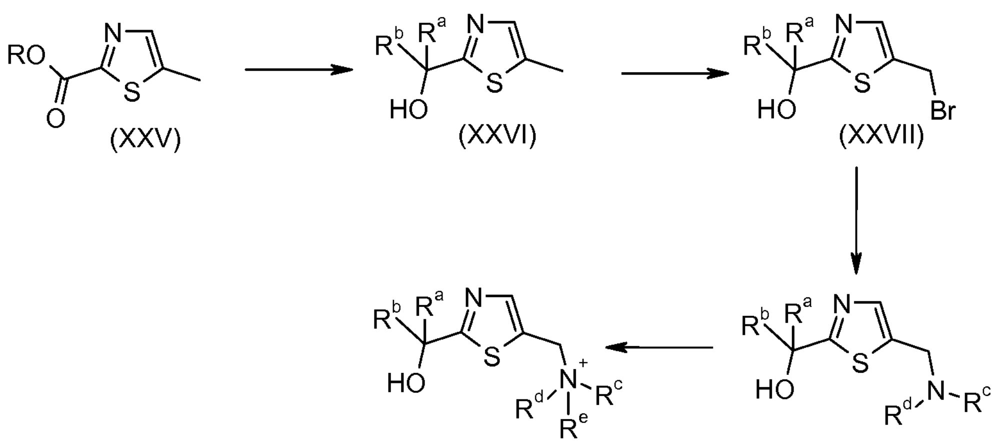

(XXV) (XXVI) (XXVII) (XXVIII) (XXIX)

**Scheme 4**

Compounds of Formula (XXIX) may be prepared from compounds of Formula (XXVI) by employing a similar sequence of reactions as used to prepare compounds of Formula (I-b) from compounds of Formula (VIII) in Scheme 1 above.

Compounds of formula (XXVI) wherein $R^a$ and $R^b$ are the same may be prepared from compounds of Formula (XXV) where R is a suitable alkyl group (such as ethyl or methyl) by treatment with an appropriate organometallic reagent

such as a Grignard reagent, in a suitable solvent such as THF or diethyl ether. Compounds of Formual (XXVI) wherein Ra and Rb are dissimilar may be prepared from compounds of Formula (XXV) by converting to an intermediate amide, preferably a Weinreb amide, and performing the introduction of $R^a$ and $R^b$ through their respective organometallic reagents in a stepwise manner.

Compounds of Formula (XXV) are known in the literature - for example, Helv. Chim. Acta 1946, 29, 1957.

The following non-limiting Examples illustrate the invention.

**General Experimental Details:**

All reactions were carried out under an atmosphere of nitrogen unless specified otherwise.
NMR spectra were obtained on a Varian Unity Inova 400 spectrometer with a 5mm inverse detection triple resonance probe operating at 400MHz or on a Bruker Avance DRX 400 spectrometer with a 5mm inverse detection triple resonance TXI probe operating at 400MHz or on a Bruker Avance DPX 300 spectrometer with a standard 5mm dual frequency probe operating at 300MHz. Shifts are given in ppm relative to tetramethylsilane.

Where products were purified by column chromatography, 'flash silica' refers to silica gel for chromatography, 0.035 to 0.070 mm (220 to 440 mesh) (e.g. Fluka silica gel 60), and an applied pressure of nitrogen up to 10 p.s.i accelerated column elution. Where thin layer chromatography (TLC) has been used, it refers to silica gel TLC using plates, typically 3 × 6 cm silica gel on aluminium foil plates with a fluorescent indicator (254 nm), (e.g. Fluka 60778). All solvents and commercial reagents were used as received.

All compounds containing a basic centre(s), which were purified by HPLC, were obtained as the TFA salt unless otherwise stated.

Preparative HPLC conditions:

C18-reverse-phase column (100 × 22.5 mm i.d Genesis column with 7 μm particle size). UV detection at 230 nm.

LC/MS Systems

The Liquid Chromatography Mass Spectroscopy (LC/MS) systems used:

LC-MS method 1

Waters Platform LCT with a C18-reverse-phase column (100 × 3.0 mm Higgins Clipeus with 5 μm particle size), elution with A: water + 0.1 % formic acid; B: acetonitrile + 0.1 % formic acid. Gradient:

| Gradient - Time | flow ml/min | %A | %B |
|---|---|---|---|
| 0.00 | 1.0 | 95 | 5 |
| 1.00 | 1.0 | 95 | 5 |
| 15.00 | 1.0 | 5 | 95 |
| 20.00 | 1.0 | 5 | 95 |
| 22.00 | 1.0 | 95 | 5 |
| 25.00 | 1.0 | 95 | 5 |

Detection - MS, ELS, UV (100 μl split to MS with in-line UV detector at 254nm)

MS ionisation method - Electrospray (positive ion)

LC-MS method 2

Waters Platform LC with a C18-reverse-phase column (30 × 4.6 mm Phenomenex Luna 3 μm particle size), elution with A: water + 0.1 % formic acid; B: acetonitrile + 0.1 % formic acid. Gradient:

| Gradient - Time | flow ml/min | %A | %B |
|---|---|---|---|
| 0.00 | 2.0 | 95 | 5 |
| 0.50 | 2.0 | 95 | 5 |
| 4.50 | 2.0 | 5 | 95 |
| 5.50 | 2.0 | 5 | 95 |

(continued)

| Gradient - Time | flow ml/min | %A | %B |
|---|---|---|---|
| 6.00 | 2.0 | 95 | 5 |

Detection - MS, ELS, UV (100 μl split to MS with in-line UV detector)
MS ionisation method - Electrospray (positive and negative ion)

LC-MS method 3

Waters Micromass ZQ with a C18-reverse-phase column (30 × 4.6 mm Phenomenex Luna 3 μm particle size), elution with A: water + 0.1 % formic acid; B: acetonitrile + 0.1 % formic acid. Gradient:

| Gradient - Time | flow ml/min | %A | %B |
|---|---|---|---|
| 0.00 | 2.0 | 95 | 5 |
| 0.50 | 2.0 | 95 | 5 |
| 4.50 | 2.0 | 5 | 95 |
| 5.50 | 2.0 | 5 | 95 |
| 6.00 | 2.0 | 95 | 5 |

Detection - MS, ELS, UV (100 μl split to MS with in-line UV detector)
MS ionisation method - Electrospray (positive and negative ion)

LC-MS method 4

Waters ZMD with a C18-reverse-phase column (30 × 4.6 mm i.d. Phenomenex Luna with 3 μm particle size), elution with solvent A (water with 0.1 % formic acid) and solvent B (acetonitrile with 0.1 % formic acid). Gradient:

| Gradient - Time | flow mL/min | %A | %B |
|---|---|---|---|
| 0.00 | 2.0 | 95 | 5 |
| 0.50 | 2.0 | 95 | 5 |
| 4.50 | 2.0 | 5 | 95 |
| 5.50 | 2.0 | 5 | 95 |
| 6.00 | 2.0 | 95 | 5 |

Detection - MS, ELS, UV (200μL/min split to MS with in-line Waters 996 DAD detection).
MS ionisation method - Electrospray (positive and negative ion).

LC-MS method 5

Waters Micromass ZQ with a C18-reverse-phase column (100 × 3.0 mm Higgins Clipeus with 5 μm particle size), elution with A: water + 0.1 % formic acid; B: acetonitrile + 0.1 % formic acid. Gradient:

| Gradient - Time | flow ml/min | %A | %B |
|---|---|---|---|
| 0.00 | 1.0 | 95 | 5 |
| 1.00 | 1.0 | 95 | 5 |
| 15.00 | 1.0 | 5 | 95 |
| 20.00 | 1.0 | 5 | 95 |
| 22.00 | 1.0 | 95 | 5 |
| 25.00 | 1.0 | 95 | 5 |

Detection - MS, ELS, UV (100 μl split to MS with in-line UV detector at 254nm)
MS ionisation method - Electrospray (positive ion)

LC-MS method 6

Waters Micromass ZQ with a C18-reverse-phase column (100 × 3.0 mm Higgins Clipeus with 5 μm particle size), elution with A: water + 0.1 % formic acid; B: acetonitrile + 0.1 % formic acid. Gradient:

| Gradient - Time | flow ml/min | %A | %B |
|---|---|---|---|
| 0.00 | 1.0 | 95 | 5 |
| 1.00 | 1.0 | 95 | 5 |
| 15.00 | 1.0 | 5 | 95 |
| 20.00 | 1.0 | 5 | 95 |
| 22.00 | 1.0 | 95 | 5 |
| 25.00 | 1.0 | 95 | 5 |

Detection - MS, ELS, UV (100 μl split to MS with in-line UV detector at 254nm)
MS ionisation method - Electrospray (positive ion)
Abbreviations used in the experimental section:

DCM = dichloromethane
DIPEA = di-isopropylethylamine
DMAP = dimethylaminopyridine
DMF = dimethylformamide
EtOAc = ethyl acetate
EtOH = ethanol
IMS = industrial methylated spirit
MeOH = methanol
RT = room temperature
Rt = retention time
TFA = trifluoroacetic acid
THF = tetrahydrofuran
Sat = saturated

Preparation of intermediates

**Intermediate 1**

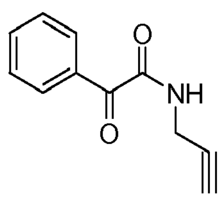

**2-Oxo-2-phenyl-N-prop-2-ynyl-acetamide. (V): $R^a$ = Ph**

Oxalyl chloride (6.1 g, 48mmol) was added to a solution of phenylglyoxylic acid (6.0g, 40mmol) and 3 drops of DMF in dry DCM (50ml). The reaction mixture was stirred at room temperature for 3 hours then the solvent was removed. The residue was taken up in dry DCM (50ml) and the solution was cooled to 0 °C. A mixture of propargyl amine (2.2g, 40 mmol) and triethylamine (4.05g, 40mmol) was added cautiously over a period of 10 minutes then the mixture was allowed to warm to room temperature. Stirring was continued for 2.5 hours then water (10ml) was added. The mixture was washed with 1 M HCl (2x20ml), sat. sodium hydrogencarbonate (aq.) (2x20ml) then brine. The organic phase was then dried ($Na_2SO_4$) and the solvent was removed. The residue was crystallized from cyclohexane to afford the product as a light brown solid.
Yield: 5.75g, 76%

LC-MS (Method 3): Rt 2.47 min, m/z 188 [$MH^+$].

**Intermediate 2**

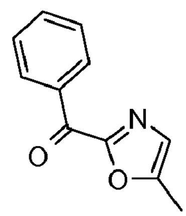

**(5-Methyl-oxazol-2-yl)-phenyl-methanone. (IV): $R^a$ = Ph**

Methane sulphonic acid (10g, 104mmol) was added drop wise to a solution of 2-oxo-2-phenyl-N-prop-2-ynyl-acetamide (Intermediate 1) (2.4g, 12.83mmol) in 1,4-dioxane (20ml). The resultant solution was heated at 90 ˚C for 66 hours. The reaction mixture was cooled and the solvent was removed. The dark residue was partitioned between DCM and water. The DCM fraction was washed with 1 M HCl (2x), sat. sodium hydrogencarbonate (2x) then brine. The solution was dried ($Na_2SO_4$) and the solvent was removed to give the crude product. Purification was achieved via column chromatography, eluting with 4:1 cyclohexane: ethyl acetate. This gave the product as an off white solid.
Yield: 1.0g (41%)
LC-MS (Method 3): Rt 2.94 min, m/z 188 [$MH^+$]

**Intermediate 3**

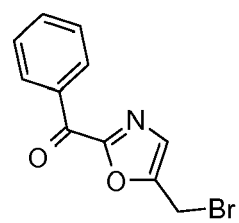

**(5-Bromomethyl-oxazol-2-yl)-phenyl-methanone. (III): $R^a$ = Ph**

A mixture of (5-methyl-oxazol-2-yl)-phenyl-methanone (Intermediate 2) (0.8g, 4.28mmol), N-bromosuccinimide (0.9g, 5.06mmol) and 2,2'-azobis(2-methylpropionitrile) (56mg, 0.34mmol) in carbon tetrachloride (8ml) was heated at reflux for 1.5 hr. The reaction mixture was cooled to ambient temperature and filtered. The filtrate was diluted with DCM (20ml) and washed with water, sat. sodium hydrogen carbonate and brine. It was dried ($Na_2SO_4$) and the solvent was removed. Purification was achieved via column chromatography eluting with 4:1 cyclohexane:ethyl acetate. This gave the product as a yellow solid.
Yield: 0.9g (79%)
LC-MS (Method 3): Rt 3.26min, m/z 266, 268 [$MH^+$]

**Intermediate 4**

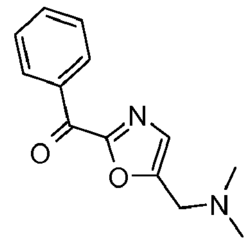

**(5-Dimethylaminomethyl-oxazol-2-yl)-phenyl-methanone. (II): $R^a$ = Ph, $R^c$, $R^d$ = $CH_3$**

(5-Bromomethyl-oxazol-2-yl)-phenyl-methanone (Intermediate 3) (0.18g, 0.68mmol) was dissolved in a 2M solution of dimethylamine in THF (3ml, 6mmol). The mixture was stirred at ambient temperature for 1h with a precipitate forming almost instantly. The solvent was removed and the residue was partitioned between DCM and saturated sodium hydrogencarbonate (aq., 5ml). The aqueous phase was extracted with DCM and the combined organic phase was dried ($Na_2SO_4$) and the solvent removed to give the product as an orange oil that crystallized on standing.
Yield: 0.16g (99%)
LC-MS (Method 2): Rt 1.22min, m/z 231 [$MH^+$]

Also prepared by a similar method by reaction of Intermediate 3 with methylamine was

**Intermediate 5**

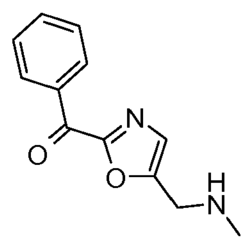

**(5-Methylaminomethyl-oxazol-2-yl)-phenyl-methanone. (II): $R^a$ = Ph, $R^c$ = $CH_3$, $R^d$ = H**

Yield: 2.37g (83%)
LCMS (Method 3): Rt 0.26 and 1.44min, m/z 217 [$MH^+$]

**Intermediate 6**

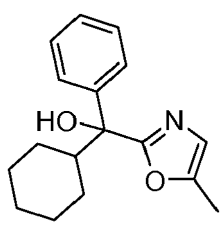

**Cyclohexyl-(5-methyl-oxazol-2-yl)-phenyl-methanol. (VIII): $R^a$ = Ph, $R^b$ = c-Hexyl**

A solution of (5-methyl-oxazol-2-yl)-phenyl-methanone (intermediate 2) (3.0g, 16mmol) in 32mL dry THF at 0˚C under nitrogen was treated dropwise over 10minutes with a 2M solution of cyclohexylmagnesium chloride in diethyl ether (10ml, 20mmol). The resulting deep yellow solution was stirred at 0˚C for about 30mins during which time a

precipitate formed, and then at room temperature for 1.5hours. The reaction mixture was cooled to 0˚C again and treated cautiously with sat. ammonium chloride solution (aq) (10mL). The mixture was stirred at room temperature for 10mins then diluted with water (10mL). The phases were separated and the organic phase was washed with brine. The combined aqueous phase was extracted with DCM (3 x 20mL) and the combined organic phase was dried ($MgSO_4$) and concentrated in vacuo to give the crude product which was triturated with ether (10mL), filtered off and dried.
Yield: 3.65g (84%)
LCMS (Method 3): Rt 3.78 min, m/z 272 [$MH^+$]

Also prepared by a similar method by reaction of Intermediate 2 with cyclopentyl magnesium chloride was

**Intermediate 7**

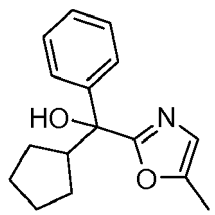

**Cyclopentyl-(5-methyl-oxazol-2-yl)-phenyl-methanol. (VIII): $R^a$ = Ph, $R^b$ = c-Pentyl**

Yield: 3.82g (70%)
LC-MS (Method 2): Rt 3.68 min 258 [$MH^+$]

Also prepared by a similar method by reaction of Intermediate 2 with phenylmagnesium bromide was

**Intermediate 8**

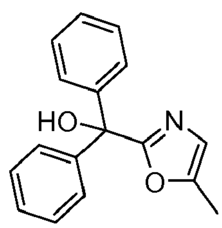

**(5-Methyl-oxazol-2-tl)-diphentl-methanol. (VIII): $R^a$, $R^b$ = Ph**

Yield: 2.06g (73%)
LC-MS (Method 3): Rt 3.78 min 272 [$MH^+$]

**Intermediate 9**

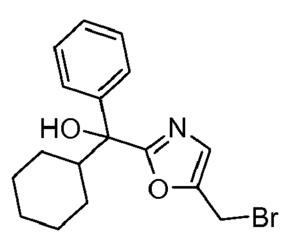

**(5-Bromomethyl-oxazol-2-yl)-cyclohexyl-phenyl-methanol. (VII): $R^a$ = Ph, $R^b$ = c-Hexyl**

A solution of cyclohexyl-(5-methyl-oxazol-2-yl)-phenyl-methanol (Intermediate 6) (3.0g, 11.1mmol) in 1,2-dichloroethane (22mL) was treated with N-bromo-succinimide (2.16g, 12.2mmol) followed by 2,2'-azobis(2-methylpropionitrile) (0.18g, 2.1 mmol). The mixture was heated to 80°C for 2.5h and then allowed to cool to room temperature. Sat. sodium hydrogen carbonate solution was added and the phases were separated. The organic layer was washed with brine and the combined aqueous layers were extracted with DCM. The combined organic phase was dried ($MgSO_4$) and concentrated *in vacuo* to give the crude product as a brown oil. Purification was achieved via column chromatography eluting with 33-100% DCM/cyclohexane followed by 25% EtOAc/DCM.
Yield: 1.85g (48%)
LCMS (Method 3): Rt 4.27 min, m/z 350, 352 [$MH^+$]
Also prepared by a similar method from Intermediate 7 was

**Intermediate 10**

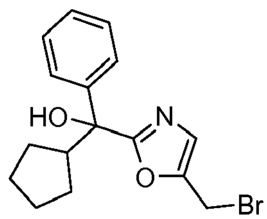

**(5-Bromomethyl-oxazol-2-yl)-cyclopentyl-phenyl-methanol. (VII): $R^a$ = Ph, $R^b$ = c-Pentyl**

Yield: 10.7g(83%)
LCMS (Method 3): Rt 3.90 min, m/z 336, 338 [$MH^+$]
Also prepared by a similar method from Intermediate 8 was

**Intermediate 11**

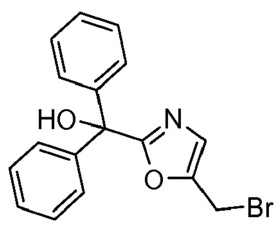

**(5-Bromomethyl-oxazol-2-yl)-diphenyl-methanol. (VII): $R^a$, $R^b$ = Ph**

Yield: 1.63g (63%)
LCMS (Method 4): Rt 3.53 m/z 326, 328 [$MH^+$-$H_2O$]

**Intermediate 12**

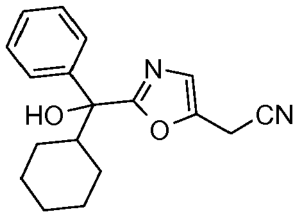

[2-(Cyclohexyl-hydroxy-ahenyl-methyl)-oxazol-5-yl]-acetonitrile. (IX): $R^a$ = Ph, $R^b$ = c-Hexyl

To a solution of ±-(5-bromomethyl-oxazol-2-yl)-cyclohexyl-phenyl-methanol (Intermediate 9) (1.05 g, 3.0 mmol) in IMS (15 mL) was added sodium cyanide (169 mg, 3.45 mmol). The mixture was heated at 70 °C for 1 hour then concentrated *in vacuo* and partitioned between ethyl acetate (30 mL) and water (30 mL). The aqueous was extracted with ethyl acetate (2 x 30 mL) and the combined organics were dried over sodium sulphate, filtered and evaporated to an orange oil. Purification by flash column chromatography over silica gel using a 40 % ethyl acetate : 60 % cyclohexane mixture as eluent then recrystallisation from DCM using hexane gave the title compound as a white crystalline solid.
Yield: 700 mg (79 %)
LC-MS (Method 3): Rt 3.66 min, m/z 279 [$MH^+$-$H_2O$].

Also prepared by a similar method from Intermediate 11 was

**Intermediate 13**

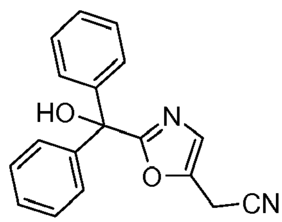

[2-(Hydroxy-diphenyl-methyl)oxazol-5-yl]-acetonitrile. (IX): $R^a$, $R^b$ = Ph

Yield: 0.11g (54%)
LC-MS (Method 3): Rt 2.96min m/z 291 [$MH^+$]

**Intermediate 14**

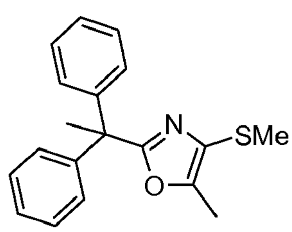

2-(1,1-Diphenyl-ethyl)-5-methyl-4-methylsulfanyl-oxazole. (XI): $R^a$, $R^b$ = Ph, $R^g$ = $CH_3$

To a colourless solution of 1-methylthio-2-propanone (0.98mL, 9.7mmol) in 13 mL dry DCM at 0°C under nitrogen was added dropwise a solution of trifluoromethanesulfonic anhydride (1.62mL, 9.7mmol) in 10mL dry DCM. The resulting yellow solution was stirred at 0°C for 1h then a solution of 2,2-diphenylpropionitrile (1 g, 4.8mmol) in 10mL dry DCM was added dropwise rapidly. The reaction mixture was stirred at 0°C for 2h then between 0°C and room

temperature for 5 days, becoming dark red in colour. The reaction mixture was cooled to 0˚C and treated cautiously with sat. sodium bicarbonate solution. The phases were separated and the aqueous layer was extracted with DCM (x2). The combined organic phase was washed with water, sat. sodium bicarbonate solution and brine, dried ($Na_2SO_4$) and evaporated to a dark viscous oil (1.67g). The crude product was purified by silica gel chromatography eluting with 5% diethyl ether/cyclohexane ($R_f$ = 0.25) to give the title compound as a pale yellow oil that crystallized on standing.
Yield: 0.79g (53%)
LCMS (Method 2): Rt 4.37min m/z 310 [$MH^+$]

**Intermediate 15**

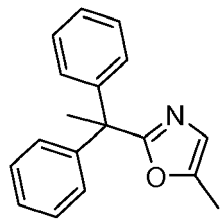

**2-(1,1-Diphenyl-ethyl)-5-methyl-oxazole. (XII): $R^a$, $R^b$ = Ph, $R^g$ = $CH_3$**

A suspension of 2-(1,1-diphenyl-ethyl)-5-methyl-4-methylsulfanyl-oxazole (Intermediate 14) (0.70g, 2.3mmol) in 15mL IMS was warmed to dissolve. Raney Nickel 2800 (~3g) was added causing rapid evolution of a gas. The reaction mixture was stirred at reflux under nitrogen. After 1.25h LCMS indicated a 1:1 mixture of starting material:product and no change after a further 1 h. Another 3g Raney Nickel 2800 was added and the reaction mixture was stirred at reflux for 1 h. Tlc indicated all the starting material had reacted. The catalyst was filtered off through hi-flow and the volatiles were evaporated to give a colourless viscous oil (0.57g). The oil was purified by silica gel chromatography eluting with 15% EtOAc/cyclohexane ($R_f$ = 0.36) to give the title compound as a colourless oil.
Yield: 0.57g (89%)
LCMS (Method 2): Rt 3.96min m/z 264 [$MH^+$]

**Intermediate 16**

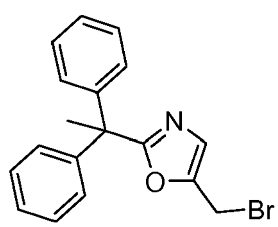

**5-Bromomethyl-2-(1,1-diphenyl-ethyl)-oxazole. (XIII): $R^a$, $R^b$ = Ph, $R^g$ = $CH_3$**

Prepared from Intermediate 15 according to the method used to prepare Intermediate 9.
Yield: 0.75g (quant.)
LCMS (Method 2): Rt 4.06min m/z 342, 344 [$MH^+$]

**Intermediate 17**

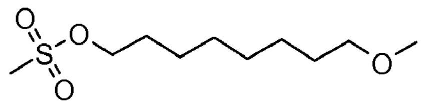

**Methanesulfonic acid 8-methoxy-octyl ester.**

Diisopropylethylamine (170mg, 1.49mmol) was added to a solution of 8-methoxyoctan-1-ol (217mg, 1.35mmol) in dry DCM (1mL). The solution was cooled in an ice bath and methanesulfonyl chloride (170mg, 1.49mmol) was added under nitrogen. The solution was allowed to warm up to ambient temperature overnight. Tlc showed the presence of some starting material. More methanesulfonyl chloride (231 mg, 2.01 mmol) was added and the solution was stirred at ambient temperature overnight. The reaction mixture was treated with water and the phases were separated. The organic layer was dried ($MgSO_4$) and the solvent evaporated. The crude product was purified by silica gel chromatography eluting with diethyl ether:cyclohexane (1:1) and then with diethyl ether:cyclohexane (2:1) to give the title compound as an oil. 8-Methoxy-octan-1-ol can be prepared according to methods described in Synthesis 2004, 4, 595.
Yield: 135mg (42%)
LCMS (Method 2): Rt 3.14min, no mass ion observed

**Intermediate 18**

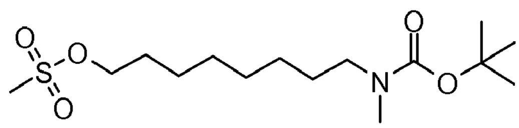

**Methanesulfonic acid 8-(tert-butoxycarbonyl-methyl-amino)-octyl ester.**

The title compound was prepared according to the procedure described for the preparation of Intermediate 17 using (8-hydroxy-octyl)-methyl-carbamic acid tert-butyl ester instead of 8-methoxy-octan-1-ol. (8-Hydroxy-octyl)-methyl-carbamic acid tert-butyl ester can be prepared according to methods described in US2005277688 or US2004254219.
Yield: 225mg (71%)
$^1H$ NMR ($CDCl_3$): δ 1.20-1.57 (m, 19H), 1.75 (m, 2H), 2.83 (s, 3H), 3.01 (s, 3H), 3.19 (t, 2H), 4.22 (t, 2H).

**Intermediate 19**

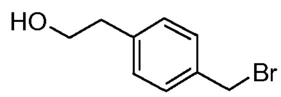

**2-(4-Bromomethyl-phenyl)-ethanol.**

A solution of 4-(bromomethyl)phenyl acetic acid (458mg, 2.0mmol) in 10mL toluene and 8mL THF under nitrogen was treated with a 2M solution of borane-dimethylsulfide complex in THF (2mL, 4.0mmol) and the reaction mixture was stirred at room temperature overnight. No reaction had occurred by LCMS. A further 1.5mL (3.0mmol) of borane-dimethylsulfide complex was added and the reaction was stirred for 2h. EtOAc and water were added and the phases were separated. The organic layer was dried ($MgSO_4$) and the solvent evaporated to give the title compound.
Yield: 347mg (81%)
$^1H$ NMR ($CDCl_3$): δ 2.89 (t, 2H), 3.49 (s, 1H), 3.86 (t, 2H), 4.49 (s, 2H), 7.21 (d, 2H), 7.35 (d, 2H).

**Intermediate 20**

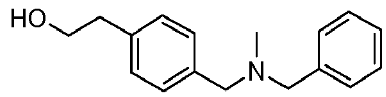

**2-{4-[(Benzyl-methyl-amino)-methyl]-phenyl}-ethanol.**

A solution of 2-(4-bromomethyl-phenyl)-ethanol (Intermediate 19) (347mg, 1.6mmol) in 15mL acetonitrile was treated with potassium carbonate (557mg, 4.0mmol) followed by N-methyl benzylamine (293mg, 2.4mmol). The reaction was stirred at reflux for 6h when LCMS indicated complete conversion of the starting material. The reaction was allowed to cool to room temperature and the solvent was evaporated. The residue was partitioned between EtOAc and water and the phases were separated. The organic layer was dried ($MgSO_4$) and the solvent evaporated. The crude product was purified by silica gel chromatography eluting with DCM to 1% MeOH/DCM to give the title compound as an oil.
Yield: 258mg (63%)
$^1$H NMR ($CDCl_3$): $\delta$ 2.09 (br s, 1H), 2.16 (s, 3H), 2.81 (t, 2H), 3.48 (s, 2H), 3.50 (s, 2H), 3.79 (t, 2H), 7.15 (d, 2H), 7.20-7.25 (m, 1H), 7.26-7.37 (m, 6H).

**Intermediate 21**

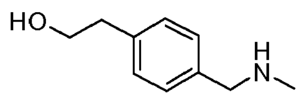

**2-(4-Methylaminomethyl-phenyl)-ethanol.**

A solution of 2-{4-[(benzyl-methyl-amino)-methyl]-phenyl}-ethanol (Intermediate 20) (258mg, 1.0mmol) in 10mL IMS was treated with 20% palladium hydroxide on carbon (50mg). The reaction mixture was stirred under an atmosphere of hydrogen for 3h when LCMS indicated complete conversion of starting material. The reaction mixture was filtered through celite and the solvent was evaporated. The crude product was loaded onto an SCX-2 cartridge using 10% MeOH/DCM and the impurities were removed by flushing the column with MeOH/DCM. The column was eluted with 1: 1 2M methanolic ammonia/DCM and the solvent was removed to give the title compound.
Yield: 91 mg (55%)
$^1$H NMR (MeOD): $\delta$ 2.35 (s, 3H), 2.79 (t, 2H), 3.65 (s, 2H), 3.72 (t, 2H), 7.18-7.25 (m, 4H).

**Intermediate 22**

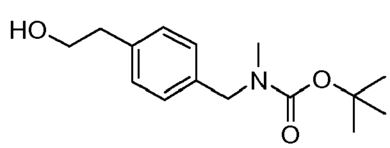

**[4-(2-Hydroxy-ethyl)-benzyl]-methyl-carbamic acid tert-butyl ester.**

A solution of 2-(4-methylaminomethyl-phenyl)-ethanol (Intermediate 21) (91 mg, 0.55mmol) in 3mL dry DCM at 0˚C was treated dropwise with a solution of di-tert-butyl carbonate (144mg. 0.66mmol) in 2mL dry DCM and the reaction mixture was allowed to warm up to room temperature. After 2h water was added and the mixture was stirred for 10mins. The two layers were separated and the organic layer was dried ($MgSO_4$) and the solvent evaporated to give the title compound.
Yield: 146mg (quant).

$^1H$ NMR ($CDCl_3$): δ 1.48 (s, 9H), 2.81 (br s, 3H), 2.86 (t, 2H), 3.86 (br t, 2H), 4.40 (br s, 2H), 7.14-7.22 (m, 4H).

**Intermediate 23**

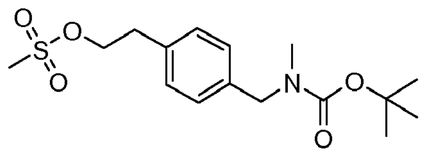

**Methanesulfonic acid 2-{4-[(tert-butoxycarbonyl-methyl-amino)-methyl]-phenyl}-ethyl ester.**

The title compound was prepared according to the procedure described for the preparation of Intermediate 17 using Intermediate 22 instead of 8-methoxy-octan-1-ol.
Yield: 106mg (50%)
$^1H$ NMR ($CDCl_3$): δ 1.48 (s, 9H), 2.75-2.84 (br s, 3H), 2.87 (s, 3H), 3.04 (t, 2H), 4.36-4.44 (m, 4H), 7.15-7.22 (m, 4H).

**Intermediate 24**

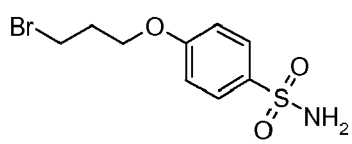

**4-(3-Bromo-propoxy)-benzenesulfonamide.**

A suspension of 4-hydroxybenzenesulfonamide (4.1g, 23mmol), 1,3-dibromopropane (6.83g, 34mmol) and potassium carbonate (3.17g, 23mmol) in acetonitrile (35mL) was heated at 55°C overnight. The mixture was allowed to cool to ambient temperature and was filtered. The filtrate was evaporated to give a residue which was partitioned between DCM and 0.1 M NaOH(aq).The organic layer was dried ($MgSO_4$) and the solvent evaporated. Purification was carried out by by silica gel column chromatography eluting with cyclohexane and then with diethyl ether, followed by recrystallisation from isopropanol.
Yield: 483mg (7%)
LCMS (Method 2): Rt 3.01 min, no mass ion observed

**Intermediate 25**

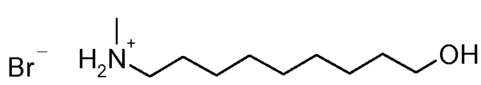

**(9-Hydroxy-nonyl)-methyl-ammonium bromide.**

To a solution of 9-bromo-1-nonanol (10.2 g, 45.7 mmol) in IMS (50 mL) at 0 °C was added a solution of methylamine (57 mL, 8 M in EtOH, 456 mmol). After 30 minutes at 0 °C the reaction mixture was allowed to warm to RT and stirred for 26 h. The solvent was evaporated to afford a white solid, which was triturated with diethyl ether to provide the title compound as a white solid.

Yield: 9.97 g, 86%.
LC-MS (Method 3): Rt 1.51 min, m/z 174 [MH+].

**Intermediate 26**

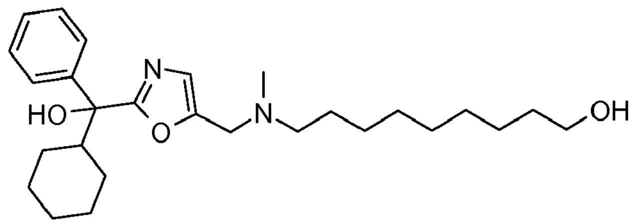

**9-{[2-(Cyclohexyl-hydroxy-phenyl-methyl)-oxazol-5-ylmethyl]-methyl-amino}-nonan-1-ol.**

(5-Bromomethyl-oxazol-2-yl)-cyclohexyl-phenyl-methanol (1.0 g, 2.86 mmol) was added to a solution of (9-hydroxy-nonyl)-methyl-ammonium bromide (686 mg, 2.86 mmol) and N,N-diisopropylethylamine (1.0 mL, 5.7 mmol) in dry DCM. After stirring the mixture at RT for 4 h satd $NaHCO_3$ (aq) was added. The phases were separated and the aqueous layer extracted with DCM. The combined organic layers were dried ($Na_2SO_4$), filtered, and concentrated to dryness to afford a yellow oil. Purification by column chromatography over silica gel using a gradient of 5-10% MeOH/DCM as eluent provided the title compound as a colourless oil.
Yield: 0.80 g, 63%.
LC-MS (Method 2): Rt 2.42 min, m/z 443 [MH+].

**Intermediate 27**

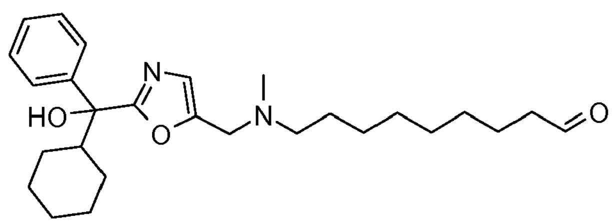

**9-{[2-(Cyclohexyl-hydroxy-phenyl-methyl)-oxazol-5-ylmethyl]-methyl-amino}-nonanal**

A solution of DMSO (0.17 mL, 2.3 mmol) in dry DCM (3 mL) was added dropwise to a solution of oxalyl chloride (94 μL) in dry DCM (3 mL) at -78 ˚C under a nitrogen atmosphere. Then a solution of 9-{[2-(cyclohexyl-hydroxy-phenyl-methyl)-oxazol-5-ylmethyl]-methyl-aminol-nonan-l-ol (0.49 g, 1.1 mmol) in dry DCM (5 mL) was added and the reaction mixture was stirred at -78 ˚C for 15 min. Triethylamine (0.62 mL, 4.4 mmol) was added and the reaction mixture was allowed to warm to RT. After 1 h satd $NaHCO_3$ (aq) was added, the phases separated, and the aqueous layer extracted with DCM. The combined organic layers were washed with brine, dried ($Na_2SO_4$), filtered, and concentrated to dryness to afford a yellow/brown viscous oil. The crude product was resubmitted under the same reaction conditions to further the conversion of starting material to afford a yellow viscous oil plus solids. This was triturated with diethyl ether and the supernatant was concentrated to dryness to afford a solid foam, which was used without further purification.
Yield: 0.59 g.
LC-MS (method 2): Rt 2.74 min, m/z 441 [MH+].

**Intermediate 28**

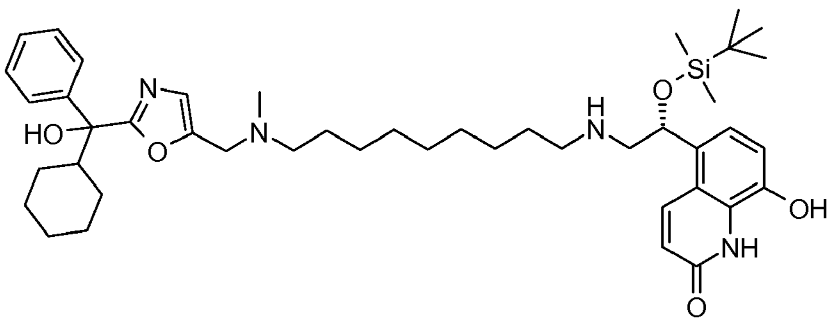

**5-[(R)-1-(*tert*-Butyl-dimethyl-silanyloxy)-2-(9-{[2-(cyclohexyl-hydroxy-phenylmethyl)-oxazol-5-ylmethyl]-methyl-amino}-nonylamino)-ethyl]-8-hydroxy-1*H*-quinolin-2-one**

A mixture of 9-{[2-(cyclohexyl-hydroxy-phenyl-methyl)-oxazol-5-ylmethyl]-methyl-amino}-nonanal (0.49 g, 1.1 mmol), 5-[(R)-2-amino-1-(*tert*-butyl-dimethyl-silanyloxy)-ethyl]-8-hydroxy-1*H*-quinolin-2-one (0.37 g, 1.1 mmol), and sodium triacetoxyborohydride (0.33 g, 1.6 mmol) in dry DCE (10 mL) was stirred under a nitrogen atmosphere at RT for 19 h. The solvents were evaporated *in vacuo* and the residue was purified by column chromatography over silica gel using a mixture of DCM/MeOH/acetic acid/water (120:15:3:2) as eluent to afford the product as a very light brown gum. The residue was taken up in MeOH, passed over a SCX-2 cartridge, and liberated using 2 M ammonia solution in MeOH. Evaporation of the solvent *in vacuo* afforded the title compound as a mixture of diastereomers as a yellow/green gum.
Yield: 78 mg, 9%.
LC-MS (method 2): Rt 2.64 min, m/z 760 [MH+].

**Intermediate 29**

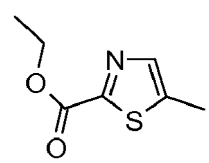

**5-Methylthiazole-2-carboxylic acid ethyl ester**

Prepared according to Helv. Chim. Acta., 1946 (29), 1957.

**Intermediate 30**

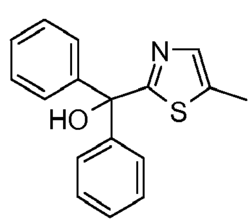

**(5-Methylthiazol-2yl)diphenyl methanol**

To an ice cold solution of Intermediate 29 (0.24 g, 1.402 mmol) in anhydrous THF (7ml) under an atmosphere of nitrogen was added, dropwise, a 3M solution of phenylmagnesium bromide in diethyl ether (0.934 ml, 2.80 mmol). Once the addition was complete, the reaction mixture was allowed to warm to RT and stirred for 20mins. After this period,

the solution was poured onto 1M HCl and extracted twice with diethyl ether. The combined organic layers were washed with satd. sodium hydrogen carbonate (aq.), water and brine, dried ($MgSO_4$) and evaporated. The residue was subjected to column chromatography ($SiO_2$, 40g) eluting with 10% EtOAc in iso-hexane to give the desired material.
Yield = 0.27 g (67%)
LC-MS (Method 6): $R_t$ 3.20 min, m/z 282 $[MH]^+$.
$^1$H NMR, 400 MHz, DMSO-$d_6$: 7.4 (1H, m), 7.3 (4H, m), 7.3-7.2 (6H, m), 7.1 (1H, s) and 2.4 (3H, d).

**Intermediate 31**

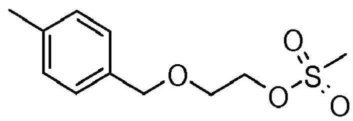

**Methanesulfonic acid 2-(4-methyl-benzyloxy)-ethyl ester**

Prepared in a similar manner to the procedure described in J. Am. Chem. Soc. 2002, 124(28), 8206.
LC-MS (Method 4): Rt 2.44 mins, no molecular ion observed.

**Intermediate 32**

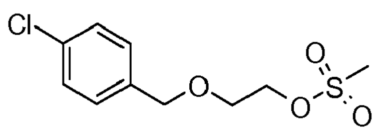

**Methanesulfonic acid 2-(4-chloro-benzyloxy)-ethyl ester**

Prepared in a similar manner to Intermediate 31.
LC-MS (Method 2): Rt 3.34 mins, no molecular ion observed.

**Intermediate 33**

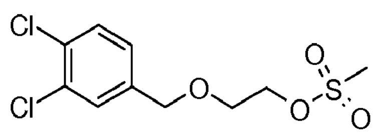

**Methanesulfonic acid 2-(4-chloro-benzyloxy)-ethyl ester**

Prepared in a similar manner to Intermediate 31.
LC-MS (Method 4): Rt 3.59 mins, no molecular ion observed.

**Intermediate 34**

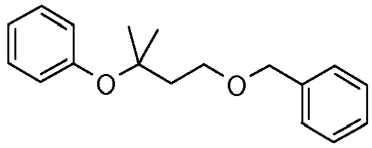

1-Benzyloxy-3-methyl-3-phenoxybutane

To a solution of (3-methyl-but-3-enyloxymethyl)-benzene (4.22g, 23.9mmol) and phenol (2.25g, 23.9mmol) in 20mL dry DCM was added under nitrogen $BF_3 \cdot Et_2O$ (0.582mL, 4.78mmol). The solution was stirred at ambient temperature overnight, $H_2O$ was added, and the phases were separated. The aqueous phase was extracted with DCM (x2) and the organic layer was dried ($MgSO_4$) and the solvent removed to give an oil. The crude product was purified by silica gel column chromatography eluting with a gradient from cyclohexane to 5% diethyl ether in cyclohexane.
Yield: 0.9g (14%)
LC-MS (Method 4): Rt 4.47min, no molecular ion observed.

Intermediate 35

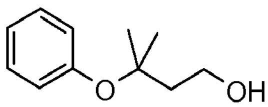

3-Methyl-3-phenoxy-butan-1-ol

Palladium (II) hydroxide on carbon (20 wt. %) (150mg) was added to a flask under inert atmosphere, followed by cooled IMS (5ml) and (3-benzyloxy-1,1-dimethyl-propoxy)-benzene (0.73g, 2.7mmol). The flask was purged with hydrogen (x3) and the mixture stirred at ambient temperature overnight under a hydrogen balloon. The mixture was filtered through celite under inert atmosphere and the filtrate was evaporated to give the crude product which was purified by column chromatography using a gradient from cyclohexane to 50% diethyl ether in cylclohexane.
Yield: 71mg, 14%
LC-MS (Method 4): Rt 2.87 min, no molecular ion observed.

Intermediate 36

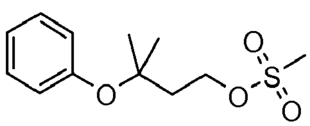

Methanesulfonic acid 3-methyl-3-phenoxy-butyl ester

The title compound was prepared according to the procedure described for the preparation of Intermediate 17.
Yield: 37mg, 37%
LC-MS (Method 2): Rt 3.45 min, no molecular ion observed.

Intermediate 37

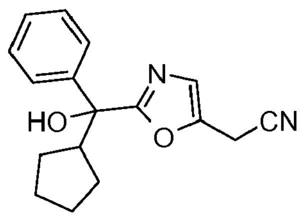

**[2-(Cyclopentyl-hydroxy-phenyl-methyl)-oxazol-5-yl]-acetonitrile. (IX): $R^a$ = Ph, $R^b$ = c-Pentyl**

Prepared according to the method used for the preparation of Intermediate 12.
Yield: 0.75 g (57%)
LC-MS (Method 4): Rt 3.47 min, m/z 265 [$MH^+$-$H_2O$].

## Examples

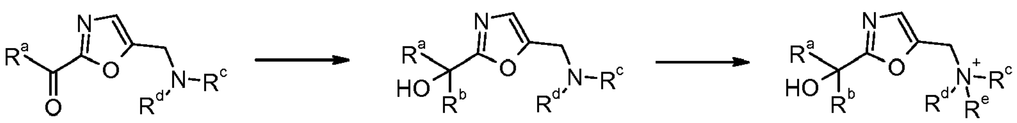

(II) (I-a) (I-b)

**Scheme 5**

The following compounds were prepared using the route shown in Scheme 5.

## Example 1

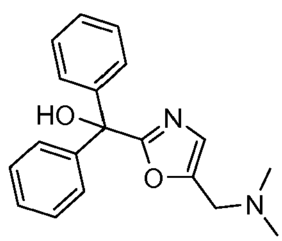

**(5-Dimethylaminomethyl-oxazol-2-yl)-diphenyl-methanol. (I-a): $R^a$, $R^b$ = Ph, $R^c$, $R^d$ = $CH_3$**

Phenylmagnesium bromide (0.75 ml of a 1M solution in THF, 0.75mmol) was added dropwise to a cold (0 ˚C) solution of (5-dimethylaminomethyl-oxazol-2-yl)-phenyl-methanone (Intermediate 4) (0.15g, 0.65mmol) in dry THF (1.5ml). The mixture was stirred cold for 1.5h then further phenylmagnesium bromide (0.4ml of a 1M solution in THF, 0.4 mmol) was added dropwise. The mix was stirred at 0 ˚C for 0.5h and was then treated with excess sat. ammonium chloride solution (aq.). The mixture was extracted with DCM (x2) and the combined organic phase was washed with brine, dried ($Na_2SO_4$) and the solvent removed to give the crude product. Purification was achieved by HPLC eluting with 5-70% acetonitrile/water containing 0.1%TFA over 18.5 mins.
Yield: 0.19g (69%, as its TFA salt)
LC-MS (Method 1): Rt 5.56 min, m/z 309 [$MH^+$]
LC-MS (Method 3): Rt 1.72 min, m/z 309 [$MH^+$]

$^1$H NMR (DMSO-$d_6$): δ 2.74 (s, 6H), 4.48 (s, 2H), 7.25-7.37 (m, 10H), 7.40 (s, 1H), 10.23 (br s, 1H).

A sample of this material was converted to the free base by passing through an SCX-2 cartridge eluting with MeOH (x3) and then 2M ammonia/MeOH (x3) to give the desired compound as a white solid.
LC-MS (Method 1): Rt 5.68 min, m/z 309 [MH$^+$]
$^1$H NMR (DMSO-$d_6$): δ 2.12 (s, 6H), 3.47 (s, 2H), 6.98 (s, 1H), 7.02 (s, 1H) 7.22-7.34 (m, 10H).

**Example 2**

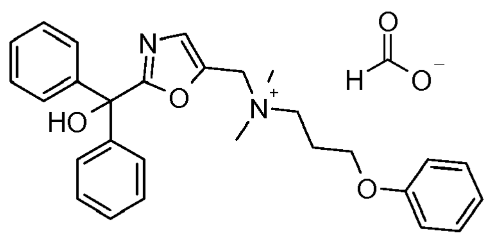

**[2-(Hydroxy-diphenyl-methyl)-oxazol-5-ylmethyl]-dimethyl-(3-phenoxv-propyl)-ammonium formate. (I-b): R$^a$ = R$^b$ = Ph, R$^c$, R$^d$ = $CH_3$, R$^e$ = 3-Phenoxypropyl.**

A solution of (5-dimethylaminomethyl-oxazol-2-yl)-diphenyl-methanol (Example 1) (24mg, 0.078mmol) in acetonitrile (0.3mL) and chloroform (0.5mL) was treated with 3-phenoxypropyl bromide (37μL, 0.23mmol) and the reaction mixture was stirred at room temperature overnight and then at 50˚C for 42h. The volatiles were evaporated and the crude product was purified by preparative HPLC eluting with 25-75% acetonitrile/water containing 0.1 % formic acid over 30mins to give the product as a colourless gum.
Yield 24mg, 63%
LC-MS (Method 1): Rt 7.56 min m/z 443 [M$^+$]
$^1$H NMR (MeOD): δ 2.29 (m, 2H), 3.11 (s, 6H), 3.45 (m, 2H), 3.98 (t, 2H), 4.79 (s, 2H), 6.85-6.90 (m, 2H), 6.93-6.98 (m, 1H), 7.24-7.38 (m, 12H), 7.56 (s, 1H), 8.51 (br s, 1H).

Also prepared by a similar method using the route shown in Scheme 2 were the following compounds:

| Example | Name | Structure | $^1$H NMR | LCMS |
|---|---|---|---|---|
| 3 | (5-Methylaminomethyl-oxazol-2-yl)-diphenyl-methanol. (I-a): R$^a$, R$^b$ = Ph, R$^c$ = H, R$^d$ = $CH_3$. | 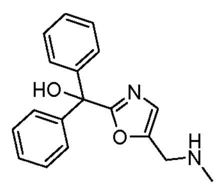 | ($CDCl_3$): 2.31 (s, 3H), 3.06 (br s, 2H), 3.68 (d, 2H), 6.81 (s, 1H), 7.25-7.36 (m, 10H). | (Method 3): Rt 1.44min, m/z 217 [MH$^+$] |

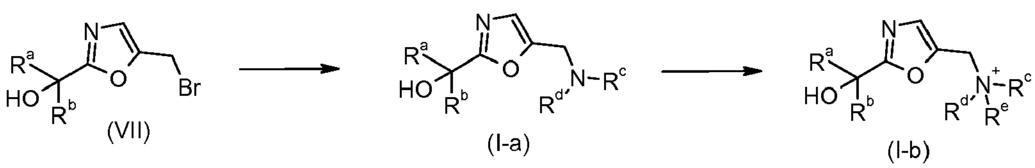

(VII) (I-a) (I-b)

**Scheme 6**

The following compounds were prepared using the route shown in Scheme 6.

Example 4

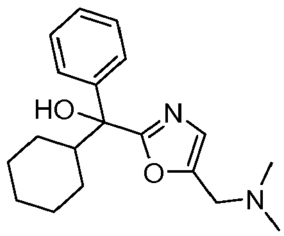

**Cyclohexyl-(5-dimethylaminomethyl-oxazol-2-yl)-phenyl-methanol. (I-a): $R^a$, = Ph, $R^b$ = c-Hexyl, $R^c$, $R^d$ = $CH_3$**

A solution of (5-bromomethyl-oxazol-2-yl)-cyclohexyl-phenyl-methanol (Intermediate 9) (3.2g, 9.2mmol) in 40mL THF was treated with a 2M solution of dimethylamine in THF (40mL, 80mmol). A suspension formed after stirring for a few minutes. The reaction mixture was stood at room temperature overnight and then the solid was filtered off and discarded. The filtrate was concentrated under reduced pressure and the residue was partitioned between DCM and sat. sodium hydrogen carbonate solution. The organic layer was dried ($Na_2SO_4$) and evaporated to give the title compound as a solid.
Yield: 2.74g (95%)
LC-MS (Method 1): Rt 6.57min, m/z 315 [$MH^+$]
$^1H$ NMR (DMSO-$d_6$): δ 0.92-1.29 (m, 6H), 1.42-1.74 (m, 4H), 2.10 (s, 6H), 2.22 (m, 1H), 3.45 (s, 2H), 5.90 (s, 1H), 6.98 (s, 1H), 7.18-7.22 (m, 1H), 7.27-7.34 (m, 2H), 7.40-7.46 (m, 2H).

The two enantiomers of cyclohexyl-(5-dimethylaminomethyl-oxazol-2-yl)-phenyl-methanol_(Example 4) (2.74g) were separated by chiral preparative HPLC using a 250x20 mm chiralpak® IA column packed with amylase tris (3,5-dimethylphenylcarbamate) immobilized on 5μm silica gel. The column was eluted with 5% ethanol in heptane buffered with 0.1% diethylamine at 15ml/min. The first eluting enantiomer (Rt 8.5 min) gave **(S)-cyclohexyl-(5-dimethylaminomethyl-oxazol-2-yl)-phenyl-methanol (I-a): $R^a$, = Ph, $R^b$ = c-Hexyl, $R^c$, $R^d$ = $CH_3$** (Example 5) as a white solid.

**Example 5**

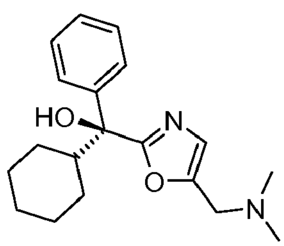

Yield: 0.73g (27%)
LC-MS (Method 1): Rt 6.50 min, m/z 315 [$MH^+$]
$^1H$ NMR ($CDCl_3$): δ 1.12-1.39 (m, 7H), 1.62-1.76 (m, 3H), 2.25 (s, 6H), 2.29-2.32 (m, 1H), 3.54 ($dd_{AB}$, 2H), 3.70 (br.s, 1H), 6.84 (s, 1H), 7.24 (t, 1H), 7.33 (t, 2H), 7.64 (d, 2H).

The second eluting enantiomer (Rt 10.3 min) gave **(R)-cyclohexyl-(5-dimethylaminomethyl-oxazol-2-yl)-phenyl-methanol (I-a): $R^a$, = Ph, $R^b$ = c-Hexyl, $R^c$, $R^d$ = $CH_3$,** (Example 6) as a white solid.

Example 6

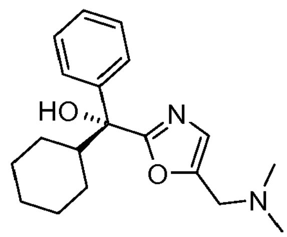

Yield: 1.04g (38%)
LC-MS (Method 1): Rt 6.48 min, m/z 315 [$MH^+$]
$^1$H NMR ($CDCl_3$): δ 1.1 0-1.39 (m, 7H), 1.62-1.76 (m, 3H), 2.25 (s, 6H), 2.29-2.35 (m, 1H), 3.54 ($dd_{AB}$, 2H), 3.70 (br.s, 1H), 6.84 (s, 1H), 7.24 (t, 1H), 7.33 (t, 2H), 7.64 (d, 2H).

**Example 7**

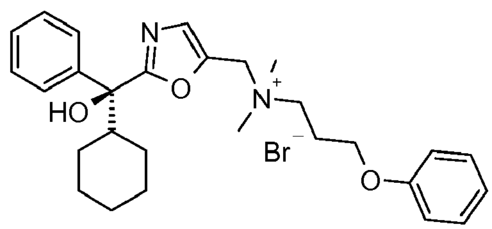

**[2-((S)-Cyclohexyl-hydroxy-phenyl-methyl)-oxazol-5-ylmethyl]-dimethyl-(3-phenoxy-propyl)-ammonium bromide. (I-b): $R^a$ =Ph, $R^b$ = c-Hexyl, $R^c$, $R^d$ =$CH_3$, $R^e$ = 3-Phenoxypropyl.**

A solution of (S)-cyclohexyl-(5-dimethylaminomethyloxazol-2-yl)-phenyl-methanol (Example 5) (0.060g, 0.19mmol) and 3-phenoxypropyl bromide (0.215g, 1mmol) in acetonitrile (1.33ml) and chloroform (2ml) was allowed to stand at RT for 5 days. The solvent was removed to give the crude product. Purification was achieved by column chromatography eluting sequentially with DCM, 2.5%, 5%, 10% then 20% methanol in DCM.
Yield: 50mg (43%)
LC-MS (Method 1): Rt 8.32 min, m/z 449 [$M^+$]
$^1$H NMR ($CDCl_3$): δ 1.06-1.17 (m, 3H), 1.23-1.36 (m, 4H), 1.52-1.85 (m, 3H), 2.28-2.35 (m, 3H), 3.32 (s, 3H), 3.33 (s, 3H), 3.63 (dd, 2H), 4.04 (t, 2H), 5.23 ($dd_{AB}$, 2H), 6.85 (d, 2H), 6.98 (t, 1H), 7.20 (t, 1H), 7.26-7.30 (m, 4H), 7.55-7.58 (m, 3H).

**Example 8**

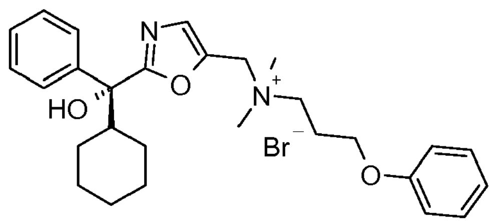

**[2-((R)-Cyclohexyl-hydroxy-phenyl-methyl)-oxazol-5-ylmethyl]-dimethyl-(3-phenoxy-propyl)-ammonium bromide. (I-b): $R^a$ =Ph, $R^b$ = c-Hexyl, $R^c$, $R^d$ = $CH_3$, $R^e$ = 3-Phenoxypropyl**

A solution of (R)-cyclohexyl-(5-dimethylaminomethyloxazol-2-yl)-phenyl-methanol (Example 6) (98 mg, 0.31 mg) and 3-phenoxypropyl bromide (740 mg, 3.44 mmol) in chloroform (1.5 mL) and acetonitrile (1.5 mL) was heated at 50 ˚C for 22 h. The RM was concentrated to dryness to afford a colourless viscous oil, which was triturated with diethyl ether to furnish a white gum. This was purified by column chromatography eluting with 2.5-25% MeOH/DCM to afford the product as a turbid viscous oil. Drying under vacuum at 45 ˚C for 1-2 days afforded a white solid.
Yield: 142 mg (86%)
LC-MS (Method 1): $R_t$ 8.41 min, m/z 449 [$MH^+$]
$^1$H NMR ($CDCl_3$): δ 1.06-1.16 (m, 3H), 1.21-1.37 (m, 4H), 1.59-1.74 (m, 3H), 2.32 (m, 3H), 3.32 (s, 3H), 3.33 (s, 3H), 3.61 (dd, 2H), 4.03 (t, 2H), 4.14 (br.s, 1H), 5.20 ($dd_{AB}$, 2H), 6.85 (d, 2H), 6.98 (t, 1H), 7.19 (t, 1H), 7.26-7.30 (m, 4H), 7.55-7.58 (m, 3H).

**Example 9**

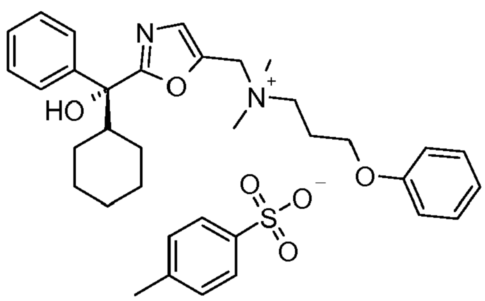

**[2-((R)-Cyclohexyl-hydroxy-ahenyl-methyl)-oxazol-5-ylmethyl]-dimethyl-(3-phenoxy-propyl)-ammonium tosylate. (I-b): $R^a$ =Ph, $R^b$ = c-Hexyl, $R^c$, $R^d$ = $CH_3$, $R^e$ = 3-Phenoxypropyl.**

Prepared according to the method used in Example 8 but using 3-phenoxypropyl tosylate in place of 3-phenoxypropyl bromide.
Yield: 80%
LC-MS (Method 5): $R_t$ 7.72 min, m/z 449 $[MH]^+$.
$^1$H NMR (DMSO-$d_6$): δ 0.96-1.25 (m, 6H), 1.54-1.71 (m, 4H), 2.18-2.27 (m, 3H), 2.28 (s, 3H), 3.03 (s, 3H), 3.04 (s, 3H), 3.33-3.39 (m, 2H), 3.99 (t, 2H), 4.76 (s, 2H), 6.10 (s, 1H), 6.92 (d, 2H), 6.96 (t, 1H), 7.11 (d, 2H), 7.22 (t, 1H), 7.31 (dt, 4H), 7.45-7.49 (m, 4H), 7.54 (s, 1H).

**Example 10**

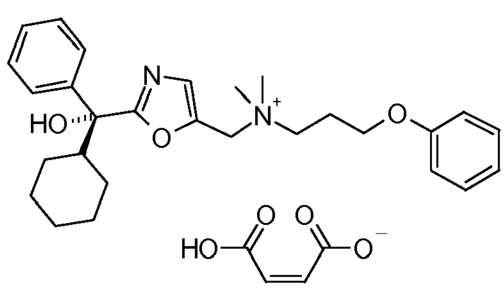

**[2-((R)-Cyclohexyl-hydroxy-phenyl-methyl)-oxazol-5-ylmethyll-dimethyl-(3-**phenoxy-aroayl)-ammonium (*Z*)-3-carboxy-acrylate. (I-b): $R^a$ =Ph, $R^b$ = c-Hexyl, **$R^c$, $R^d$ = $CH_3$, $R^e$ = 3-Phenoxypropyl.**

A mixture or silver(I) oxide (59 mg, 0.25 mmol) and [(R)-2-(cyclohexyl-hydroxyphenyl-methyl)-oxazol-5-ylmethyl]-dimethyl-(3-phenoxy-propyl)-ammonium bromide (Example 8) (265 mg, 0.500 mmol) in water (10 mL) was stirred at RT for 5 h. Maleic acid (58 mg, 0.50 mmol) was added to the reaction mixture, followed by MeOH (10 mL). The suspension was stirred vigorously at RT for 1 h, then filtered over Celite and lyophilised to afford the title compound as a white solid.
Yield: 97%
LC-MS (Method 5): Rt 7.92 min, m/z 449 [$M^+$]
$^1$H NMR ($CDCl_3$): δ 1.05-1.42 (m, 7H), 1.59-1.72 (m, 3H), 2.23-2.33 (m, 3H), 3.13 (s, 6H), 3.53 (m, 2H), 4.00 (m, 2H), 4.89 ($dd_{AB}$, 2H), 6.20 (s, 2H), 6.83 (d, 2H), 6.96 (t, 1H), 7.19 (t, 1H), 7.25-7.30 (m, 4H), 7.47 (s, 1H), 7.55 (d, 2H).

## Example 11

Prepared according to the method used in Example 10 but using succinic acid in place of maleic acid was:

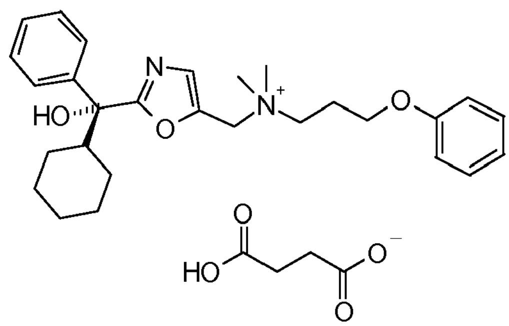

**[2-((R)-Cyclohexyl-hydroxy-phenyl-methyl)-oxazol-5-ylmethyl]-dimethyl-(3-phenoxy-propyl)-ammonium 3-carboxy-propionate. (I-b): $R^a$ =Ph, $R^b$ = c-Hexyl, $R^c$, $R^d$ = $CH_3$, $R^e$ = 3-Phenoxypropyl**

Yield: 97%
LC-MS (Method 5): Rt 7.90 min, m/z 449 [$M^+$]
$^1$H NMR ($CDCl_3$): δ 1.03-1.35 (m, 6H), 1.42-1.45 (m, 1H), 1.59-1.73 (m, 3H), 2.22-2.33 (m, 3H), 2.46 (s, 4H), 3.14 (s, 6H), 3.52 (m, 2H), 4.00 (m, 2H), 4.93 ($dd_{AB}$, 2H), 6.84 (d, 2H), 6.97 (t, 1H), 7.19 (t, 1H), 7.26-7.30 (m, 4H), 7.48 (s, 1H), 7.55 (d, 2H).

## Example 12

Prepared according to the method used in Example 10 but using (S)-malic acid in place of maleic acid was

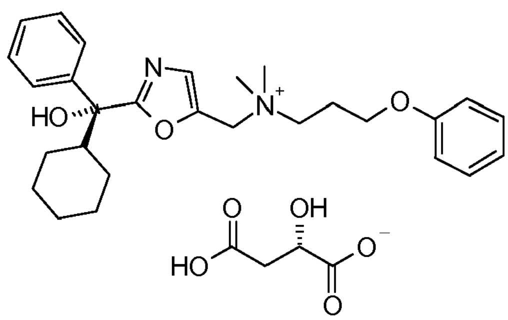

**[2-((R)-Cyclohexyl-hydroxy-phenyl-methyl)-oxazol-5-ylmethyl]-dimethyl-(3-phenoxy-propyl)-ammonium (S)-3-carboxy-2-hydroxy-propionate. (I-b): $R^a$ =Ph, $R^b$ = c-Hexyl, $R^c$, $R^d$ = $CH_3$, $R^e$ = 3-Phenoxypropyl**

Yield: 89%
LC-MS (Method 5): Rt 7.90 min, m/z 449 [$M^+$]
$^1$H NMR ($CDCl_3$): δ 1.02-1.34 (m, 6H), 1.38-1.47 (m, 1H), 1.58-1.72 (m, 3H), 2.17-2.35 (m, 3H), 2.60-2.71 (m, 2H), 3.09 (br.s, 6H), 3.44 (br.s, 2H), 3.98-4.10 (m, 3H), 4.85 (br.s, 2H), 6.84 (d, 2H), 6.96 (t, 1H), 7.18 (t, 1H), 7.25-7.29 (m, 4H), 7.47 (s, 1H), 7.54 (d, 2H).

## Example 13

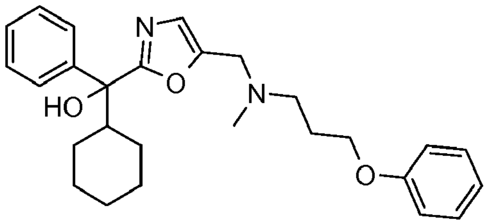

**Cyclohexyl-(5-{[methyl-(3-phenoxy-propyl)-amino]-methyl}-oxazol-2-yl)-phenyl-methanol. (I-a): $R^a$, = Ph, $R^b$ = c-Hexyl, $R^c$ = $CH_3$, $R^d$ = 3-Phenoxypropyl**

A mixture of (5-bromomethyl-oxazol-2-yl)-cyclohexyl-phenyl-methanol (Intermediate 9) (102 mg, 0.286 mmol), *N*-methyl-3-phenoxy-propylamine (57 mg, 0.34 mmol), and diisopropylethylamine (65 μL, 0.37 mmol) in THF (2 mL) wsa stirred at RT for 1.75 h. The reaction mixture was treated with sat. sodium hydrogen carbonate solution (aq) and the organic phase was separated. The aqueous layer was extracted with DCM. The combined organic layers were washed with brine, dried ($Na_2SO_4$), filtered, and concentrated to dryness to afford a pale brown oil. Purification by column chromatography over silica gel using a gradient of 25-30% ethyl acetate/DCM as eluent provided the title compound as a colourless oil.
Yield: 60%
LC-MS (Method 1): Rt 8.61 min, m/z 435 [$M^+$]
$^1$H NMR ($CDCl_3$): δ 1.09-1.39 (m, 7H), 1.61-1.74 (m, 3H), 1.96 (p, 2H), 2.27-2.33 (m, 4H), 2.53 (t, 2H), 3.67 (s, 3H), 3.99 (t, 2H), 6.83 (s, 1H), 6.89 (d, 2H), 6.94 (t, 1H), 7.20-7.33 (m, 5H), 7.63 (d, 2H).

## Example 14

The two enantiomers of cyclohexyl-(5-{[methyl-(3-phenoxy-propyl)-amino]-methyl}-oxazol-2-yl)-phenyl-methanol (Example 13,) were separated in a similar way to those of Example 4 eluting with 10% EtOH/heptane + 0.1% diethylamine. The first eluting enantiomer (Rt 8.3 min) gave **(S)-Cyclohexyl-(5-{[methyl-(3-ahenoxy-propyl)-amino]-methyl}-oxazol-2-yl)-phenyl-methanol. (I-a): $R^a$, = Ph, $R^b$ = c-Hexyl, $R^c$ = $CH_3$, $R^d$ = 3-Phenoxypropyl.**

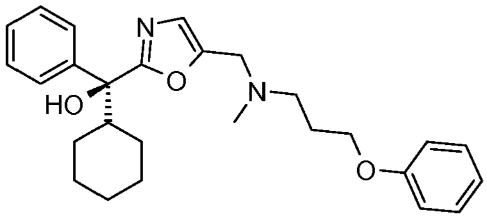

LC-MS (Method 1): Rt 8.44 min, m/z 435 [$MH^+$]
$^1$H NMR ($CDCl_3$): δ 1.09-1.39 (m, 7H), 1.61-1.74 (m, 3H), 1.96 (p, 2H), 2.27-2.33 (m, 4H), 2.53 (t, 2H), 3.67 (s, 3H), 3.99 (t, 2H), 6.83 (s, 1H), 6.89 (d, 2H), 6.94 (t, 1H), 7.20-7.33 (m, 5H), 7.63 (d, 2H).

## Example 15

The second eluting enantiomer (Rt 10.9 min) gave **(R)-Cyclohexyl-(5-{[methyl-(3-phenoxy-propyl)-amino]-methyl}-oxazol-2-yl)-phenyl-methanol. (I-a): $R^a$, = Ph, $R^b$ = c-Hexyl, $R^c$ = $CH_3$, $R^d$ = 3-Phenoxypropyl**

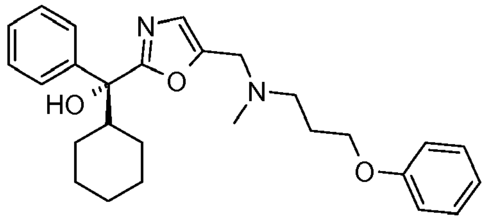

LC-MS (Method 1): Rt 8.51 min, m/z 435 [$MH^+$]
$^1H$ NMR ($CDCl_3$): δ 1.09-1.39 (m, 7H), 1.61-1.74 (m, 3H), 1.96 (p, 2H), 2.27-2.33 (m, 4H), 2.53 (t, 2H), 3.67 (s, 3H), 3.99 (t, 2H), 6.83 (s, 1H), 6.89 (d, 2H), 6.94 (t, 1H), 7.20-7.33 (m, 5H), 7.63 (d, 2H).

The following example compounds were prepared in a similar manner, using the route shown in Scheme 3.

| Example | Name | Structure | $^1H$ NMR | LCMS |
|---|---|---|---|---|
| 16 | Cyclohexyl-(5-methylaminomethyl -oxazol-2-yl)- phenyl-methanol. (1-a): $R^a$ = Ph, $R^b$ = c-Hexyl, $R^c$ = $CH_3$, $R^d$ = H | 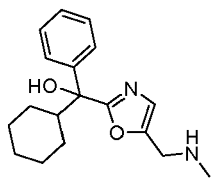 | ($CDCl_3$): δ 1.05-1.42 (m, 7H), 1.57-1.81 (m, 3H), 2.31 (m, 1H), 2.42 (s, 3H), 8.30 (d, 2H), 6.85 (s, 1H), 7.21-7.27 (m, 1H), 7.31-7.37 (m, 2H), 7.60-7.66 (m, 2H). | (Method 3): Rt 2.13 and 2.20min, m/z 301 [$MH^+$] |
| 17 | Cyclopentyl-(5-dimethylaminometh yl-oxazol-2-yl)- phenyl-methanol. (1-a): $R^a$ = Ph, $R^b$ = c-Pentyl, $R^c$ = $R^d$ = $CH_3$ | 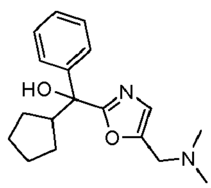 | ($CDCl_3$): δ 1.14-1.76 (9H, m), 2.25 (6H, s), 2.95-3.07 (1H, m), 3.46-3.60 (2H, 2xd), 3.72 (1H, bs), 6.84 (1H, s), 7.19-7.28 (1H, m), 7.28-7.36 (2H, m), 7.59-7.65 (2H, m). | (Method 2): Rt 2.1min, m/z 301 [$MH^+$] |

(continued)

| Example | Name | Structure | $^1H$ NMR | LCMS |
|---|---|---|---|---|
| 18 | Cyclopentyl-(5-dimethylaminometh yl-oxazol-2-yl)- phenyl-methanol - first eluting enantiomer (1-a): $R^a$ = Ph, $R^b$ = c- Pentyl, $R^c$ = $R^d$ = $CH_3$ | 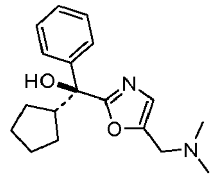 | ($CDCl_3$): δ 1.14-1.76 (9H, m), 2.25 (6H, s), 2.95-3.07 (1H, m), 3.46-3.60 (2H, 2xd), 3.72 (1H, bs), 6.84 (1H, s), 7.19-7.28 (1H, m), 7.28-7.36 (2H, m), 7.59-7.65 (2H, m). | (Method 2): Rt 2.1min, m/z 301 [$MH^+$] |
| 19 | Cyclopentyl-(5-dimethylaminometh yl-oxazol-2-yl)- phenyl-methanol - second eluting enantiomer (1-a): $R^a$ = Ph, $R^b$ = c- Pentyl, $R^c$ = $R^d$ = $CH_3$ | 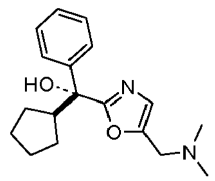 | ($CDCl_3$): δ 1.14-1.76 (9H, m), 2.25 (6H, s), 2.95-3.07 (1H, m), 3.46-3.60 (2H, 2xd), 3.72 (1H, bs), 6.84 (1H, s), 7.19-7.28 (1H, m), 7.28-7.36 (2H, m), 7.59-7.65 (2H, m). | (Method 2): Rt 2.1min, m/z 301 [$MH^+$] |
| 20 | Cyclohexyl-phenyl- (5-piperidin-1- ylmethyl-oxazol-2- yl)-methanol. (1-a): $R^a$ = Ph, $R^b$ = c- Hexyl, $R^c$ = $R^d$ = Piperidinyl | 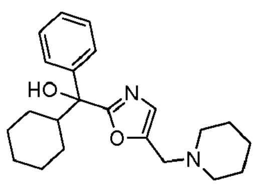 | (CDCb): δ 1.04-1.50 (10H, m), 1.50-1.85 (6H, m), 2.23-2.51 (5H, m),3.61 (2H, s), 3.67 (1H,s), 6.82 (1H, s), 7.19-7.28 (1H, m), 7.28-7.37 (2H, m), 7.60-7.67 (2H, m). | (Method 2): Rt 2.21 and 2.35min, m/z 355 [$MH^+$] |
| 21 | Cyclohexyl-phenyl- (5-piperidin-1- ylmethyl-oxazol-2-yl)-methanol - first eluting enantiomer. (1-a): $R^a$ = Ph, $R^b$ = c-Hexyl, $R^c$ = $R^d$ = Piperidinyl | 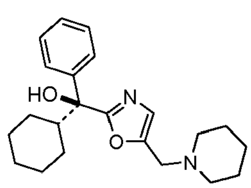 | ($CDCl_3$): δ 1.04-1.50 (10H, m), 1.50-1.85 (6H, m), 2.23-2.51 (5H, m),3.54-3.70 (3H, 2xs), 6.82 (1H, s), 7.19-7.28 (1H, m), 7.28-7.37 (2H, m), 7.60-7.67 (2H, m). | (Method 2): Rt 2.21 and 2.35min, m/z 355 [$MH^+$] |

(continued)

| Example | Name | Structure | $^1$H NMR | LCMS |
|---|---|---|---|---|
| 22 | Cyclohexyl-phenyl- (5-piperidin-1- ylmethyl-oxazol-2- yl)-methanol - second eluting enantiomer. (1-a): $R^a$ = Ph, $R^b$ = c- Hexyl, $R^c$ = $R^d$ = Piperidinyl | 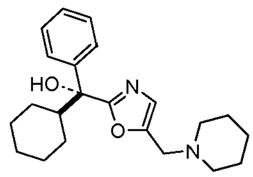 | ($CDCl_3$): δ 1.04-1.50 (10H, m), 1.50-1.85 (6H, m), 2.23-2.51 (5H, m),3.54-3.70 (3H, 2xs), 6.82 (1H, s), 7.19-7.28 (1H, m), 7.28-7.37 (2H, m), 7.60-7.67 (2H, m). | (Method 2): Rt 2.21 and 2.35min, m/z 355 [$MH^+$] |
| 23 | Cyclohexyl-phenyl- (5-pyrrolidin-1- ylmethyl-oxazol-2- yl)-methanol. (1-a): $R^a$ = Ph, $R^b$ = c- Hexyl, $R^c$ = $R^d$ = Pyrrolidinyl | 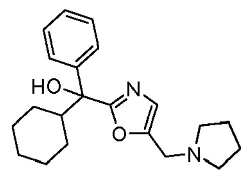 | ($CDCl_3$): δ 1.04-1.44 (8H, m), 1.50-1.86 (6H, m), 2.23-2.37 (1H, m), 2.50-2.64 (4H, m), 3.65 (1H, s), 3.72 (2H, s), 6.85 (1H, s), 7.20-7.28 (1H, m), 7.28-7.36 (2H, m), 7.59-7.66 (2H,m). | (Method 2): Rt 1.84 and 1,98min, m/z 341 [$MH^+$] |
| 24 | Cyclohexyl-phenyl- (5-pyrrolidin-1- ylmethyl-oxazol-2-yl)-methanol - first eluting enantiomer. (1-a): $R^a$ = Ph, $R^b$ = c-Hexyl, $R^c$ = $R^d$ = Pyrrolidinyl | 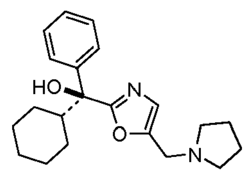 | ($CDCl_3$): δ 1.04-1.44 (8H, m), 1.50-1.86 (6H, m), 2.23-2.37 (1H, m), 2.50-2.64 (4H, m), 3.69 (2H, s), 3.91 (1H, bs), 6.85 (1H, s), 7.20-7.28 (1H, m), 7.28-7.36 (2H, m), 7.59-7.66 (2H,m). | (Method 2): Rt 1.84 and 1.98min, m/z 341 [$MH^+$] |
| 25 | Cyclohexyl-phenyl- (5-pyrrolidin-1- ylmethyl-oxazol-2- yl)-methanol - second eluting enantiomer. (1-a): $R^a$ = Ph, $R^b$ = c- Hexyl, $R^c$ = $R^d$ = Pyrrolidinyl | 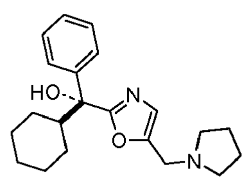 | ($CDCl_3$): δ1.04-1.44 (8H, m), 1.50-1.86 (6H, m), 2.23-2.37 (1H, m), 2.50-2.64 (4H, m), 3.67-3.77 (3H, 2xs), 6.85 (1H, s), 7.20-7.28 (1H, m), 7.28-7.36 (2H, m), 7.59-7.66 (2H,m). | (Method 2): Rt 1.84 and 1.98min, m/z 341 [$MH^+$] |

(continued)

| Example | Name | Structure | $^1$H NMR | LCMS |
|---|---|---|---|---|
| 26 | [5-(4-Phenoxy- piperidin-1-ylmethyl)-oxazol-2-yl]-diphenyl- methanol. (1-a): $R^a$ =$R^b$ = Ph, $R^c$ = $R^d$ = 4-Phenoxy- piperidinyl | 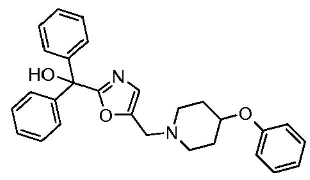 | (CDCb): δ 1.82 (m, 2H), 1.97 (m, 2H), 2.34 (m, 2H), 2.71 (m, 2H), 3.65 (s, 2H), 4.17-4.33 (m, 2H), 6.87 (d, 2H), 6.93 (t, 2H), 7.24-7.38 (m, 12H). | (Method 1): Rt 7.89min, m/z 441 [MH$^+$] |
| 27 | {5-[(Benzyl-methyl-amino)-methyl]- oxazol-2-yl}- cyclopentyl-phenyl-methanol. (I-a): $R^a$ =Ph, $R^b$ = c- Pentyl, $R^c$ = $CH_3$, $R^d$ = Benzyl | 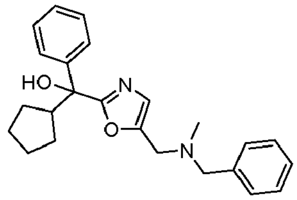 | ($CDCl_3$) δ 1.36, (1H, m), 1.44-1.66 (7H, m), 2.24 (3H, s), 3.02 (1H, m), 3.49 (2H, s), 3.64 (2H, s), 3.69 (1H, s), 6.84 (1H, s), 7.24-7.35 (8H, m), 7.65 (2H, m). | (Method 4) Rt 2.63 min, m/z 377 [MH$^+$] |
| 28 | {5-[(Benzyl-methyl-amino)-methyl]- oxazol-2-yl}- cyclopentyl-phenyl-methanol - second eluting enantiomer. (I-a): $R^a$ = Ph, $R^b$ = c-Pentyl, $R^c$ = $CH_3$, $R^d$ = Benzyl | 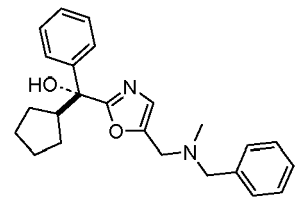 | (DMSO-$d_6$) δ 1.19, (1H, m), 1.37 (2H, m), 1.51 (3H, m), 1.63 (2H, m), 2.08 (3H, s), 2.94 (1H, m), 3.41 (2H, d, J = 2.7Hz), 3.58 (2H, s), 5.99 (1H, s), 7.02 (1H, s), 7.24, (4H, m), 7.31 (4H, m), 7.46 (2H, m). | (Method 1): Rt 7.54min, m/z 377 [MH$^+$] |
| 29 | {5-[(Benzyl-methyl-amino)-methyl]- oxazol-2-yl}- cyclopentyl-phenyl-methanol - first eluting enantiomer. (I-a): $R^a$ = Ph, $R^b$ = c-Pentyl, $R^c$ = $CH_3$, $R^d$ = Benzyl | 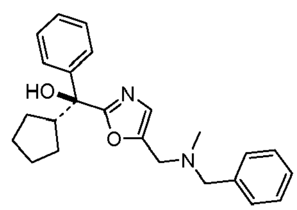 | (DMSO-$d_6$) δ 1.19, (1H, m), 1.38 (2H, m), 1.51 (3H, m), 1.63 (2H, m), 2.08 (s, 3H), 2.94 (1H, m), 3.41 (2H, d, J = 2.8Hz), 3.58 (2H, s), 5.99 (1H, s), 7.02 (1H, s), 7.23, (4H, m), 7.32 (4H, m), 7.46 (2H, m). | (Method 1): Rt 7.29min, m/z 377 [MH$^+$] |
| 30 | [2-(1,1-Diphenyl-ethyl)-oxazol-5-ylmethyl]-dimethyl- amine. (I-j): $R^a$ = $R^b$ = Ph, $R^c$ = $R^d$ = $R^g$ = $CH_3$ | 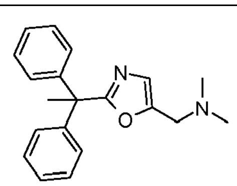 | ($CDCl_3$) δ 2.17 (s, 3H), 2.21 (s, 6H), 3.51 (s, 2H), 6.91 (s, 1H), 7.15 (m, 4H), 7.21-7.32 (m, 6H). | (Method 1): Rt 6.57min, m/z 307 [MH$^+$] |

(continued)

| Example | Name | Structure | $^1$H NMR | LCMS |
|---|---|---|---|---|
| 31 | [2-((S)-Cyclohexyl- hyd roxy-phenyl- methyl)-oxazol-5- ylmethyl]-dimethyl- phenethyl- ammonium bromide. (I-b): $R^a$ =Ph, $R^b$ = c-Hexyl, $R^c$, $R^d$ = $CH_3$, $R^e$ = 2-Phenethyl | 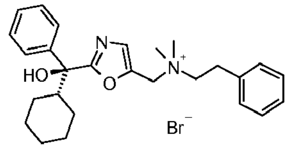 | ($CDCl_3$) δ 1.01-1.39 (m, 7H), 1.65 (m, 3H), 2.30 (m, 2H), 3.13 (t, 2H), 3.32 (s, 3H), 3.33 (s, 3H), 3.66 (m, 2H), 5.21 (dd, 2H), 7.16-7.32 (m, 8H), 7.53 (m, 3H). | (Method 1): Rt 8.05min, m/z 419 [$M^+$] |
| 32 | [2-((R)-Cyclohexyl- hyd roxy-phenyl- methyl)-oxazol-5- ylmethyl]-dimethyl- phenethyl- ammonium bromide. (I-b): $R^a$ =Ph, $R^b$ = c-Hexyl, $R^c$, $R^d$ = $CH_3$, $R^e$ = 2-Phenethyl | 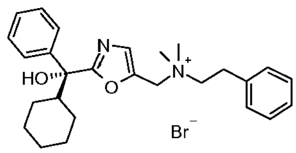 | ($CDCl_3$) δ 1.02-1.38 (m, 8H), 1.66 (m, 3H), 2.30 (br s, 1H), 3.12 (br s, 2H), 3.32 (br s, 6H), 3.67 (br s, 2H), 5.21 (br dd, 2H), 7.16- 7.32 (m, 8H), 7.53 (m, 3H). | (Method 1): Rt 7.93min, m/z 419 [$M^+$] |
| 33 | [2-((R)-Cyclohexyl- hyd roxy-phenyl- methyl)-oxazol-5- ylmethyl]-dimethyl- (4- methyl-pent-3- enyl)-ammonium bromide. (I-b): $R^a$ =Ph, $R^b$ = c-Hexyl, $R^c$, $R^d$ = $CH_3$, $R^e$ = 4-Methyl- pent-3- enyl | 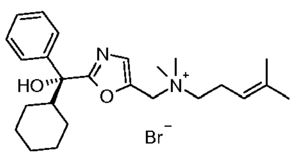 | (MeOD): δ 1.04-1.40 (m, 6H), 1.52-1.81 (m, 10H), 2.41 (m, 1H), 2.49-2.59 (m, 2H), 3.06 (s, 3H), 3.07 (s, 3H), 3.17 (dt, 2H), 4.72 (s, 2H), 4.99 (m, 1H), 7.22-7.28 (m, 1H), 7.29-7.36 (m, 2H), 7.48-7.54 (m, 3H). | (Method 1): Rt 8.12min, m/z 397 [$M^+$] |
| 34 | [2-((R)-Cyclohexyl- hyd roxy-phenyl- methyl)-oxazol-5- ylmethyl]-[2-(2,3- dihydro- benzofuran-5-yl)- ethyl]-dimethyl- ammonium bromide. (I-b): $R^a$ =Ph, $R^b$ = c-Hexyl, $R^c$, $R^d$ = $CH_3$, $R^e$ = 2-(2,3-Dihydro- benzofuran-5-yl)- ethyl | 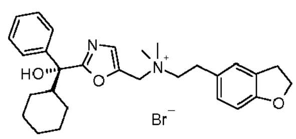 | ($CDCl_3$): δ 51.07-1.36 (m, 8H), 1.59-1.72 (m, 3H), 2.29 (m, 1H), 3.04 (t, 2H), 3.16 (t, 2H), 3.32 (s, 3H), 3.33 (s, 3H), 3.61 (m, 2H), 4.53 (t, 2H), 5.21 ($dd_{AB}$, 2H), 6.68 (d, 1H), 6.96 (d, 1H), 7.15-7.28 (m, 4H), 7.54 (m, 3H). | (Method 1): Rt 8.14 min, m/z 461 [$M^+$] |

(continued)

| Example | Name | Structure | $^1$H NMR | LCMS |
|---|---|---|---|---|
| 35 | [2-((R)-cyclohexyl- hyd roxy-phenyl- methyl)-oxazol-5- ylmethyl]-di methyl-(6- methyl- pyridin-2- ylmethyl )-ammonium formate. (I-b): $R^a$ =Ph, $R^b$ = c-Hexyl, $R^c$, $R^d$ = $CH_3$, $R^e$ = 6-Methyl-pyridin-2-ylmethyl | 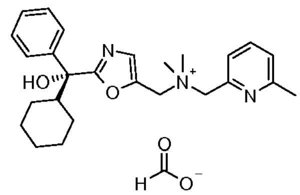 | (MeOD): δ 1.05-1.24 (m, 3H), 1.28-1.40 (m, 3H), 1.68 (m, 4H), 2.42 (m, 1H), 2.60 (s, 3H), 3.06 (s, 3H), 3.07 (s, 3H), 4.48 (s, 2H), 7.25 (tt, 1H), 7.29-7.36 (m, 2H), 7.39 (dd, 2H) 7.50-7.56 (m, 2H), 7.60 (s, 1H), 7.81 (t, 1H), 8.53 (br s, 1H). | (Method 1): Rt 7.57min, m/z 420 [$M^+$] |
| 36 | [2-(Cyclopentyl- hyd roxy- phenyl- methyl)-oxazol-5- ylmethyl]-dimethyl- (3- phenoxy- propyl)-ammonium bromide (I-b): Ra =Ph, $R^b$ = c-Pentyl, $R^c$, $R^d$ = $CH_3$, $R^e$ = 3-Phenoxypropyl | 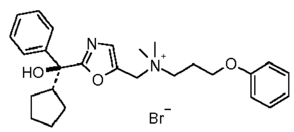 | ($CDCl_3$) δ 1.24-1.49 (4H, m), 1.51-1.69 (4H, m), 2.32 (2H, m), 3.00 (1H, m), 3.34 (6H, s), 3.64 (2H, m), 4.03 (2H, m), 5.22 (2H, m), 6.85 (2H, d), 6.88 (1H, t), 7. 20 (1H, t), 7.25-7.32 (5H, m), 7.52-7.60 (3H, m). | (Method 1): Rt 7.99min, m/z 436 [$M^+$] |
| 37 | [2-(Cyclopentyl- hyd roxy- phenyl- methyl)-oxazol-5- ylmethyl]-dimethyl- (3- phenoxy- propyl)-ammonium bromide (I-b): $R^a$ =Ph, $R^b$ = c-Pentyl, $R^c$, $R^d$ = $CH_3$, $R^e$ = 3-Phenoxypropyl | 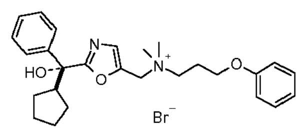 | ($CDCl_3$) δ 1.20-1.80 (8H), 2.14 (2H, m), 3.00 (1H, m), 3.35 (6H, s), 3.64 (3H, m), 4.04 (2H, s), 5.21 (2H, m), 6.85 (2H, d), 6.98 (1H, t), 7.17-7.32 (5H, m), 7.50-7.62 (3H, m). | (Method 1): Rt 7.96min, m/z 436 [$M^+$] |
| 38 | 1-[2-(Cyclohexyl- hyd roxy- phenyl- methyl)-oxazol-5- ylmethyl]-1-(3- phenoxy- propyl)- piperidinium bromide. (I-b): $R^a$ =Ph, $R^b$ = c-Hexyl, $R^c$, $R^d$ = Piperidinyl, $R^e$ = 3- Phenoxypropyl | 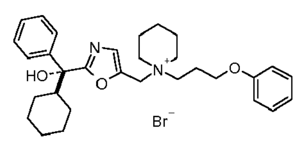 | ($CDCl_3$) δ 1.01-1.38 (8H, m), 1.56-1.99 (8H, m), 2.35-2.38 (3H, m), 3.41-3.57 (4H, m), 3.65 (1H, s), 3.78-4.03 (4H, m), 5.19 (1H, d), 5.34 (1H, d), 6.81 (2H, m), 6.97 (1H, t), 7.17-7.31 (5H, m), 7.50-7.60 (3H, m). | (Method 1): Rt 8.77min, m/z 489 [$M^+$] |

(continued)

| Example | Name | Structure | $^1$H NMR | LCMS |
|---|---|---|---|---|
| 39 | 1-[2-(Cyclohexyl- hyd roxy-phenyl- methyl)-oxazol-5-ylmethyl]-1-(3- phenoxy-propyl)- piperidinium bromide. (I-b): $R^a$ =Ph, $R^b$ = c-Hexyl, $R^c$, $R^d$ = Piperidinyl, $R^e$ = 3-Phenoxypropyl | 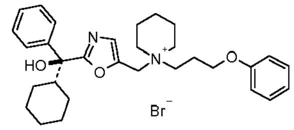 | ($CDCl_3$) δ 1.01-1.38 (8H, m), 1.56-1.99 (8H, m), 2.35-2.38 (3H, m), 3.41-3.57 (4H, m), 3.71-4.10 (5H,), 5.19 (1H, d), 5.34 (1H, d), 6.81 (2H, m), 6.97 (1H, t), 7.17- 7.31 (5H, m), 7.50- 7.60 (3H, m). | (Method 1): Rt 8.78min, m/z 489 [M+] |
| 40 | 1-[2-(Cyclohexyl- hyd roxy-phenyl- methyl)-oxazol-5-ylmethyl]-1-(3- phenoxy-propyl)- pyrrolidinium bromide. (I-b): $R^a$ =Ph, $R^b$ = c-Hexyl, $R^c$, $R^d$ = Pyrrolidinyl, $R^e$ = 3-Phenoxypropyl | 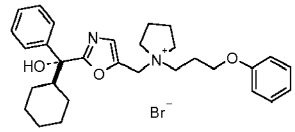 | ($CDCl_3$) δ 1.03-1.39 (8H, m), 1.53-1.80 (2H, m), 2.05-2.44 (7H, m), 3.48 (2H, m), 3.62 (2H, m), 3.80 (1H, s), 3.99-4.18 (4H, m), 5.16 (1H, d), 5.29 (1H, d), 6.84 (2H,m), 6.99 (1H, t), 7.19-7.34 (5H, m), 7.54-7.60 (3H, m). | (Method 1): Rt 8.75min, m/z 475 [M+] |
| 41 | 1-[2-(Cyclohexyl- hyd roxy-phenyl- methyl)-oxazol-5-ylmethyl]-1-(3- phenoxy-propyl)- pyrrolidinium bromide. (I-b): $R^a$ =Ph, $R^b$ = c-Hexyl, $R^c$, $R^d$ = Pyrrolidinyl, $R^e$ = 3-Phenoxypropyl | 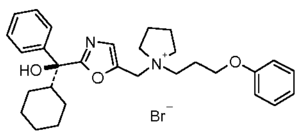 | ($CDCl_3$) δ 1.03-1.39 (8H, m), 1.53-1.80 (2H, m), 2.05-2.44 (7H, m), 3.48 (2H, m), 3.62 (2H, m), 3.80 (1H, s), 3.99-4.18 (4H, m), 5.16 (1H, d), 5.29 (1H, d), 6.84 (2H,m), 6.99 (1H, t), 7.19-7.34 (5H, m), 7.54-7.60 (3H, m). | (Method 1): Rt 8.57min, m/z 475 [M+] |

(continued)

| Example | Name | Structure | $^1$H NMR | LCMS |
|---|---|---|---|---|
| 42 | [2-((R)-Cyclohexyl- hyd roxy-phenyl-methyl)-oxazol-5-ylmethyl]-(8- methoxy-octyl)- dimethyl- ammonium formate. (I-b). $R^a$ =Ph, $R^b$ = c-Hexyl, $R^c$, $R^d$ = $CH_3$, $R^e$ = 8-Methoxyoctyl | 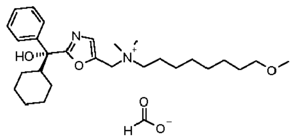 | ($CDCl_3$) δ 0.99-1.42 (14H, m), 1.44-1.83 (8H, m), 2.29 (1H, m), 2.78-3.27 (8H, m), 3.28-3.46 (5H,m), 4.95 (2H, m), 7.16- 7.34 (3H, m), 7.43 (1H, m), 7.55 (2H, d), 8.67 (1H, s). | (Method 1): Rt 8.50min, m/z 457 [$M^+$] |
| 43 | [2-((R)-Cyclohexyl- hyd roxy-phenyl-methyl)-oxazol-5-ylmethyl]-dimethyl- (4-phenoxy-butyl)- ammonium bromide. (I-b): $R^a$ =Ph, $R^b$ = c-Hexyl, $R^c$, $R^d$ = $CH_3$, $R^e$ = 4-Phenoxybutyl | 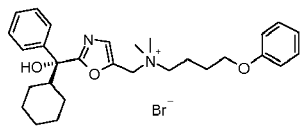 | ($CDCl_3$) δ 1.05-1.88 (12H, m), 1.96-2.08 (2H,m), 2.34 (1H, m), 3.31 (6H, 2 x s), 3.44- 3.59 (2H, m), 3.78 (1H, s), 4.01 (2H, t), 5.15 (1H, d), 5.32 (1H, d), 6.87 (2H, d), 6.97 (1H, t), 7.20-7.36 (5H, m), 7.52-7.60 (3H, m). | (Method 1): Rt 8.85min, m/z 463 [$M^+$] |
| 44 | (2-Benzyloxy-ethyl)-[2-((R)- cyclohexyl-hyd roxy-phenyl-methyl)-oxazol-5-ylmethyl]-dimethyl-ammonium bromide. (I-b): $R^a$ =Ph, $R^b$ = c-Hexyl, $R^c$, $R^d$ = $CH_3$, $R^e$ = 2-Benzyloxyethyl | 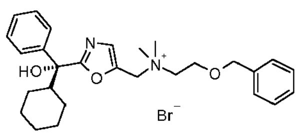 | ($CDCl_3$) δ 1.04-1.83 (10H, m), 2.31 (1H, m), 3.30 (6H, s), 3.72 (1H,s), 3.82-4.00 (4H, m), 4.56 (2H, s), 4.98- 5.26 (2H, m), 7.20- 7.41 (8H, m), 7.44 (1H, s), 7.56 (2H, d). | (Method 5): Rt 7.52min, m/z 499 [$M^+$] |
| 45 | [2-((R)-Cyclohexyl- hyd roxy-phenyl-methyl)-oxazol-5-ylmethyl]-dimethyl- (4-phenyl-butyl)- ammonium bromide. (I-b): $R^a$ =Ph, $R^b$ = c-Hexyl, $R^c$, $R^d$ = $CH_3$, $R^e$ = 4-Phenylbutyl | 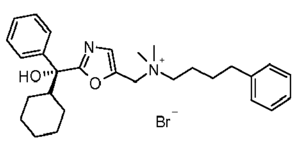 | ($CDCl_3$) δ 1.04-1.85 (14H, m), 2.31 (1H, m), 2.70 (2H, t), 3.12- 3.44 (8H, m), 3.65 (1H, s), 5.10 (1H, d), 5.28 (1H, d), 7.11- 7.37 (8H, m), 7.46 (3H, m). | (Method 5): Rt 7.79min, m/z 447 [$M^+$] |

(continued)

| Example | Name | Structure | $^1$H NMR | LCMS |
|---|---|---|---|---|
| 46 | [2-((R)-Cyclohexyl- hyd roxy-phenyl-methyl)-oxazol-5-ylmethyl]-[3-(4- fluoro-phenoxy)- propyl]-dimethyl-ammonium bromide. (I-b): $R^a$ =Ph, $R^b$ = c-Hexyl, $R^c$, $R^d$ = $CH_3$, $R^e$ = 3-(4-Fluorophenoxy)- propyl | 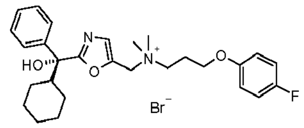 | ($CDCl_3$) δ 1.01-1.41 (8H, m), 1.56-1.80 (2H, m), 2.25-2.39 (3H, m), 3.34 (6H, s), 3.59-3.70 (2H, m), 3.92-4.05 (3H, m), 5.19 (1H, d), 5.30 (1H, d), 6.75-6.82 (2H, m), 6.92-7.01 (2H, m), 7.17-7.33 (3H, m), 7.51-7.60 (3H, m). | (Method 5): Rt 7.51 min, m/z 467 [$M^+$] |
| 47 | [8-(tert- butoxycarbonyl-methyl-amino)-octyl]-[2-((R)- cyclohexyl-hyd roxy-phenyl-methyl)-oxazol-5-ylmethyl]-dimethyl-ammonium methanesulfonate. (I-b): $R^a$ =Ph, $R^b$ = c-hexyl, $R^c$, $R^d$ = Me, $R^e$ = 8-(tert-butoxycarbonyl- methyl-amino)- octyl. | 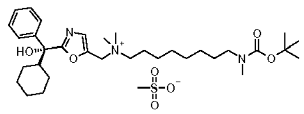 | ($CDCl_3$) δ 1.02-1.81 (31H, m), 2.30 (1H, m), 2.70 (3H, s), 2.83 (3H, s), 2.97-3.32 (10H, m), 4.82-5.06 (2H, m), 7.17-7.37 (3H, m), 7.43-7.64 (3H, m). | (Method 5): Rt 8.50min, m/z 556 [M+] |
| 48 | [2-((R)-cyclohexyl- hyd roxy-phenyl-methyl)-oxazol-5-ylmethyl]-dimethyl- (3-phenyl-propyl)- ammonium formate. (I-b): $R^a$ =Ph, $R^b$ = c-Hexyl, $R^c$, $R^d$ = $CH_3$, $R^e$ = 3-Phenylpropyl. | 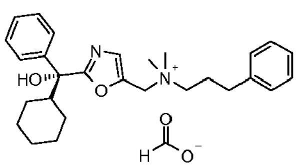 | ($CDCl_3$) δ 0.98-1.35 (6H, m), 1.46-1.80 (4H, m), 1.88-2.06 (2H, m), 2.28 (1H, s), 2.55 (2H, b s), 2.97 (6H, s), 3.07-3.27 (2H, m), 4.47-5.04 (2H, b s), 7.06-7.39 (9H, m), 7.46-7.61 (2H, m), 8.67 (1H, b s). | (Method 1): Rt 8.27min, m/z 433 [$M^+$] |

(continued)

| Example | Name | Structure | $^1$H NMR | LCMS |
|---|---|---|---|---|
| 49 | [2-((R)-cyclohexyl- hyd roxy-phenyl- methyl)-oxazol-5-ylmethyl]-dimethyl- (2-phenoxy-ethyl)- ammonium formate. (I-b): $R^a$ =Ph, $R^b$ = c-Hexyl, $R^c$, $R^d$ = $CH_3$, $R^e$ = 2-Phenoxyethyl | 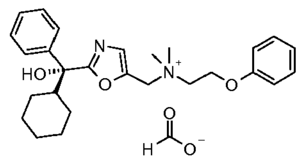 | ($CDCl_3$) δ 0.98-1.35 (6H, m), 1.50-1.77 (4H, m), 2.29 (1H, m), 3.14 (6H, s), 3.86 (2H, b s), 4.35 (2H, b s), 4.93 (2H, b s), 6.87 (2H, d), 6.96-7.03 (1H, m), 7.13- 7.19 (5H,m), 7.44 (1H, s), 7.54 (2H, d), 8.64 (1H b s) | (Method 1): Rt 8.09min, m/z 435 [M+] |
| 50 | (2-{4-[(Tert-butoxycarbonyl- methyl-amino)- methyl]-phenyl}-ethyl)-[2-((R)- cyclohexyl-hyd roxy-phenyl-methyl)-oxazol-5-ylmethyl]-dimethyl-ammonium methanesulfonate. (I-b): $R^a$ =Ph, $R^b$ = c-Hexyl, $R^c$, $R^d$ = $CH_3$, $R^e$ = 2-{4- [(Tert-butoxycarbonyl- methyl-amino)- methyl]-phenyl}-ethyl | 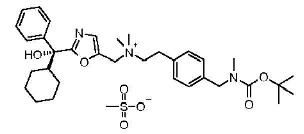 | ($CDCl_3$) δ 1.00-1.80 (19H, m), 2.29 (1H, m), 2.73 (3H, s), 2.80 (3H, s), 3.09 (2H, t), 3.18 (6H, s), 3.43- 3.58 (2H, m), 4.34 (1H, s), 4.38 (2H, s), 4.95 (1H, d), 5.05 (1H, d), 7.16-7.29 (7H, m), 7.51-7.57 (3H, m). | (Method 5): Rt 8.11 min, m/z 562 [M+] |
| 51 | [3-(4-Carbamoyl-phenoxy)-propyl]-[2-((R)-cyclohexyl- hyd roxy-phenyl-methyl)-oxazol-5-ylmethyl]-dimethyl-ammonium bromide. (I-b): $R^a$ =Ph, $R^b$ = c-Hexyl, $R^c$, $R^d$ = $CH_3$, $R^e$ = 3-(4-Carbamoyl- phenoxy) propyl | 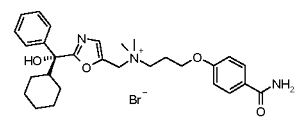 | (DMSO-$d_6$) δ 0.90- 1.30 (6H, m), 1.49- 1.75 (4H, m), 2.17-2.31 (3H, m), 3.04 (6H, s), 3.28-3.43 (2H, m), 4.06 (2H, t), 4.77 (2H, s), 6.09 (1H, s), 6.93-6.99 (2H, m), 7.15-7.26 (2H, m), 7.27-7.35 (2H, m), 7.44-7.50 (2H, m), 7.54 (1H, s), 7.77-7.91 (3H, m). | (Method 5): Rt 6.11 min, m/z 792 [M+] |

(continued)

| Example | Name | Structure | $^1H$ NMR | LCMS |
|---|---|---|---|---|
| 52 | [2-((R)-Cyclohexyl- hyd roxy-phenyl-methyl)-oxazol-5-ylmethyl]-(isoxazol- 3-ylcarbamoylmethyl) -dimethyl- ammonium bromide. (I-b): $R^a$ =Ph, $R^b$ = c-Hexyl, $R^c$, $R^d$ = $CH_3$, $R^e$ = Isoxazol-3-ylcarbamoylmethyl | 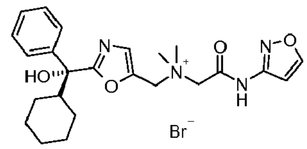 | ($CDCl_3$) δ 0.99-1.83 (10H, m), 3.22 (1H, m), 3.44 (6H, s), 4.28 (1H, s), 5.04 (2H, s), 5.16-5.27 (2H, m), 6.77 (1H, d), 7.17- 7.22 (1H, m), 7.27- 7.33 (2H, m), 7.50-7.58 (3H, m), 8.21 (1H, d), 11.40 (1H,s). | (Method 1): Rt 6.93min, m/z 439 [$M^+$] |
| 53 | [2-((R)-Cyclohexyl- hyd roxy-phenyl-methyl)-oxazol-5-ylmethyl]-[3-(4-methoxycarbonyl-phenoxy)-propyl]- dimethyl-ammonium bromide. (I-b): $R^a$ =Ph, $R^b$ = c-Hexyl, $R^c$, $R^d$ = $CH_3$, $R^e$ = 3-(4-Methoxycarbonyl-phenoxy)propyl | 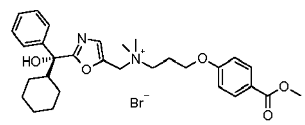 | ($CDCl_3$) δ 1.02-1.40 (8H, m), 1.56-1.80 (2H, m), 2.26-2.42 (3H, m), 3.35 (6H, s), 3.60-3.76 (2H, m), 3.89 (3H, s), 3.98 (1H, s), 4.03-4.14 (2H, m), 5.20 (1H, d), 5.30 (1H, d), 6.87 (2H, d), 7.17- 7.24 (1H, m), 7.24-7.33 (2H, m), 7.53- 7.64 (3H, m), 7.98 (2H, d). | (Method 1): Rt 8.02min, m/z 507 [$M^+$] |
| 54 | [2-((R)-Cyclohexyl- hyd roxy-phenyl-methyl)-oxazol-5-ylmethyl]-dimethyl- [3-(4-sulfamoyl-phenoxy)-propyl]-ammonium bromide. (I-b): $R^a$ =Ph, $R^b$ = c-Hexyl, $R^c$, $R^d$ = $CH_3$, $R^e$ = 3-(4-Sulfamoyl- phenoxy)propyl | 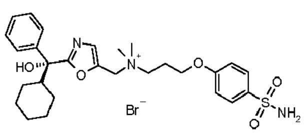 | (MeOD) δ 1.02-1.42 (6H, m), 1.51-1.82 (4H, m), 2.27-2.46 (3H, m), 3.12 (6H, m), 3.34-3.54 (2H, m), 4.04-4.17 (2H, m), 4.78 (2H, s), 6.98- 7.07 (2H, m), 7.17-7.35 (3H, m), 7.47-.54 (3H, m), 7.78-7.88 (2H, m). | (Method 1): Rt 7.05min, m/z 528 [$M^+$] |

(continued)

| Example | Name | Structure | $^1$H NMR | LCMS |
|---|---|---|---|---|
| 55 | [2-((R)-Cyclohexyl- hyd roxy-phenyl-methyl)-oxazol-5-ylmethyl]-dimethyl- (3-p-tolyloxy-propyl)-ammonium bromide. (I-b): $R^a$ =Ph, $R^b$ = c-Hexyl, $R^c$, $R^d$ = $CH_3$, $R^e$ = 3-*p*-Tolyloxypropyl | 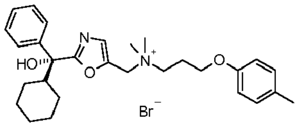 | ($CDCl_3$) δ 1.00-1.39 (7H, m), 1.50-1.77 (3H, m), 2.22-2.37 (6H, m), 3.30 (3H, s), 3.32 (3H, s), 3.57 (2H, m), 3.75 (1H, s), 3.94- 4.02 (2H, m), 5.17 (1H, d), 5.34 (1H, d), 6.71 (2H, d), 7.05 (2H, d), 7.15-7.30 (3H, m), 7.51-7.57 (3H, m). | (Method 5): Rt 7.88min, m/z 463 [$M^+$] |
| 56 | [3-(4-Chloro-phenoxy)-propyl]-[2-((R)-cyclohexyl- hyd roxy-phenyl-methyl)-oxazol-5-ylmethyl]-dimethyl-ammonium bromide. (I-b): $R^a$ =Ph, $R^b$ = c-Hexyl, $R^c$, $R^d$ = $CH_3$, $R^e$ = 3-(4-Chloro-phenoxy)propyl | 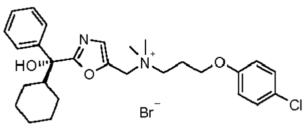 | ($CDCl_3$) δ 1.02-1.41 (7H, m), 1.57-1.79 (3H, m), 2.58-2.38 (3H, m), 3.33 (6H, s), 3.61-3.70 (2H, m), 3.94-4.04 (3H, m), 5.18 (1H, d), 5.30 (1H, d), 6.75-6.81 (2H, m), 7.18-7.32 (5H, m), 7.53-7.59 (3H, m). | (Method 1): Rt 8.07min, m/z 483 [$M^+$] |
| 57 | [2-((R)-Cyclohexyl- hyd roxy-phenyl-methyl)-oxazol-5-ylmethyl]-[3-(3,4- dichloro-phenoxy)-propyl]-dimethyl-ammonium bromide. (I-b): $R^a$ =Ph, $R^b$ = c-Hexyl, $R^c$, $R^d$ = $CH_3$, $R^e$ = 3-(3,4-Dichloro- phenoxy)propyl | 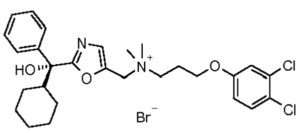 | ($CDCl_3$) δ 1.02-1.40 (8H, m), 1.52-1.80 (2H), 2.26-2.39 (3H, m), 3.34 (6H, s), 3.60- 3.73 (2H, m), 3.86 (1H, s), 3.95-4.04 (2H, m), 5.20 (1H, d), 5.33 (1H, d), 6.69-6.75 (1H, m), 6.95 (1H, d), 7.19-7.36 (4H, m), 7.53-7.59 (3H, m). | (Method 5): Rt 8.62min, m/z 517 [$M^+$] |

Also prepared using the route shown in Scheme 3 were:

**Example 58**

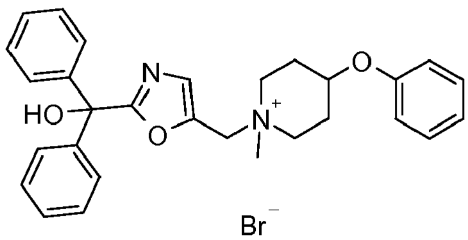

**1-[2-(Hydroxy-diphenyl-methyl)-oxazol-5-ylmethyl]-1-methyl-4-phenoxy-piperidinium bromide. (I-b): $R^a = R^b =$ Ph, $R^c$, $R^d$ = Piperidinyl, $R^e = CH_3$**

To a solution of [5-(4-phenoxy-piperidin-1-ylmethyl)-oxazol-2-yl]-diphenyl-methanol (Example 26) (35mg, 0.08mmol) in 0.5mL acetonitrile was added 1.5mL (- 5mmol) of a 40 wt % solution of methyl bromide in acetonitrile. The reaction mixture was heated at 40°C in a sealed vial for 18h during which a white precipitate formed. The solvent was evaporated and the residue triturated with diethyl ether. The solid was recrystallised from acetonitrile to give the title compound as an 8:1 mixture of cis:trans isomers as a white solid.
Yield: 6mg (14%)
LCMS (Method 1): Rt 7.93min, m/z 455 [$M^+$]
$^1H$ NMR ($CDCl_3$) $\delta$ 2.11-2.37 (m, 4H), 3.15 (s, 3H, minor isomer), 3.30 (s, 3H), 3.58 (br s, 4H), 3.67 (br s, 4H, minor isomer), 4.67 (br s, 1H), 4.71 (br s, 1H, minor isomer), 5.04 (br s, 2H), 5.23 (br s, 2H, minor isomer), 6.87 (d, 2H), 6.91 (d, 2H, minor isomer), 7.02 (t, 1H), 7.26-7.38 (m, 12H), 7.58 (br s, 1H), 7.63 (br s, 1H, minor isomer).

**Example 59**

Prepared according to the method used in Example 58 but using Example 28 in place of Example 26 was

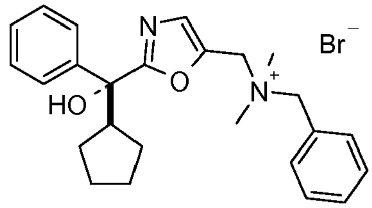

**Benzyl-[2-(cyclopentyl-hydroxy-phenyl-methyl)-oxazol-5-ylmethyl]-dimethylammonium bromide. (I-b): $R^a$ = Ph, $R^b$ = c-Pentyl $R^c$, $R^d = CH_3$, $R^e$ = Benzyl**

Yield: 11 mg, (17%)
LC-MS (Method 1): Rt 6.70min, m/z 391 [$M^+$]
$^1H$ NMR (DMSO-$d_6$) $\delta$ 1.22 (1H, m), 1.38 (2H, m), 1.52 (3H, m), 1.68 (2H, m), 2.88 (3H, s), 2.92 (3H, s) 2.97 (1H, m), 4.55 (2H, s), 4.70 (2H, s), 6.18 (1H, s), 7.25 (1H, m), 7.34, (2H, m), 7.48-7.58 (7H, m).

**Example 60**

Prepared according to the method used in Example 58 but using Example 29 in place of Example 26 was

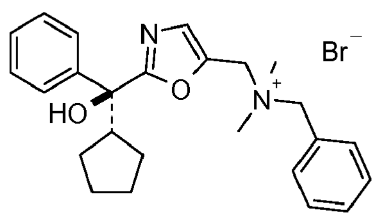

**Benzyl-[2-(cyclopentyl-hydroxy-phenyl-methyl)-oxazol-5-ylmethyl]-dimethylammonium bromide. (I-b): $R^a$ = Ph, $R^b$ = c-Pentyl, $R^c$ = $R^d$ = $CH_3$, $R^e$ = Benzyl**

Yield: 27 mg, (58%)
LC-MS (Method 1): Rt 7.30min, m/z 391 [$M^+$]
$^1$H NMR (DMSO-$d_6$) δ 1.23 (1H, m), 1.37 (2H, m), 1.52 (3H, m), 1.67 (2H, m), 2.88 (3H, s), 2.92 (3H, s) 2.97 (1H, m), 4.55 (2H, s), 4.70 (2H, s), 6.18 (1H, s), 7.25 (1H, m), 7.34, (2H, m), 7.48-7.58 (7H, m).

**Example 61**

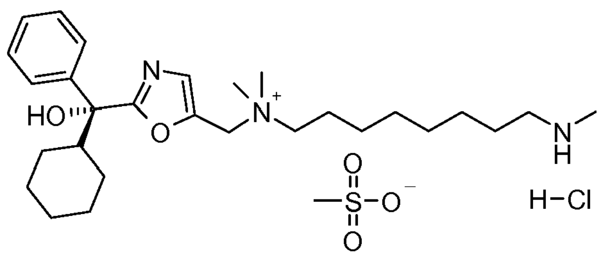

**[2-((R)-cyclohexyl-hydroxy-phenyl-methyl)-oxazol-5-ylmethyl]-dimethyl-(8-methylamino-octyl)-ammonium methanesulfonate hydrochloride salt. (I-b): $R^a$ = Ph, $R^b$ = c-Hexyl $R^c$ = $R^d$ = $CH_3$, $R^e$ = 8-Methylamino-octyl**

**[8-(tert-butoxycarbonyl-methyl-amino)-octyl]-[2-((R)-cyclohexyl-hydroxy**phenyl-methyl)-oxazol-5-ylmethyl]-dimethyl-ammonium methanesulfonate (Example 47) (60mg, 0.09mmol) was dissolved in 6mL 1M HCl in dioxane. The solution was stirred at ambient temperature overnight. The solvent was removed and the crude product was purified by silica gel column chromatography, eluting with a gradient DCM to 20%MeOH/DCM to give title compound as a solid.
Yield: 21 mg (42%)
LC-MS (Method 1): Rt 5.50, m/z 456 [$M^+$]
$^1$H NMR ($CDCl_3$) δ 1.01-2.19 (22H, m), 2.33 (1H,m), 2.64 (3H, s), 2.69 (3H, s), 2.21 (2H, m), 3.16 (6H, s), 3.35-3.50 (2H, m), 4.78-5.08 (2H, m), 7.16-7.36 (3H, m), 7.46-7.60 (3H,m), 9.31 (2H, s).

**Example 62**

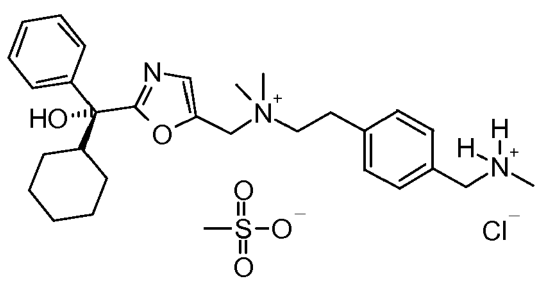

Prepared according to the method used in Example 61 but using Example 50 in place of Example 47 was **[2-((R)-cyclohexyl- hydroxy- phenyl- methyl)-oxazol- 5- ylmethyl]-dimethyl-[2-(4- methylaminomethyl- phenyl)-ethyl]-ammonium methanesulfonate hydrochloride. (I-b): $R^a$ =Ph, $R^b$ = c-Hexyl, $R^c$, $R^d$ = $CH_3$, $R^e$ = 2-(4-Methylaminomethyl-phenyl)-ethyl**
Yield: 51 %
LC-MS (Method 5): Rt 4.64min, m/z 462 [$M^+$]
$^1$H NMR (MeOD) δ 1.01-1.41 (6H, m), 1.50-1.81 (4H, m), 2.39 (1H, m), 2.69 (3H, s), 2.71 (3H, s), 3.08-3.27 (8H, m), 3.39-3.57 (2H, m), 4.19 (2H, s), 7.20-7.35 (3H, m), 7.39-7.59 (7H, m).

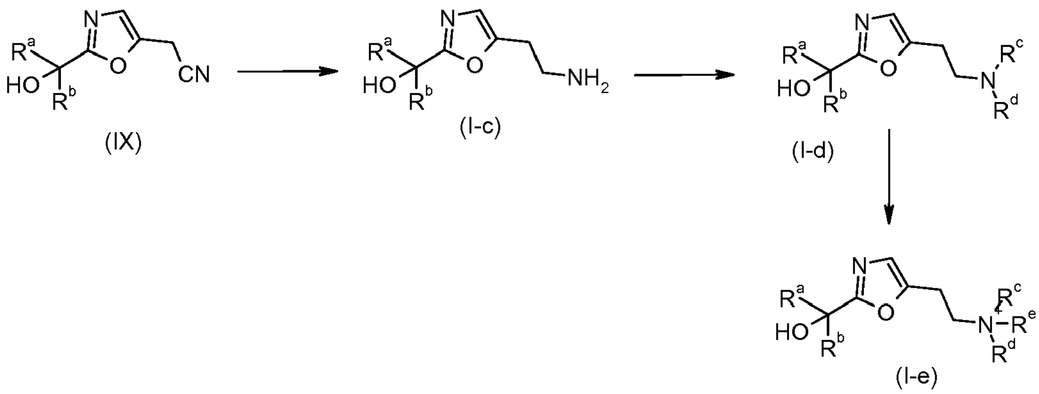
(IX) (I-c) (I-d) (I-e)

## Scheme 7

The following example compounds were prepared using the route shown in Scheme 7.

**Example 63**

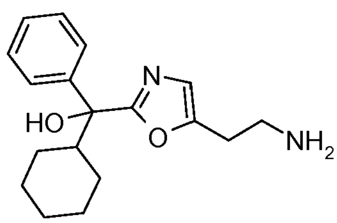

**[5-(2-Amino-ethyl)-oxazol-2-yl]-cyclohexyl-phenyl-methanol. (I-c): $R^a$ = Ph, $R^b$ = c-Hexyl**

To a solution of [2-(cyclohexyl-hydroxy-phenyl-methyl)-oxazol-5-yl]-acetonitrile (Intermediate 12) (600 mg, 2.0 mmol) in THF (20 mL) heated at 55 °C was added dropwise a solution of borane-dimethylsulphide complex (2 mL of 2 M in THF, 4 mmol). The mixture was heated at reflux for 90 minutes then allowed to cool to room temperature. The mixture was then cooled in an ice-bath and quenched by the dropwise addition of methanol (5 mL) followed by hydrochloric acid (1 N, 2 mL). This mixture was stirred for 30 minutes then neutralised with sat. aqueous sodium hydrogen carbonate. The mixture was partitioned between water (80 mL) and ethyl acetate (80 mL), the aqueous extracted with ethyl acetate (2 x 50 mL) and the combined organics dried over sodium sulphate, filtered and evaporated to an oil. The residue was purified by SCX cartridge, washing with methanol then eluting with 4N ammonia in methanol to give the title compound as a colourless oil.
Yield: 400 mg (67%)
LC-MS (Method 2): Rt 2.18 min, m/z 283 [$MH^+$-$H_2O$]
$^1H$ NMR ($CDCl_3$) δ 1.10-1.38 (7 H, m), 1.54-1.77 (3 H, m), 2.28 (1H, m), 2.80 (2H, t), 2.98 (2H, t), 3.67 (1H, br s), 6.72 (1H, s), 7.24 (1H, m), 7.34 (2H, m), 7.62 (2H, m).

**Example 64**

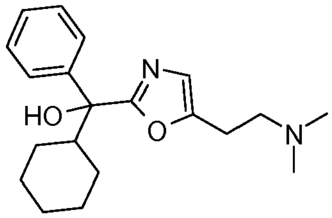

**Cyclohexyl-[5-(2-dimethylamino-ethyl)-oxazol-2-yl]-phenyl-methanol. (I-d): $R^a$ = Ph, $R^b$ = c-Hexyl, $R^c$ = $R^d$ = $CH_3$**

To a solution of [5-(2-amino-ethyl)-oxazol-2-yl]-cyclohexyl-phenyl-methanol (Example 63) (250 mg, 0.83 mmol) in 1,2-dichloroethane (5 mL) was added formaldehyde (0.3 mL of 37 % solution in water, 4.0 mmol) and sodium triacetoxyborohydride (352 mg, 1.7 mmol). This mixture was stirred at room temperature for 6 hours then DCM (10 mL) and sat. aqueous sodium hydrogen carbonate (10 mL) were added and mixed thoroughly. The organics were isolated through a phase separation cartridge and evaporated to an oil. Purification by flash column chromatography over silica gel using a mixture of 10 % methanol : 90 % DCM as eluent gave the title compound as a white solid.
Yield: 200 mg (73%)
LC-MS (Method 2): Rt 2.20 min, m/z 329 [$MH^+$]
NMR corresponds to Example 66.

**Example 65**

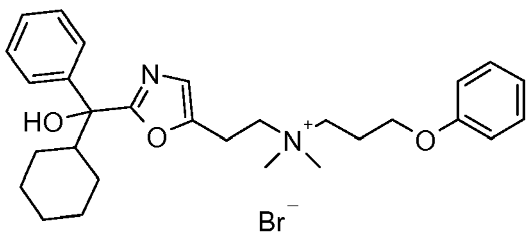

**{2-[2-(Cyclohexyl-hydroxy-phenyl-methyl)-oxazol-5-yl]-ethyl}-dimethyl-(3-phenoxy-aropyl)-ammonium bromide. (I-e): $R^a$ = Ph, $R^b$ = c-Hexyl, $R^c$ = $R^d$ = $CH_3$, $R^e$ = 3-Phenoxyaropyl**

To a solution of cyclohexyl-[5-(2-dimethylamino-ethyl)-oxazol-2-yl]-phenyl-methanol (Example 64) (10.5 mg, 0.03 mmol) in a mixture of acetonitrile (0.5 mL) and chloroform (0.75 mL) was added 3-phenoxypropyl bromide (50 μL, 0.32 mmol). This mixture was heated at 50 ˚C for 6 hours then the solvent removed *in vacuo.* Purifcation by flash column chromatography over silica gel using a gradient of 2-10 % methanol in DCM as eluent gave the title compound as a white solid.
Yield: 8.6 mg (53%)
LC-MS (Method 1): Rt 8.66 min, m/z 463 [$M^+$]
$^1$H NMR (MeOD) δ 1.06-1.35 (6H, m), 1.66 (4H, m), 2.24 (2H, m), 2.37 (2H, m), 3.17 (6H, s), 3.29 (1H, m), 3.59 (2H, m), 3.66 (2H, m), 4.06 (2H, t), 6.91-6.97 (3H, m), 6.99 (1H, s), 7.20-7.33 (5H, m), 7.50 (2H, m).

**Example 66**

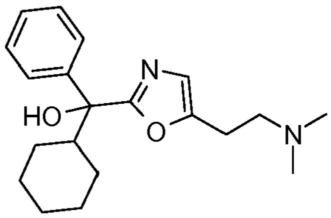

**[5-(2-Amino-ethyl)-oxazol-2-yl]-cyclohexyl-phenyl-methanol. (I-d): $R^a$ = Ph, $R^b$ = c-Hexyl, $R^c$ = $R^d$ = $CH_3$ (first eluting enantiomer)**

The title compound was isolated following preparative chiral HPLC of Example 64. (Chiralpax IA, 250 x 20mm i.d.; 5% ethanol / 95% heptane / 0.1% diethylamine; 15 mL/min; Rt 12 mins).
LC-MS (Method 1): Rt 6.74 min, m/z 329 [$MH^+$]
$^1H$ NMR ($CDCl_3$) δ 1.10-1.37 (7H, m), 1.61-1.76 (3H, m), 2.26 (6H, s), 2.57 (2H, t), 2.81 (2 H, t), 3.66 (1H, s), 6.69 (1H, S), 7.24 (1H, m), 7.34 (2H, t), 7.63 (2H, d).

**Example 67**

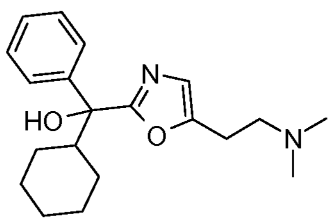

**[5-(2-Amino-ethyl)-oxazol-2-yl]-cyclohexyl-phenyl-methanol. (I-d): $R^a$ = Ph, $R^b$ = c-Hexyl, $R^c$ = $R^d$ = $CH_3$ (second eluting enantiomer)**

The title compound was isolated following preparative chiral HPLC of Example 64 using the conditions reported for Example 66; (Rt 13.5 mins).
LC-MS (Method 1): Rt 6.76 min, m/z 329 [$MH^+$]
NMR corresponds to Example 66.

**Example 68**

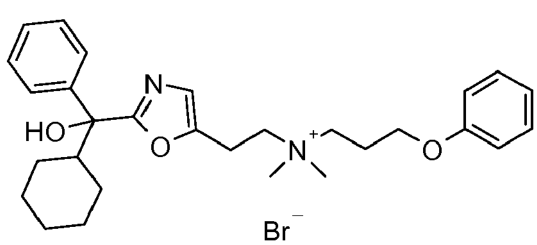

**{2-[2-(Cyclohexyl-hydroxy-phenyl-methyl)-oxazol-5-yl]-ethyl}-dimethyl-(3-phenoxy-propyl)-ammonium bromide (I-e): $R^a$ = Ph, $R^b$ = c-Hexyl, $R^c$ = $R^d$ = $CH_3$, $R^e$ = 3-phenoxyaropyl - Enantiomer 1**

The title compound was prepared from the compound in Example 66 using the method in Example 65.

LC-MS (Method 1): Rt 8.54 min, m/z 463 [$M^+$]
$^1$H NMR (DMSO-$d_6$) δ 0.95-1.28 (6H, m), 1.48-1.70 (4H, m), 2.12-2.28 (3H, m), 3.11 (6H, s), 3.20 (2H, t), 3.51 (2H, m), 3.61 (2H, m), 4.03 (2H, t), 5.89 (1H, s), 6.92-6.98 (3H, m), 7.00 (1H, s), 7.20-7.24 (1H, m), 7.28-7.34 (4H, m), 7.43-7.47 (2H, m).

**Example 69**

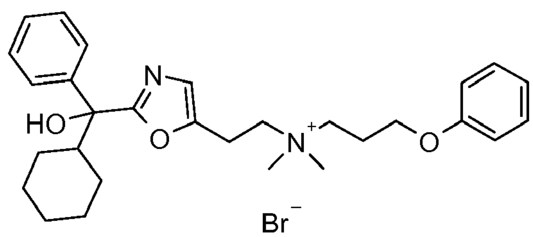

**{2-[2-(Cyclohexyl-hydroxy-phenyl-methyl)-oxazol-5-yl]-ethyl}-dimethyl-(3-phenoxy-propyl)-ammonium bromide (I-e): $R^a$ = Ph, $R^b$ = c-Hexyl, $R^c$ = $R^d$ = $CH_3$, $R^e$ = 3-phenoxyaroayl - Enantiomer 2**

The title compound was prepared from the compound in Example 67 using the method in Example 65.
LC-MS (Method 1): Rt 8.62 min, m/z 463 [$M^+$]
NMR corresponds to Example 68.

**Example 70**

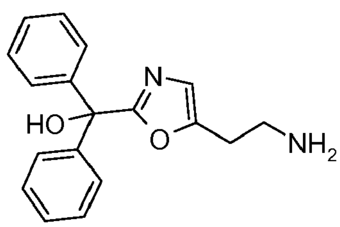

**[5-(2-Amino-ethyl)-oxazol-2-yl]-diphenyl-methanol. (I-c): $R^a$ = $R^b$ = Ph**

To a solution of [2-(hydroxy-diphenyl-methyl)oxazol-5-yl]-acetonitrile (Intermediate 13) (0.36 g, 1.2 mmol) in IMS (7 mL) was added Raney nickel (catalytic amount) and the suspension was stirred under an atmospere of hydrogen gas (balloon) at RT overnight. The reaction mixture was filtered over Celite, washed with IMS and the filtrate concentrated *in vacuo* to afford a brown oil, which was used without further purification.
LC-MS (Method 2): Rt 1.85 min, m/z 295 [$M^+$]
$^1$H NMR ($CDCl_3$) δ 2.66-2.73 (m, 2H), 2.81-2.86 (m, 2H), 6.64 (s, 1H), 7.25-7.37 (m, 10 H).

The following example compounds were prepared in a similar manner, using the route shown in Scheme 7.

| Example | Name | Structure | $^1$H NMR | LCMS |
|---|---|---|---|---|
| 71 | [5-(2-Dimethylamino-ethyl)-oxazol-2-yl]- diphenyl-methanol. (I-d): $R^a$ = $R^b$ = Ph, $R^c$ = $R^d$ = $CH_3$ | 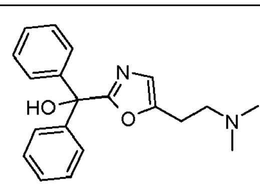 | ($CDCl_3$): δ2.23 (s, 6H), 2.55 (t, 2H), 2.80 (t, 2H), 4.50 (br.s, 1H), 6.76 (s, 1H), 7.27-7.37 (m, 10H). | (Metho d 5): Rt 5.03 min, m/z 323 [$MH^+$] |

(continued)

| Example | Name | Structure | $^1$H NMR | LCMS |
|---|---|---|---|---|
| 72 | {2-[2-(Hydroxy-diphenyl-methyl)-oxazol-5-yl]-ethyl}-dimethyl-(3-phenoxy-propyl)-ammonium bromide. (I-e): $R^a = R^b = Ph$, $R^c = R^d = CH_3$, $R^e$ = 3-Phenoxypropyl | 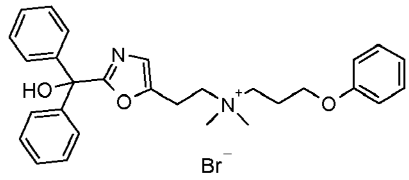 | (DMSO-$d_6$): δ 2.17 (m, 2H), 3.12 (s, 6H), 3.24 (t, 2H), 3.52 (m, 2H), 3.64 (m, 2H), 4.04 (t, 2H), 6.95 (m, 3H), 6.99 (s, 1H), 7.04 (s, 1H), 7.24-7.34 (m, 12H). | (Metho d 1): Rt 7.43 min, m/z 457 [$M^+$] |

The following example compounds were prepared using the route shown in Scheme 8.

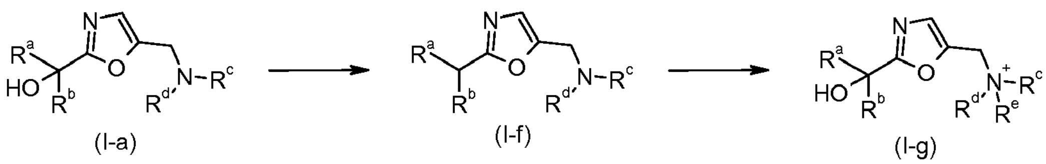

(I-a) (I-f) (I-g)

**Scheme 8**

**Example 73**

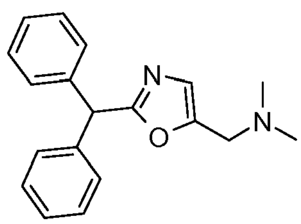

**(2-Benzhydryl-oxazol-5-ylmethyl)-dimethyl-amine. (I-f): $R^a = R^b = Ph$, $R^c$, $R^d = CH_3$**

Triethylsilane (720 μL, 4.51 mmol) was added to a solution of (5-dimethylaminomethyl-oxazol-2-yl)-diphenyl-methanol (Example 1) (100 mg, 0.325 mmol) in DCM (0.7 mL), followed by TFA (0.7 mL), and heated at reflux for 6 h. The reaction mixture was concentrated, passed over a SCX-2 cartridge and liberated with 2M ammonia solution in methanol. After evaporation of the volatiles the residue was purified over silica using a gradient of 1-5% MeOH/DCM as eluent to afford the title compound as a colourless oil

Yield: 74 mg (78%)

LC-MS (Method 5): Rt 5.03 min, m/z 293 [$MH^+$]

$^1$H NMR ($CDCl_3$): δ 2.24 (s, 6H), 3.50 (s, 2H), 5.59 (s, 1H), 6.91 (s, 1H), 7.22-7.33 (m, 10H).

**Example 74**

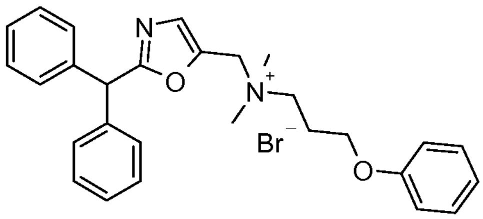

**(2-Benzhydryl-oxazol-5-ylmethyl)-dimethyl-(3-phenoxy-propyl)-ammonium bromide. (I-g): $R^a$ = $R^b$ = Ph, $R^c$, $R^d$ = Me, $R^e$ = 3-Phenoxylpropyl.**

The title compound was prepared from Example 73 according to the method used to prepare Example 8.
Yield: 87%
LC-MS (Method 1): Rt 8.05 min, m/z 427 [$M^+$]
$^1H$ NMR ($CDCl_3$): δ 2.32 (m, 2H), 3.39 (s, 6H), 3.59 (m, 2H), 3.93 (m, 2H), 5.30 (s, 2H), 5.62 (s, 1H), 6.79 (d, 2H), 6.97 (t, 1H), 7.20-7.32 (m, 12H), 7.61 (s, 1H).

**Example 75**

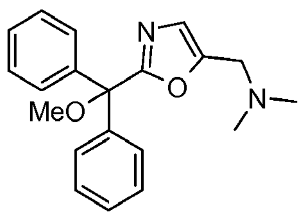

**[2-(Methoxy-diphenyl-methyl)-oxazol-5-ylmethyl]-dimethyl-amine. (I-h): $R^a$ = $R^b$ = Ph, $R^c$ = $R^d$ = $R^f$ = Me**

To a solution of (5-dimethylaminomethyl-oxazol-2-yl)-diphenyl-methanol (Example 1) (100mg, 0.32mmol) in 3mL DMF under nitrogen was added sodium hydride (60% dispersion in oil, 16mg, 0.40mmol) and the reaction mixture was stirred for 5 mins. Iodomethane (40μL, 0.65mmol) was added and the reaction mixture was stirred at room temperature for 18h. Water and EtOAc were added and the phases separated. The organic layer was dried ($Na_2SO_4$) and the solvent evaporated. The crude product was purified using a 10g Isolute $NH_2$ cartridge eluting with 5-10% EtOAc/cyclohexane to give the title compound.
Yield: 56mg (53%)
LCMS (Method 4): Rt 2.22min, m/z 323 [$MH^+$]
$^1H$ NMR ($CDCl_3$): δ 2.21 (s, 6H), 3.27 (s, 3H), 3.55 (s, 2H), 6.99 (s, 1H), 7.23-7.37 (m, 6H), 7.43-7.52 (m, 4H).

**Example 76**

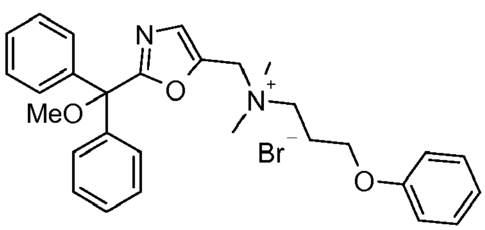

**[2-(Methoxy-diphenyl-methyl)-oxazol-5-ylmethyl]-dimethyl-(3-phenoxy-propyl)-ammonium bromide. (I-i): $R^a$ = $R^b$ = Ph, $R^c$, $R^d$ = Me, $R^e$ = 3-Phenoxyaropyl, $R^f$ = Me.**

The title compound was prepared from Example 75 according to the method used to prepare Example 8.
Yield: 40mg (45%)
LCMS (Method 5): Rt 7.46min, m/z 457 [$M^+$]
$^1$H NMR (MeOD): δ 2.27 (m, 2H), 3.09 (s, 6H), 3.23 (s, 3H), 3.42 (m, 2H), 3.98 (t, 2H), 4.78 (s, 2H), 6.88 (dd, 2H), 6.96 (m, 1H), 7.25-7.34 (m, 8H), 7.47 (m, 4H), 7.62 (s,1H).

**Example 77**

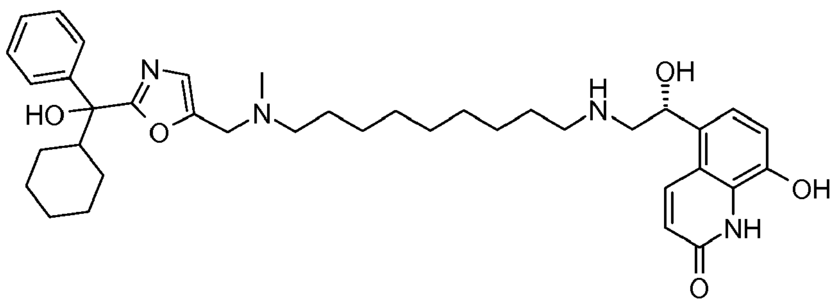

**5-[(R)-2-(9-{[2-(Cyclohexyl-hydroxy-phenyl-methyl)-oxazol-5-ylmethyl]-methylamino}-nonylamino)-1-hydroxy-ethyl]-8-hydroxy-1*H*-quinolin-2-one.**

A solution of 5-[(R)-1-(*tert*-butyl-dimethyl-silanyloxy)-2-(9-{[2-(cyclohexyl-hydroxyphenyl-methyl)-oxazol-5-ylmethyl]-methyl-amino}-nonylamino)-ethyl]-8-hydroxy-1*H*-quinolin-2-one (75 mg, 0.10 mmol) in dry THF (1 mL) under a nitrogen atmosphere was treated with trietylamine trihydrofluoride (48 μL, 0.30 mmol). After stirring at RT overnight the reaction mixture was neutralised with satd $NaHCO_3$ (aq) and extracted with DCM. The combined organic phases were washed with brine, dried ($Na_2SO_4$), filtered, and concentrated to dryness to afford a green/brown gum. This was purified by preparative HPLC (system1, 25% B + 1.7% B/min). The product fractions were concentrated, the residue taken up in MeOH/DCM, passed over a SCX-2 cartridge, and liberated using 2 M ammonia solution in MeOH. Evaporation of the solvent in *vacuo* afforded the title compound as a yellow gum.
Yield : 21 mg, 33%.
LC-MS (method :) : Rt 2.22 min, m/z 645 [MH+].

**Example 78**

**(5-Dimethylaminomethylthiazol-2-yl)diphenyl methanol. (XXVIII): $R^a$ = Ph, $R^b$ = Ph, $R^c$ = Me, $R^d$ = Me**

DCE (3ml) was added to a mixture of AIBN (5.84 mg, 0.036 mmol), N-bromosuccinimide (0.070 g, 0.391 mmol) and Intermediate 30 (0.1 g, 0.355 mmol). The suspension was placed in a preheated oil bath at 90 ˚C. After 40mins the reaction was judged to be complete according to LC-MS analysis. The solution was poured onto a mixture of diethyl ether and satd. sodium hydrogen carbonate and the layers separated. The organic phase was washed with water and brine, dried ($MgSO_4$) and evaporated. The residue was dissolved in THF (2ml), cooled to -10 ˚C under an atmosphere of nitrogen and treated with a 2M solution of dimethylamine in THF (0.178 ml, 0.355 mmol). After warming to RT, the solvent was evaporated, and the residue subjected to column chromatography ($SiO_2$, 8g) eluting with 50% EtOAc in iso-hexane to give the desired material.
Yield = 0.06 g (51 %)
LC-MS (Method 6): Rt 2.84 min, m/z 325 [MH]+.
1H NMR, 400 MHz, DMSO-$d_6$: 7.5 (1H, s), 7.4 (4H, m), 7.3-7.2 (7H, m), 3.6 (2H, s) and 2.1 (6H, s).

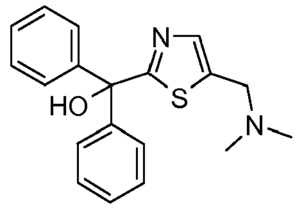

## Example 79

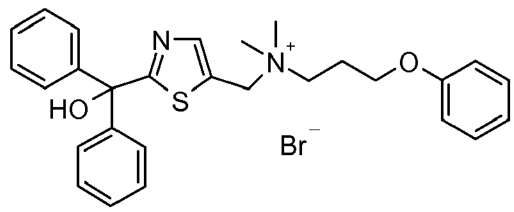

**[2-(Hydroxydiphenylmethyl)thiazol-5-ylmethyl]dimethyl-(3-phenoxyaropyl)ammonium bromide. (XXIX): $R^a$ = Ph, $R^b$ = Ph, $R^c$ = Me, $R^d$ = Me, $R^e$ = 3-phenoxy-1-propyl**

Phenoxypropylbromide (0.438 g, 2.034 mmol) was added to a solution of intermediate 3 (0.055 g, 0.170 mmol) in $CHCl_3$ (1 ml) and MeCN (1ml). The solution was heated at 55 ˚C for 20h, whereupon TLC (10% MeOH in DCM) indicated near completion of the reaction. The solvents were evaporated and the residue subjected to column chromatography ($SiO_2$, 8g) eluting with 5 to 15% MeOH in DCM to give the desired material as a white sold.
Yield = 0.061 g (66.7%)
LC-MS (Method 1): Rt 2.39 min, m/z 459 [M-Br]+.
1H NMR, 400 MHz, DMSO-d6: 8.0 (1H, s), 7.5 (1H, s), 7.4 (4H, m), 7.3-7.2 (8H, m), 6.9 (3H, m), 4.9 (2H, s), 4.0 (2H, s), 3.4 (2H, m), 6.1 (6H, s) and 2.2 (2H, m).

## Example 80

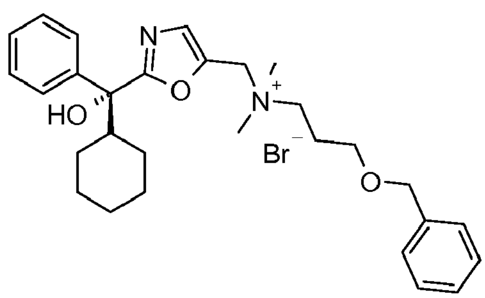

**(3-Benzyloxy-propyl)-[2-((R)-cyclohexyl-hydroxy-phenyl-methyl)-oxazol-5-ylmethyl]-dimethyl-ammonium bromide. (I-b): $R^a$ =Ph, $R^b$ = c-Hexyl, $R^c$, $R^d$ = $CH_3$, $R^e$ = 3-Benzyloxy-propyl**

The title compound was prepared according to the method used to prepare Example 8.
LC-MS (Method 5): Rt 7.26 min, m/z 463 [$M^+$]
$^1$H NMR (DMSO-$d_6$): δ 0.90-1.29 (m, 6H), 1.50-1.74 (m, 4H), 2.04 (m, 2H), 2.26 (m, 1H), 2.98 (s, 3H), 2.99 (s, 3H), 3.27 (m, 2H), 3.45 (t, 2H), 4.46 (s, 2H), 4.72 (s, 2H), 6.08 (s, 1H), 7.24 (t, 1H), 7.28-7.39 (m, 7H), 7.46 (d, 2H), 7.52 (s, 1H).

**Example 81**

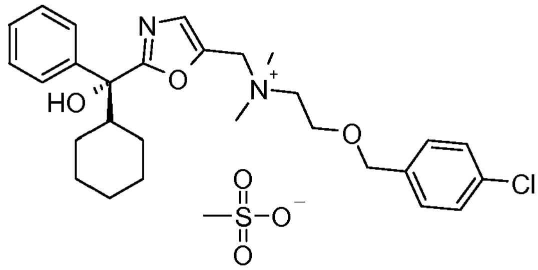

**[2-(4-Chloro-benzyloxy)-ethyl]-[2-((R)-cyclohexyl-hydroxy-phenyl-methyl)-oxazol-5-ylmethyl]-dimethyl-ammonium methanesulfonate. (I-b): $R^a$ =Ph, $R^b$ = c- Hexyl, $R^c$, $R^d$ = $CH_3$, $R^e$ = 2-(4-Chloro-benzyloxy)-ethyl**

The title compound was prepared according to the method used to prepare Example 8.
LC-MS (Method 1): Rt 8.73 min, m/z 483 [M+].
$^1H$ NMR ($CDCl_3$): δ 1.02-1.49 (7H, m), 1.56-1.79 (3H, m), 2.21-2.34 (1H, m), 2.67 (3H, s), 3.14 (6H, s), 3.64 (2H, b s), 3.88 (2H, b s), 4.49 (2H, s), 4.79-4.99 (3H, m), 7.16-7.36 (7H, m), 7.43 (1H, s), 7.51-7.58 (2H, m).

**Example 82**

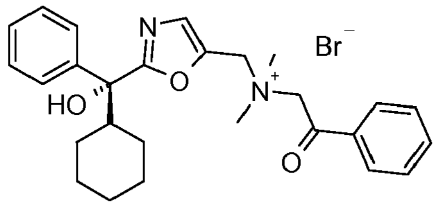

**[2-((R)-Cyclohexyl-hydroxy-phenyl-methyl)-oxazol-5-ylmethyl]-dimethyl-(2-oxo-2-phenyl-ethyl)-ammonium bromide. (I-b): $R^a$ =Ph, $R^b$ = c-Hexyl, $R^c$, $R^d$ = $CH_3$, $R^e$ = 2-Oxo-2-phenyl-ethyl**

The title compound was prepared according to the method used to prepare Example 8.
LC-MS (Method 2): Rt 2.53 and 2.60 min, m/z 433 [M+].
$^1H$ NMR ($CDCl_3$): δ 1.02-1.87 (10H, m), 2.17-2.32 (1H, m), 3.59 (3H, s), 3.60 (3H, s), 3.89 (1H, b s), 5.28 (1H, d), 5.47 (1H, d), 5.76 (2H, s), 7.17-7.33 (3H, m), 7.42 (1H, s), 7.45-7.54 (4H, m), 7.61-7.67 (1H, m), 8.05-8.11 (2H, m).

**Example 83**

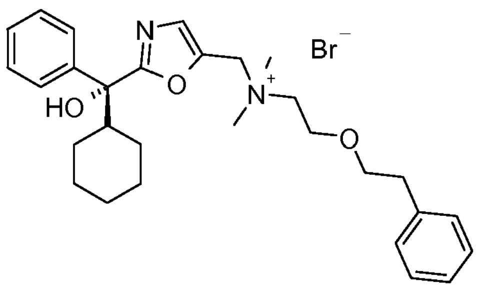

**[2-((R)-Cyclohexyl-hydroxy-phenyl-methyl)-oxazol-5-ylmethyl]-dimethyl-(2-phenethyloxy-ethyl)-ammonium bromide. (I-b): $R^a$ =Ph, $R^b$ = c-Hexyl, $R^c$, $R^d$ = $CH_3$, $R^e$ = 2-Phenethyloxy-ethyl**

The title compound was prepared according to the method used to prepare Example 8.
LC-MS (Method 1): Rt 8.51 min, m/z 463 [M+].
$^1$H NMR ($CDCl_3$): δ 1.07-1.40 (m, 7H), 1.60-1.80 (m, 3H), 2.32 (m, 1H), 2.87 (t, 2H), 3.12 (s, 6H), 3.72-3.87 (m, 6H), 4.17 (s, 1H), 4.91 (dd, 2H), 7.16 (m, 3H), 7.20-7.28 (m, 3H), 7.29-7.37 (m, 3H), 7.57 (d, 2H).

**Example 84**

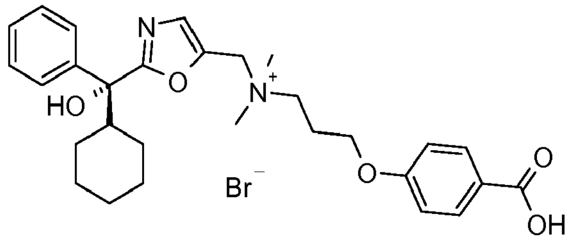

**[3-(4-Carboxy-phenoxy)-propyl]-[2-((R)-cyclohexyl-hydroxy-phenyl-methyl)-oxazol-5-ylmethyl]-dimethyl-ammonium bromide. (I-b): $R^a$ =Ph, $R^b$ = c-Hexyl, $R^c$, $R^d$ = $CH_3$, $R^e$ = 3-(4-Carboxy-phenoxy)-propyl**

1M NaOH (0.209mL) was added to a solution of [2-((R)-Cyclohexyl-hydroxy-phenylmethyl)-oxazol-5-ylmethyl]-[3-(4-methoxycarbonyl-phenoxy)-propyl]-dimethyl-ammonium bromide (Example 53) (41 mg, 0.069mmol) in $H_2O$ (3mL) and MeOH (3mL). The solution was stirred at ambient temperature for 2 days, and then 1M HCl (2mL) was added. The solution was freeze-dried and the crude product was purified via column chromatography eluting with 20% MeOH in DCM to give the title compound which was assumed to be the bromide salt.
Yield: 40mg (100%)
LC-MS (Method 1): Rt 7.35 min, m/z 493 [M+].
$^1$H NMR ($CD_3OD$): δ 1.02-1.39 (6H, m), 1.53-1.79 (4H, m), 2.22-1.46 (3H, m), 3.12 (6H, s), 3.39-3.52 (2H, m), 4.05-4.16 (2H, m), 4.77 (2H, s), 6.93-7.01 (2H, m), 7.16-7.35 (3H, m), 7.48-7.56 (4H, m), 7.95-8.02 (2H, m).

**Example 85**

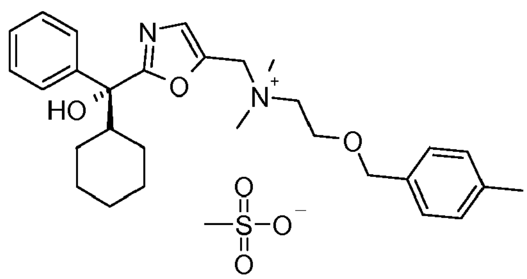

**[2-((R)-cyclohexyl-hydroxy-phenyl-methyl)-oxazol-5-ylmethyl]-dimethyl-[2-(4-methyl-benzyloxy)-ethyl]-ammonium methanesulfonate. (I-b): $R^a$ =Ph, $R^b$ = c-Hexyl, $R^c$, $R^d$ = $CH_3$, $R^e$ = 2-(4-Methyl-benzyloxy)-ethyl**

The title compound was prepared according to the method used to prepare Example 8.
LC-MS (Method 1): Rt 8.67 min, m/z 463 [M+].
$^1$H NMR ($CDCl_3$): δ 1.03-2.07 (10H, m), 2.22-2.43 (4H, m), 2.69 (3H, s), 3.14 (6H, s), 3.64 (2H, b s), 3.86 (2H, b s), 4.48 (2H, s), 4.60 (1H, b s), 4.82-5.02 (2H, m), 7.04-7.37 (7H, m), 7.42 (1H, s), 7.55 (2H, d).

**Example 86**

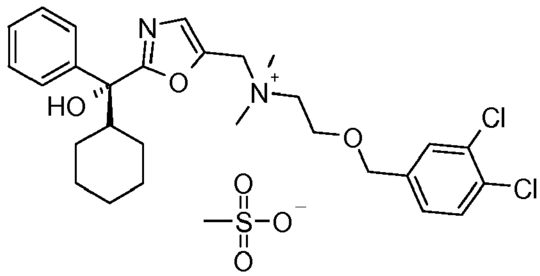

**[2-((R)-cyclohexyl-hydroxy-phenyl-methyl)-oxazol-5-ylmethyl]-[2-(3,4-dichlorobenzyloxy)-ethyl]-dimethyl-ammonium methanesulfonate. (I-b): $R^a$ =Ph, $R^b$ = c-Hexyl, $R^c$, $R^d$ = $CH_3$, $R^e$ = 2-(3,4-Dichloro-benzyloxy)-ethyl**

The title compound was prepared according to the method used to prepare Example 8.
LC-MS (Method 1): Rt 9.09 min, m/z 517 [M+].
$^1$H NMR ($CDCl_3$): δ 1.03-1.39 (7H, m), 1.58-1.80 (3H, m), 2.23-2.35 (1H, m), 2.71 (3H, s), 3.17 (6H, s), 3.71-3.77 (2H, m), 3.89-3.98 (2H, m), 4.42 (1H, s), 4.49 (2H, s), 4.87-5.04 (2H, m), 7.11-7.33 (4H, m), 7.36-7.49 (3H, m), 7.53-7.59 (2H, m).

**Example 87**

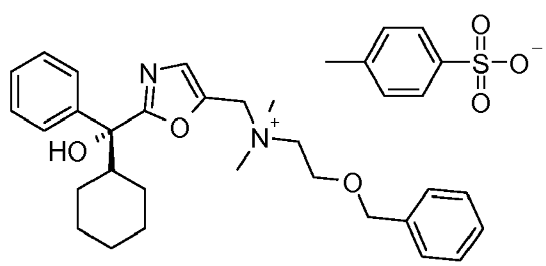

**(2-benzyloxy-ethyl)-[2-((R)-cyclohexyl-hydroxy-phenyl-methyl)-oxazol-5-ylmethyl]-dimethyl-ammonium toluene-4-sulfonate. (I-b): $R^a$ =Ph, $R^b$ = c-Hexyl, $R^c$, $R^d$ = $CH_3$, $R^e$ = 2-Benzyloxy-ethyl**

The title compound was prepared according to the method used to prepare Example 8 using 2-(benzyloxyethyl)-*p*-toluenesulfonate.
LC-MS (Method 2): Rt 2.60 m/z 449 [M+].
$^1$H NMR ($CDCl_3$): δ 1.02-1.76 (10H, m), 2.22-2.37 (4H, m), 3.19 (6H, s), 3.69 (2H, b s), 3.86 (2H, b s), 4.25 (1H, b s), 4.48 (2H, s), 4.89-5.07 (2H, m), 7.05-7.15 (2H, m), 7.16-7.43 (9H, m), 7.55 (2H, d), 7.73 (2H, d).

**Example 88**

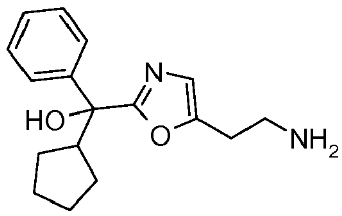

**[5-(2-Amino-ethyl)-oxazol-2-yl]-cycloaentyl-phenyl-methanol. (I-c): R$^a$ = Ph, R$^b$ = c-Pentyl.**

Prepared according to the method used to prepare Example 70.
LC-MS (Method 2): Rt 2.03 min, m/z 287 [MH$^+$]
$^1$H NMR ($CDCl_3$) δ 1.28-1.71 (m, 11H), 2.78 (t, 2H), 2.97 (m, 3H), 6.72 (s, 1H), 7.22-7.28 (m, 1H), 7.30-7.36 (m, 2H), 7.59-7.64 (m, 2H).

**Example 89**

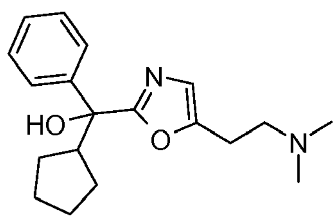

**Cyclopentyl-[5-(2-dimethylamino-ethyl)-oxazol-2-yl]-phenyl-methanol. (I-c): R$^a$ = Ph, R$^b$ = c-Pentyl, R$^c$ = R$^d$ = $CH_3$.**

Prepared according to the method used to prepare Example 64.
LC-MS (Method 2): Rt 2.05 min, m/z 315 [MH$^+$]
$^1$H NMR ($CDCl_3$) δ 1.29-1.70 (m, 8H), 2.26 (s, 6H), 2.57 (m, 2H), 2.80 (t, 2H), 2.97 (m, 1H), 3.68 (br s, 1H), 6.69 (s, 1H), 7.21-7.27 (m, 1H), 7.33 (t, 2H), 7.62 (d, 2H).

**Example 90**

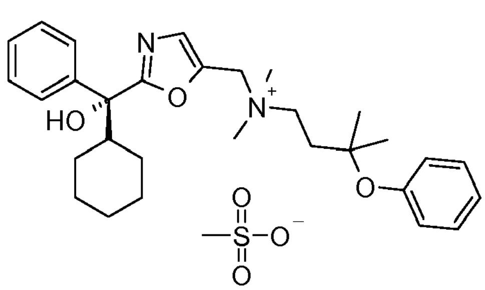

**[2-((R)-cyclohexyl-hydroxy-phenyl-methyl)-oxazol-5-ylmethyl]-dimethyl-(3-methyl-3-phenoxy-butyl)-ammonium methanesulfonate. (I-b): R$^a$** =Ph, **R$^b$ = c-Hexyl, R$^c$, R$^d$** = **$CH_3$, R$^e$ = 3-Methyl-3-phenoxy-butyl**

The title compound was prepared according to the method used to prepare Example 8.
Yield: 5mg (6%)

LC-MS (Method 4): Rt. 2.85 min, m/z 477 [M+].
$^1$H NMR ($CDCl_3$): δ 0.70-2.00 (16H, m), 2.02-2.13 (2H, m), 2.16-2.30 (1H, m), 2.62 (3H, s), 3.07 (6H, s), 3.44-3.55 (2H, m), 4.77-4.91 (2H, m), 6.83 (2H, d), 6.97-7.27 (5H, m), 7.43-7.52 (4H, m).

## BIOLOGICAL EXAMPLES

The inhibitory effects of compounds of the present invention at the M3 muscarinic receptor and (in the case of Example 77) the $\beta_2$ adrenergic receptor, were determined by the following binding assays:

## Muscarinic Receptor Radioligand Binding Assays

Radioligand binding studies utilising [$^3$H]-N-methyl scopolamine ([$^3$H]-NMS) and commercially available cell membranes expressing the human muscarinic receptors (M2 and M3) were used to assess the affinity of muscarinic antagonists for M2 and M3 receptors. Membranes in TRIS buffer were incubated in 96-well plates with [$^3$H]-NMS and M3 antagonist at various concentrations for 3 hours. Membranes and bound radioligand were then harvested by filtration and allowed to dry overnight. Scintillation fluid was then added and the bound radioligand counted using a Canberra Packard Topcount scintillation counter

The half-life of antagonists at each muscarinic receptor was measured using the alternative radioligand [$^3$H]-QNB and an adaptation of the above affinity assay. Antagonists were incubated for 3 hours at a concentration 10-fold higher than their Ki, as determined with the [$^3$H]-QNB ligand, with membranes expressing the human muscarinic receptors. At the end of this time, [$^3$H]-QNB was added to a concentration 25-fold higher than its Kd for the receptor being studied and the incubation continued for various time periods from 15 minutes up to 180 minutes. Membranes and bound radioligand were then harvested by filtration and allowed to dry overnight. Scintillation fluid was then added and the bound radioligand counted using a Canberra Packard Topcount scintillation counter.

The rate at which [3H]-QNB is detected binding to the muscarinic receptors is related to the rate at which the antagonist dissociates from the receptor, ie. to the half life of the antagonists on the receptors.

The following compounds were tested in the receptor binding assay:

| Example | M3 binding Ki |
|---|---|
| 1 | ++ |
| 2 | +++ |
| 3 | NT |
| 4 | +++ |
| 5 | + |
| 6 | +++ |
| 7 | ++ |
| 8 | +++ |
| 9 | +++ |
| 10 | NT |
| 11 | +++ |
| 12 | +++ |
| 13 | ++ |
| 14 | + |
| 15 | ++ |
| 16 | NT |
| 17 | NT |
| 18 | NT |
| 19 | NT |

(continued)

| Example | M3 binding Ki |
|---|---|
| 20 | NT |
| 21 | NT |
| 22 | NT |
| 23 | NT |
| 24 | NT |
| 25 | NT |
| 26 | + |
| 27 | NT |
| 28 | + |
| 29 | + |
| 30 | + |
| 31 | + |
| 32 | +++ |
| 33 | +++ |
| 34 | +++ |
| 35 | ++ |
| 36 | +++ |
| 37 | +++ |
| 38 | ++ |
| 39 | + |
| 40 | +++ |
| 41 | + |
| 42 | ++ |
| 43 | +++ |
| 44 | +++ |
| 45 | +++ |
| 46 | +++ |
| 47 | ++ |
| 48 | +++ |
| 49 | +++ |
| 50 | + |
| 51 | ++ |
| 52 | ++ |
| 53 | ++ |
| 54 | ++ |
| 55 | +++ |
| 56 | +++ |
| 57 | +++ |

(continued)

| Example | M3 binding Ki |
|---|---|
| 58 | ++ |
| 59 | ++ |
| 60 | + |
| 61 | +++ |
| 62 | +++ |
| 63 | NT |
| 64 | NT |
| 65 | +++ |
| 66 | ++ |
| 67 | +++ |
| 68 | ++ |
| 69 | +++ |
| 70 | NT |
| 71 | ++ |
| 72 | +++ |
| 73 | + |
| 74 | + |
| 75 | NT |
| 76 | + |
| 77 | + |
| 78 | NT |
| 79 | +++ |
| 80 | +++ |
| 81 | +++ |
| 82 | NT |
| 83 | NT |
| 84 | NT |
| 85 | NT |
| 86 | NT |
| 87 | NT |
| 88 | NT |
| 89 | NT |
| 90 | NT |

Compounds with a M3 binding Ki < 1 nM are indicated '+++'; those with Ki between 1 and 10nM as '++' and those greater than 10nM as '+'. All compounds tested had Ki values <5μM. Compounds marked 'NT' were not tested in this assay.

## β- Adrenergic Receptor Radioligand Binding Assays

Radioligand binding studies utilising [$^{125}$I]-Iodocyanopindolol and commercially available cell membranes expressing the human $\beta_2$ adrenergic receptor were used to assess the affinity of antagonists for $\beta_2$-adrenergic receptor. Membranes and SPA-beads were incubated with [$^{125}$I]-Iodocyanopindolol and $\beta_2$ antagonist at various concentrations for 3 hours at room temperature in TRIS buffer. The assay was performed in 96-well plates which were read using the Wallac Microbeta counter. Example 77 exhibited a $K_i$ value of <100nM in this assay.

## Analysis of Inhibition of M3 Receptor Activation via Calcium Mobilization

CHO cells expressing the human M3 receptor were seeded and incubated overnight in 96 well collagen coated plates (black-wall, clear bottom) at a density of 50000 / 75μl of medium in 3% serum. The following day, a calcium-sensitive dye (Molecular Devices, Cat # R8041) was prepared in HBSS buffer with the addition of 5mM probenecid (pH 7.4). An equal volume of the dye solution (75μl) was added to the cells and incubated for 45 minutes followed by addition of 50μl of muscarinic antagonists or vehicle. After a further 15 minutes the plate was read on a FLEXstation™ (excitation 488nm, emission 525nm) for 15 seconds to determine baseline fluorescence. The muscarinic agonist Carbachol was then added at an $EC_{80}$ concentration and the fluorescence measured for a further 60 seconds. The signal was calculated by subtracting the peak response from the mean of the baseline fluorescence in control wells in the absence of antagonist. The percentage of the maximum response in the presence of antagonist was then calculated in order to generate $IC_{50}$ curves.

## Evaluation of potency and duration of action in Isolated Guinea Pig Trachea

Experiments were carried out at 37˚C in modified Krebs-Henseleit solution, (114mM NaCl, 15mM $NaHCO_3$ 1mM $MgSO_4$, 1.3mM $CaCl_2$, 4.7mM KCl, 11.5mM glucose and 1.2mM $KH_2PO_4$, pH 7.4) gassed with 95% $O_2$/5% $CO_2$. Indomethacin was added to a final concentration of 3μM

Tracheae were removed from adult male Dunkin Hartley Guinea pigs and dissected free of adherent tissue before being cut open longitudinally in a line opposite the muscle. Individual strips of 2-3 cartilage rings in width were cut and suspended using cotton thread in 10ml water-jacketed organ baths and attached to a force transducer ensuring that the tissue is located between two platinum electrodes. Responses were recorded via a MP100W/Ackowledge data acquisition system connected to a PC. Tissues were equilibrated for one hour under a resting tone of 1g and were then subjected to electrical field stimulation at a frequency of 80Hz with a pulse width of 0.1 ms, a unipolar pulse, triggered every 2 minutes. A "voltage-response" curve was generated for each tissue and a submaximal voltage then applied to every piece of tissue according to its own response to voltage. Tissues were washed with Krebs solution and allowed to stabilize under stimulation prior to addition of test compound. Concentration response curves were obtained by a cumulative addition of test compound in half-log increments. Once the response to each addition had reached a plateau the next addition was made. Percentage inhibition of EFS-stimulated contraction is calculated for each concentration of each compound added and dose response curves constructed using Graphpad Prism software and the $EC_{50}$ calculated for each compound.

Onset time and duration of action studies were performed by adding the previously determined $EC_{50}$ concentration of compound to EFS contracted tissues and the response allowed to plateau. The time taken to reach 50% of this response was determined to be the onset time. Tissues were then washed free of compound by flushing the tissue bath with fresh Krebs solution and the time taken for the contraction in response to EFS to return to 50% of the response in the presence of compound is measured. This is termed the duration of action.

## Methacholine Induced Bronchoconstriction in vivo

Male Guinea pigs (Dunkin Hartley), weighing 500-600g housed in groups of 5 were individually identified. Animals were allowed to acclimatize to their local surroundings for at least 5 days. Throughout this time and study time animals were allowed access to water and food *ad libitum.*

Guinea pigs were anaesthetized with the inhaled anaesthetic Halothane (5%). Test compound or vehicle (0.25 - 0.50 ml/kg) was administered intranasally. Animals were placed on a heated pad and allowed to recover before being returned to their home cages.

Up to 72hrs post dosing guinea pigs were terminally anaesthetized with Urethane (250μg/ml, 2ml/kg). At the point of surgical anaesthesia, the jugular vein was cannulated with a portex i.v. cannula filled with heparinised phosphate buffered saline (hPBS) (10U/ml) for i.v. administration of methacholine. The trachea was exposed and cannulated with a rigid portex cannula and the oesophagus cannulated transorally with a flexible portex infant feeding tube.

The spontaneously breathing animal was then connected to a pulmonary measurement system (EMMS, Hants,

UK) consisting of a flow pneumotach and a pressure transducer. The tracheal cannula was attached to a pneumotach and the oesophageal cannula attached to a pressure transducer.

The oesophageal cannula was positioned to give a baseline resistance of between 0.1 and 0.2cmH20/ml/s. A 2 minute baseline reading was recorded before i.v. administration of methacholine (up to 30μg/kg, 0.5ml/kg). A 2 minute recording of the induced constriction was taken from the point of i.v. administration.

The software calculated a peak resistance and a resistance area under the curve (AUC) during each 2 minute recording period which were used to analyse the bronchoprotective effects of test compounds. The results obtained in this assay for the compound of Example 32 (0.1, 0.3 and 1 μg/kg i.n.) 4 hours prior to MCh (10μg/kg i.v.) induced bronchoconstriction, and the comparator compound tiotropium, are shown in Figure 1.

**Inhibition of pilocarpine induced salivation by i.n. administered compounds.**

Guinea pigs (450-550g) supplied by Harlan UK or David Hall, Staffs UK and acclimatised to the in-house facilities for a minimum of three days before use. Guinea pigs were randomly assigned into treatment groups and weighed. Each animal was lightly anaesthetised (4% Halothane) and administered compound or vehicle intranasally (0.5ml/kg) at up to 24 hours before challenge with pilocarpine. At the test time point, guinea pigs were terminally anaesthetised with urethane (25% solution in H20, 1.5g/kg). Once sufficient anaesthesia had developed (absence of toe pinch reflex) each animal had an absorbent pad placed in the mouth for 5 minutes to dry residual saliva, this pad was removed and replaced with a new pre-weighed pad for 5 minutes to establish a reading of baseline saliva production. At the end of this 5 minute period the pad was removed and weighed. A new pre-weighed pad was inserted into the mouth before each animal received s.c. pilocarpine administered under the skin at the back of the neck (0.6mg/kg @ 2ml/kg). The pad was removed, weighed and replaced with a new pre-weighed pad every 5 minutes up to 15 minutes. Saliva production was calculated by subtracting the pre-weighed weight of the pad from each 5 minute period post weighed pad and these numbers added together to produce an accumulation of saliva over 15 minutes. Each 5 minute period could be analysed in addition to the whole 15 minute recording period. Baseline production of saliva was assumed to be constant and multiplied by three to produce a reading for baseline saliva production over 15 minutes.

Inhibition of saliva produced by the compound could be calculated by using the following equation:

$$(1-(\text{Test-baseline})/(\text{Veh-baseline}))*100.$$

**Claims**

**1.** A pharmaceutical composition for inhalation comprising a compound of formula (I):

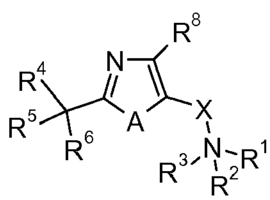

(I)

wherein

(i) $R^1$ is $C_1$-$C_6$-alkyl or hydrogen; and $R^2$ is hydrogen or a group $-R^7$, $-Z-Y-R^7$, $-Z-NR^9R^{10}$; $-Z-CO-NR^9R^{10}$, $-Z-NR^9-C(O)O-R^7$, or $-Z-C(O)-R^7$; and $R^3$ is a lone pair, or $C_1$-$C_6$-alkyl; **or**

(ii) **$R^1$ and $R^3$** together with the nitrogen to which they are attached form a heterocycloalkyl ring, and $R^2$ is a lone pair or a group $-R^7$, $-Z-Y-R^7$, $-Z-NR^9R^{10}$, $-Z-CO-NR^9R^{10}$, $-Z-NR^9-C(O)O-R^7$; or ; $-Z-C(O)-R^7$; **or**

(iii) **$R^1$ and $R^2$** together with the nitrogen to which they are attached form a heterocycloalkyl ring, said ring being substituted by a group $-Y-R^7$, $-Z-Y-R^7$, $-Z-NR^9R^{10}$; $-Z-CO-NR^9R^{10}$; $-Z-NR^9-C(O)O-R^7$; or; $-Z-C(O)-R^7$; and $R^3$ is a lone pair, or $C_1$-$C_6$-alkyl;

$R^4$ and $R^5$ are independently selected from the group consisting of aryl, aryl-fused-heterocycloalkyl, heteroaryl,

$C_1$-$C_6$-alkyl, cycloalkyl;
**$R^6$** is -OH, $C_1$-$C_6$-alkyl, $C_1$-$C_6$-alkoxy, hydroxy-$C_1$-$C_6$-alkyl, nitrile, a group $CONR^8{}_2$ or a hydrogen atom;
**A** is an oxygen or a sulfur atom;
**X** is an alkylene, alkenylene or alkynylene group;
**$R^7$** is an $C_1$-$C_6$-alkyl, aryl, aryl-fused-cycloalkyl, aryl-fused-heterocycloalkyl, heteroaryl, aryl($C_1$-$C_8$-alkyl)-, heteroaryl ($C_1$-$C_8$-alkyl)-, cycloalkyl or heterocycloalkyl group;
**$R^8$** is $C_1$-$C_6$-alkyl or a hydrogen atom;
**Z** is a $C_1$-$C_{16}$-alkylene, $C_2$-$C_{16}$-alkenylene or $C_2$-$C_{16}$-alkynylene group;
**Y** is a bond or oxygen atom;
**$R^9$** and **$R^{10}$** are independently a hydrogen atom, $C_1$-$C_6$-alkyl, aryl, aryl-fused-heterocycloalkyl, aryl-fused-cycloalkyl, heteroaryl, aryl($C_1$-$C_6$-alkyl)-, or heteroaryl($C_1$-$C_6$-alkyl)- group; or **$R^9$** and **$R^{10}$** together with the nitrogen atom to which they are attached form a heterocyclic ring of 4-8 atoms, optionally containing a further nitrogen or oxygen atom;
or a pharmaceutically acceptable salt, solvate, N-oxide or prodrug thereof.

**2.** A pharmaceutical composition as claimed in claim 1 wherein:

$R^1$ is $C_1$-$C_6$-alkyl or a hydrogen atom; $R^2$ is $C_1$-$C_6$-alkyl, a hydrogen atom or a group -Z-Y-$R^7$ and $R^3$ is a lone pair or $C_1$-$C_6$-alkyl, or
$R^1$ and $R^2$ together with the nitrogen to which they are attached represent a heterocycloalkyl ring, or $R^1$ and $R^3$ together with the nitrogen to which they are attached represent a heterocycloalkyl ring;
$R^4$ and $R^5$ are independently selected from the group consisting of aryl, heteroaryl, $C_1$-$C_6$-alkyl, cycloalkyl;
$R^6$ is -OH, halogen, $C_1$-$C_6$-alkyl, hydroxy-$C_1$-$C_6$-alkyl or a hydrogen atom;
A is an oxygen or a sulfur atom;
X is an alkylene, alkenylene or alkynylene group;
Z is an alkylene, alkenylene or alkynylene group;
Y is a bond or oxygen atom;
$R^7$ is aryl, heteroaryl, heterocycloalkyl.

**3.** A pharmaceutical composition as claimed in claim 1 or claim 2 wherein **$R^1$** is methyl or ethyl, or a hydrogen atom; **$R^2$** is hydrogen, $C_1$-$C_6$-alkyl, or a group -$R^7$, -Z-Y-$R^7$, -Z-$NR^9R^{10}$, -Z-CO-$NR^9R^{10}$, -Z-$NR^9$-C(O)O-$R^7$ $_{or}$; -Z-C(O)-$R^7$; and **$R^3$** is a lone pair, or $C_1$-$C_6$-alkyl in which case the nitrogen atom to which it is attached is a quaternary nitrogen and carries a positive charge.

**4.** A pharmaceutical composition as claimed in claim 3 wherein $R^3$ is methyl, so that the nitrogen atom to which it is attached is a quaternary nitrogen and carries a positive charge.

**5.** A pharmaceutical composition as claimed in claim 1 or claim 2 wherein **$R^1$ and $R^3$** together with the nitrogen to which they are attached form a monocyclic heterocycloalkyl ring of from 3 to 7 ring atoms, in which the hetero-atoms are nitrogen; and **$R^2$** is a lone pair or $C_1$-$C_6$-alkyl, or a group -$R^7$, -Z-Y-$R^7$, -Z-$NR^9R^{10}$, - Z-$NR^9$-C(O)O-$R^7$ or-Z-C (O)-$R^7$.

**6.** A pharmaceutical composition as claimed in claim 5 wherein **$R^1$ and $R^3$** together with the nitrogen to which they are attached form an azetidinyl, piperidinyl, piperazinyl, N-methylpiperazinyl, or pyrrolidinyl ring.

**7.** A pharmaceutical composition as claimed in claim 5 or claim 6 wherein the nitrogen atom to which $R^1$ and $R^3$, or $R^1$ and $R^2$, are attached is a quaternary nitrogen and carries a positive charge.

**8.** A pharmaceutical composition as claimed in any of the preceding claims wherein (i) each of $R^4$ and $R^5$ is thienyl; or (ii) each of $R^4$ and $R^5$ is phenyl; or (iii) one of $R^4$ and $R^5$ is phenyl and the other is cyclopentyl or cyclohexyl; or (iv) one of $R^4$ and $R^5$ is thienyl, and the other is cyclopentyl or cyclohexyl.

**9.** A pharmaceutical composition as claimed in claim 8 wherein $R^6$ is -OH.

**10.** A pharmaceutical composition as claimed in any of the preceding claims wherein $R^8$ is hydrogen.

**11.** A pharmaceutical composition as claimed in any of the preceding claims wherein X is $-CH_2-$ or $-CH_2CH_2-$.

**12.** A pharmaceutical composition as claimed in claim 1 which has formula (IA)

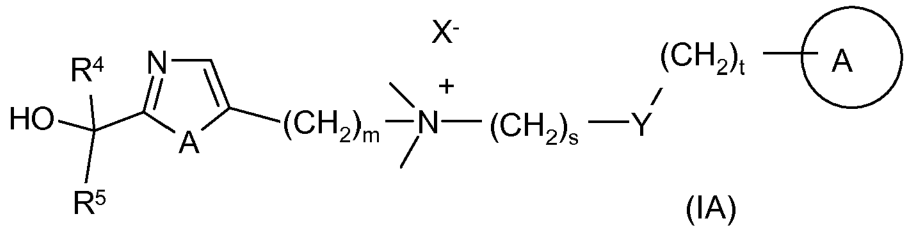

(IA)

wherein A is -O- or -S-; m is 1 or 2; ring A is an optionally substituted phenyl ring, or monocyclic heterocyclic ring of 5 or 6 ring atoms, or phenyl-fused-heterocycloalkyl ring system wherein the heterocycloalkyl ring is a monocyclic heterocyclic ring of 5 or 6 ring atoms; $R^4$ is phenyl, thienyl, cyclopentyl or cyclohexyl; $R^5$ is phenyl; thienyl, cyclopentyl or cyclohexyl; s is 1, 2, 3, 4, 5, 6 or 7 and t is 0, 1, 2, 3, 4, 5, 6 or 7 provided that s+t is not greater than 16; Y is a bond or -O-, and X- is a pharmaceutically acceptable anion.

**13.** A pharmaceutical composition as claimed in claim 1, selected from the group consisting of

[2-(Hydroxy-diphenyl-methyl)-oxazol-5-ylmethyl]-dimethyl-(3-phenoxy-propyl)-ammonium salts
[2-((R)-Cyclohexyl-hydroxy-phenyl-methyl)-oxazol-5-ylmethyl]-dimethyl-(3-phenoxypropyl)-ammonium salts
[2-((R)-Cyclohexyl-hydroxy-phenyl-methyl)-oxazol-5-ylmethyl]-dimethyl-phenethyl-ammonium salts
[2-((R)-Cyclohexyl- hydroxy- phenyl- methyl)-oxazol- 5- ylmethyl]-dimethyl-(4- methylpent- 3- enyl)-ammonium salts
[2-((R)-Cyclohexyl- hydroxy- phenyl- methyl)-oxazol- 5- ylmethyl]-[2-(2,3- dihydrobenzofuran- 5- yl)-ethyl]-dimethyl-ammonium salts
[2-((R)-cyclohexyl-hydroxy-phenyl-methyl)-oxazol-5-ylmethyl]-dimethyl-(6-methylpyridin-2-ylmethyl)-ammonium salts
[2-(Cyclopentyl-hydroxy-phenyl-methyl)-oxazol-5-ylmethyl]-dimethyl-(3-phenoxypropyl)-ammonium salts
[2-(Cyclopentyl-hydroxy-phenyl-methyl)-oxazol-5-ylmethyl]-dimethyl-(3-phenoxypropyl)-ammonium salts
1-[2-(Cyclohexyl-hydroxy-phenyl-methyl)-oxazol-5-ylmethyl]-1-(3-phenoxy-propyl)-pyrrolidinium salts
[2-((R)-Cyclohexyl-hydroxy-phenyl-methyl)-oxazol-5-ylmethyl]-dimethyl-(4-phenoxybutyl)-ammonium salts
(2-Benzyloxy-ethyl)-[2-((R)-cyclohexyl-hydroxy-phenyl-methyl)-oxazol-5-ylmethyl]-dimethyl-ammonium salts
[2-((R)-Cyclohexyl-hydroxy-phenyl-methyl)-oxazol-5-ylmethyl]-dimethyl-(4-phenylbutyl)-ammonium salts
[2-((R)-Cyclohexyl-hydroxy-phenyl-methyl)-oxazol-5-ylmethyl]-[3-(4-fluoro-phenoxy)-propyl]-dimethyl-ammonium salts
[2-((R)-cyclohexyl-hydroxy-phenyl-methyl)-oxazol-5-ylmethyl]-dimethyl-(3-phenylpropyl)-ammonium salts
[2-((R)-cyclohexyl-hydroxy-phenyl-methyl)-oxazol-5-ylmethyl]-dimethyl-(2-phenoxyethyl)-ammonium salts
[2-((R)-Cyclohexyl-hydroxy-phenyl-methyl)-oxazol-5-ylmethyl]-dimethyl-(3-p-tolyloxypropyl)-ammonium salts
[3-(4-Chloro-phenoxy)-propyl]-[2-((R)-cyclohexyl-hydroxy-phenyl-methyl)-oxazol-5-ylmethyl]-dimethyl-ammonium salts
[2-((R)-Cyclohexyl-hydroxy-phenyl-methyl)-oxazol-5-ylmethyl]-[3-(3,4-dichlorophenoxy)-propyl]-dimethyl-ammonium salts
[2-((R)-cyclohexyl- hydroxy- phenyl- methyl)-oxazol- 5- ylmethyl]-dimethyl-(8- methylamino- octyl)-ammonium salts
[2-((R)-cyclohexyl- hydroxy- phenyl- methyl)-oxazol- 5- ylmethyl]-dimethyl-[2-(4- methylaminomethyl- phenyl)-ethyl]-ammonium salts
{2-[2-(Cyclohexyl-hydroxy-phenyl-methyl)-oxazol-5-yl]-ethyl}-dimethyl-(3-phenoxypropyl)-ammonium salts
{2-[2-(Cyclohexyl-hydroxy-phenyl-methyl)-oxazol-5-yl]-ethyl}-dimethyl-(3-phenoxy-propyl)-ammonium salts, and
{2-[2-(Hydroxy-diphenyl-methyl)-oxazol-5-yl]-ethyl}-dimethyl-(3-phenoxy-propyl)-ammonium salts
[2-(Hydroxydiphenylmethyl)thiazol-5-ylmethyl]dimethyl-(3-phenoxypropyl)ammonium salts
(3-Benzyloxypropyl)-[2-((R)-cyclohexyl-hydroxy-phenyl-methyl)-oxazol-5-ylmethyl]-dimethyl-ammonium salts
[2-(4-Chloro-benzyloxy)-ethyl]-[2-((R)-cyclohexyl-hydroxy-phenyl-methyl)-oxazol-5-ylmethyl]-dimethyl-ammonium salts.

**14.** A pharmaceutical composition as claimed in claim 1 comprising a [2-(4-Chloro-benzyloxy)-ethyl]-[2-((R)-cyclohexyl-hydroxy-phenyl-methyl)-oxazol-5-ylmethyl]-dimethyl-ammonium salt.

Figure 1

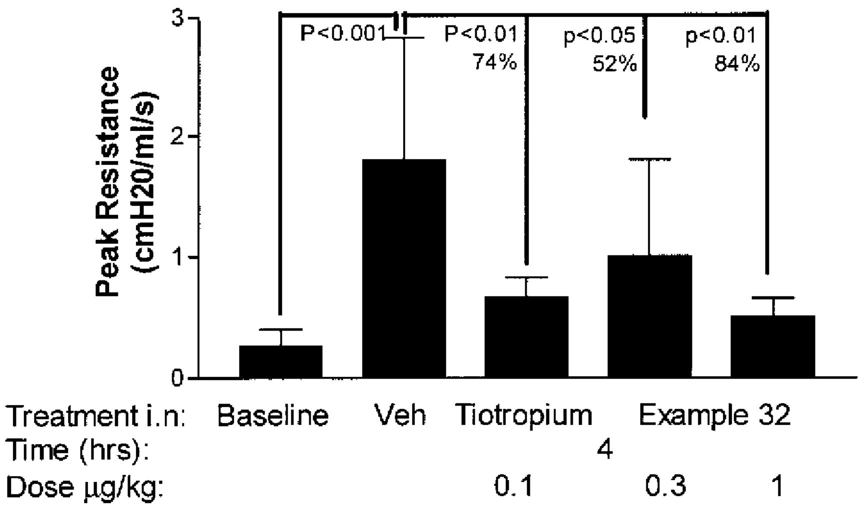
Peak Resistance (cmH20/ml/s)
3
2
1
0
P<0.001
P<0.01
74%
p<0.05
52%
p<0.01
84%
Treatment i.n: Baseline Veh Tiotropium Example 32
Time (hrs): 4
Dose µg/kg: 0.1 0.3 1

Europäisches Patentamt
European Patent Office
Office européen des brevets

# EUROPEAN SEARCH REPORT

Application Number
EP 10 17 0818

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 97/30994 A (PFIZER RES & DEV [IE]; PFIZER LTD [GB]; PFIZER INC[US]; MACKENZIE ALEX) 28 August 1997 (1997-08-28)<br>* the whole document * | 1-14 | INV.<br>C07D263/32<br>C07D277/30<br>A61K31/421<br>A61K31/426 |
| A | EP 0 307 141 A2 (MERCK SHARP & DOHME [GB]) 15 March 1989 (1989-03-15)<br>* the whole document * | 1-14 | |
| A,D | EP 0 323 864 A2 (MERCK SHARP & DOHME [GB]) 12 July 1989 (1989-07-12)<br>* the whole document * | 1-14 | |
| A | US 6 242 448 B1 (KELLY MICHAEL GERARD [US] ET AL) 5 June 2001 (2001-06-05)<br>* the whole document * | 1-14 | |

TECHNICAL FIELDS SEARCHED (IPC)

C07D
A61K

The present search report has been drawn up for all claims

1

| Place of search | Date of completion of the search | Examiner |
|---|---|---|
| Munich | 9 December 2010 | Ladenburger, Claude |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 17 0818

This annex lists the patent family members relating to the patent documents cited in the above-mentioned European search report.
The members are as contained in the European Patent Office EDP file on
The European Patent Office is in no way liable for these particulars which are merely given for the purpose of information.

09-12-2010

| Patent document cited in search report | Publication date | Patent family member(s) | Publication date |
|---|---|---|---|
| WO 9730994 A | 28-08-1997 | AP 751 A | 12-07-1999 |
| | | AT 200899 T | 15-05-2001 |
| | | AU 712230 B2 | 04-11-1999 |
| | | AU 1601497 A | 10-09-1997 |
| | | BG 102699 A | 30-09-1999 |
| | | BG 63971 B1 | 29-08-2003 |
| | | BR 9707733 A | 27-07-1999 |
| | | CA 2247164 A1 | 28-08-1997 |
| | | CN 1211981 A | 24-03-1999 |
| | | CO 4650040 A1 | 03-09-1998 |
| | | CZ 9802678 A3 | 11-08-1999 |
| | | DE 69704701 D1 | 07-06-2001 |
| | | DE 69704701 T2 | 16-08-2001 |
| | | DK 885218 T3 | 28-05-2001 |
| | | DZ 2181 A1 | 01-12-2002 |
| | | EP 0885218 A1 | 23-12-1998 |
| | | ES 2156357 T3 | 16-06-2001 |
| | | GR 3036032 T3 | 28-09-2001 |
| | | HK 1018274 A1 | 11-04-2003 |
| | | HR 970098 A2 | 30-04-1999 |
| | | HU 9900501 A2 | 28-06-1999 |
| | | ID 16642 A | 23-10-1997 |
| | | IL 125413 A | 12-09-2002 |
| | | IS 4803 A | 21-07-1998 |
| | | JP 3148252 B2 | 19-03-2001 |
| | | JP 11504051 T | 06-04-1999 |
| | | NO 983871 A | 21-08-1998 |
| | | NZ 331039 A | 28-10-1999 |
| | | OA 10838 A | 13-08-2001 |
| | | PL 328633 A1 | 15-02-1999 |
| | | PT 885218 E | 30-08-2001 |
| | | RU 2162848 C2 | 10-02-2001 |
| | | SK 112398 A3 | 10-04-2000 |
| | | TR 9801644 T2 | 23-11-1998 |
| | | TW 492961 B | 01-07-2002 |
| | | UA 61068 C2 | 17-11-2003 |
| | | US 5977142 A | 02-11-1999 |
| | | ZA 9701513 A | 21-08-1998 |
| EP 0307141 A2 | 15-03-1989 | DE 3877470 D1 | 25-02-1993 |
| | | DE 3877470 T2 | 24-06-1993 |
| | | DK 503288 A | 28-04-1989 |
| | | ES 2053748 T3 | 01-08-1994 |
| | | JP 1151576 A | 14-06-1989 |
| EP 0323864 A2 | 12-07-1989 | AU 628311 B2 | 17-09-1992 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 10 17 0818

This annex lists the patent family members relating to the patent documents cited in the above-mentioned European search report.
The members are as contained in the European Patent Office EDP file on
The European Patent Office is in no way liable for these particulars which are merely given for the purpose of information.

09-12-2010

| Patent document cited in search report | Publication date | Patent family member(s) | Publication date |
|---|---|---|---|
| EP 0323864 A2 | | AU 2779889 A | 20-07-1989 |
| | | DK 4189 A | 09-07-1989 |
| | | JP 2149580 A | 08-06-1990 |
| | | NZ 227517 A | 26-04-1990 |
| | | PT 89381 A | 08-02-1990 |
| US 6242448 B1 | 05-06-2001 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9730994 A
- EP 0323864 A
- US 20050025718 A
- WO 02060532 A
- WO 02060533 A
- WO 0410575 A
- WO 03087097 A
- WO 04074246 A
- WO 04089892 A
- WO 0511104 A
- WO 06023457 A
- WO 06023460 A
- WO 0288167 A
- WO 0212266 A
- WO 02100879 A
- WO 0200679 A
- WO 0335668 A
- WO 0348181 A
- WO 0362259 A
- WO 0364445 A
- WO 0372592 A
- WO 0439827 A
- WO 0466920 A
- EP 1440966 A
- JP 05025045 B
- WO 9318007 A
- WO 9964035 A
- US 20020055651 A
- US 20050133417 A
- US 20055159448 A
- WO 00075114 A
- WO 0142193 A
- WO 0183462 A
- WO 0266422 A
- WO 0270490 A
- WO 0276933 A
- WO 0324439 A
- WO 0342160 A
- WO 0342164 A
- WO 0372539 A
- WO 0391204 A
- WO 0399764 A
- WO 0416578 A
- WO 04016601 A
- WO 0422547 A
- WO 0432921 A
- WO 0433412 A
- WO 0437768 A
- WO 0437773 A
- WO 0437807 A
- WO 0439762 A
- WO 0439766 A
- WO 0445618 A
- WO 0446083 A
- WO 0471388 A
- WO 0480964 A
- EP 1460064 A
- WO 04087142 A
- WO 0489892 A
- EP 01477167 A
- US 20040242622 A
- US 20040229904 A
- WO 04108675 A
- WO 04108676 A
- WO 05033121 A
- WO 05040103 A
- WO 05044787 A
- WO 04071388 A
- WO 05058299 A
- WO 05058867 A
- WO 05065650 A
- WO 05066140 A
- WO 05070908 A
- WO 05092840 A
- WO 05092841 A
- WO 05092860 A
- WO 05092887 A
- WO 05092861 A
- WO 05090288 A
- WO 05092087 A
- WO 05080324 A
- WO 05080313 A
- US 20050182091 A
- US 20050171147 A
- WO 05092870 A
- WO 05077361 A
- DE 10258695
- WO 05111002 A
- WO 05111005 A
- WO 05110990 A
- US 20050272769 A
- WO 05110359 A
- WO 05121065 A
- US 20060019991 A
- WO 06016245 A
- WO 06014704 A
- WO 06031556 A
- WO 06032627 A

- US 20060106075 A
- US 20060106213 A
- WO 06051373 A
- WO 06056471 A
- WO 04043942 A
- WO 05021509 A
- WO 05021512 A
- WO 05026123 A
- WO 05026124 A
- WO 04024700 A
- WO 04024701 A
- WO 04020410 A
- WO 04020412 A
- WO 05080372 A
- WO 05082863 A
- WO 05082864 A
- WO 03053930 A
- EP 1052264 A
- EP 1241176 A
- WO 200242298 A
- EP 0505321 A
- GB 2214180 A
- US 2005277688 A
- US 2004254219 A

**Non-patent literature cited in the description**


- **Caulfield.** *Pharmac. Ther.,* 1993, vol. 58, 319-379
- **Fryer ; Jacoby.** *Am J Resp Crit Care Med.,* 1998, vol. 158, 154-160
- **Gross et al.** *Chest,* 1989, vol. 96, 984-987
- **Gross et al.** *Am Rev Respir Dis,* 1984, vol. 129, 856-870
- **Fryer et al.** *Life Sci.,* 1999, vol. 64 (6-7), 449-455
- **Pauwels et al.** *Am Rev Respir Crit Care Med,* 2001, vol. 163, 1256-1276
- *Chem. Pharm. Bull.,* 1979, vol. 27 (12), 3149-3152
- *J. Pharm. Sci,* 1980, vol. 69 (5), 534-537
- *Med. Chem. Res,* 2001, vol. 10 (9), 615-633
- *Expert Opin. Investig. Drugs,* 2005, vol. 14 (7), 775-783
- The Practice of Medicinal Chemistry. 2001, 561-585
- **F. J. Leinweber.** *Drug Metab. Res.,* 1987, vol. 18, 379
- **T. W. Greene ; P. G. M. Wuts.** Protective groups in organic chemistry. John Wiley and Sons, 1999
- *J. Chem. Soc.,* 1948, 1960
- *Tetrahedron,* 2002, vol. 58 (14), 2813
- *J. Org. Chem.,* vol. 2, 319
- *J. Org. Chem.,* 2006, vol. 71 (8), 3026
- *Helv. Chim. Acta,* 1946, vol. 29, 1957
- *Synthesis,* 2004, vol. 4, 595
- *Helv. Chim. Acta.,* 1946, vol. 29, 1957
- *J. Am. Chem. Soc.,* 2002, vol. 124 (28), 8206